# EUROPÄISCHE PATENTSCHRIFT

(11) **EP 1 419 255 B1**
(45) Veröffentlichungstag und Bekanntmachung des Hinweises auf die Patenterteilung: **29.02.2012**
(21) Anmeldenummer: 02754724.9
(22) Anmeldetag: 20.06.2002
(51) Int. Cl.: C12N 15/56, C12N 15/62, C12N 9/28, C12N 5/10, C12Q 1/68

(54) **EINE NEUE GRUPPE VON ALPHA-AMYLASEN SOWIE EIN VERFAHREN ZUR IDENTIFIZIERUNG UND GEWINNUNG NEUER ALPHA-AMYLASEN**
A NOVEL GROUP OF ALPHA-AMYLASES AND A METHOD FOR IDENTIFICATION AND PRODUCTION OF NOVEL ALPHA-AMYLASES
NOUVEAU GROUPE D'ALPHA-AMYLASES, ET PROCEDE D'IDENTIFICATION ET D'OBTENTION DE NOUVELLES ALPHA-AMYLASES

(30) Priorität: 29.06.2001 DE 10131441
(43) Veröffentlichungstag der Anmeldung: 19.05.2004
(73) Patentinhaber: Henkel AG & Co. KGaA, 40589 Düsseldorf (DE); B.R.A.I.N. Biotechnology Research And Information Network AG, 64673 Zwingenberg (DE)
(72) Erfinder: BREVES, Roland, 40822 Mettmann (DE); KOTTWITZ, Beatrix, 40593 Düsseldorf (DE); MAURER, Karl-Heinz, 40699 Erkrath (DE); ECK, Jürgen, 64646 Heppenheim (DE); LORENZ, Patrick, 69493 Hirschberg (DE); ZINKE, Holger, 64673 Zwingenberg (DE)
(74) Vertreter: Vossius & Partner
(86) Internationale Anmeldenummer: PCT/EP2002/006842
(87) Internationale Veröffentlichungsnummer: WO 2003/002711

(56) Entgegenhaltungen:
- WO-A-90/14426
- WO-A-03/054177
- MELLOULI LOTFI ET AL: "Alpha-amylase gene of thermophilic Streptomyces sp. TO1: Nucleotide sequence, transcriptional and amino acid sequence analysis" FEMS MICROBIOLOGY LETTERS, Bd. 160, Nr. 1, 1. März 1998 (1998-03-01), Seiten 17-23, XP002355887 ISSN: 0378-1097 -& DATABASE NCBI 12. März 1998 (1998-03-12), XP002355890 Database accession no. Y13332
- DATABASE NCBI 8. April 1996 (1996-04-08), XP002355891 Database accession no. U51129
- YIN X-H ET AL: "Cloning and characterization of a new alpha-amylase gene from Streptomyces lividans TK24" GENE: AN INTERNATIONAL JOURNAL ON GENES AND GENOMES, ELSEVIER SCIENCE PUBLISHERS, BARKING, GB, Bd. 197, Nr. 1-2, 15. September 1997 (1997-09-15), Seiten 37-45, XP004126400 ISSN: 0378-1119
- VIGAL T ET AL: "CLONING CHARACTERIZATION AND EXPRESSION OF AN ALPHA AMYLASE GENE FROM STREPTOMYCES-GRISEUS IMRU3570" MOLECULAR & GENERAL GENETICS, Bd. 225, Nr. 2, 1991, Seiten 278-288, XP002225551 ISSN: 0026-8925
- VIROLLE M-J ET AL: "CLONING, CHARACTERISATION AND REGULATION OF AN X-AMYLASE GENE FROM STREPTOMYCES VENEZUELAE" GENE, ELSEVIER BIOMEDICAL PRESS. AMSTERDAM, NL, Bd. 74, Nr. 2, 30. Dezember 1988 (1988-12-30), Seiten 321-334, XP000006072 ISSN: 0378-1119
- LONG C M ET AL: "ALPHA AMYLASE GENE OF STREPTOMYCES-LIMOSUS NUCLEOTIDE SEQUENCE EXPRESSION MOTIFS AND AMINO ACID SEQUENCE HOMOLOGY TO MAMMALIAN AND INVERTEBRATE ALPHA AMYLASES" JOURNAL OF BACTERIOLOGY, Bd. 169, Nr. 12, 1987, Seiten 5745-5754, XP009003065 ISSN: 0021-9193
- PEALE FRANKLIN V JR ET AL: "Multiplex display polymerase chain reaction amplifies and resolves related sequences sharing a single moderately conserved domain." ANALYTICAL BIOCHEMISTRY, Bd. 256, Nr. 2, 15. Februar 1998 (1998-02-15), Seiten 158-168, XP000752498 ISSN: 0003-2697
- KIM SOON-OK ET AL: "Efficient cloning of the genes for RNA polymerase sigma-like factors from actinomycetes." JOURNAL OF MICROBIOLOGY AND BIOTECHNOLOGY, Bd. 8, Nr. 3, Juni 1998 (1998-06), Seiten 280-283, XP009003030 ISSN: 1017-7825
- KIM HEE-OK ET AL: "A gene encoding Achlya bisexualis beta-amylase and its expression in Saccharomyces cerevisiae." BIOTECHNOLOGY LETTERS, Bd. 23, Nr. 14, Juli 2001 (2001-07), Seiten 1101-1107, XP009002956 ISSN: 0141-5492

### SEQUENZPROTOKOLL

<110> Henkel Kommanditgesellschaft auf Aktien
<120> Eine neue Gruppe von Alpha-Amylasen sowie ein Verfahren zur Identifizierung und Gewinnung neuer Alpha-Amylasen
<130> H 4890 PCT
<140>
<141>
<150> DE 10131441.8
<151> 2001-06-29
<160> 263
<170> PatentIn Ver. 2.1
<210> 1
   <211> 302
   <212> DNA
   <213> Streptomyces sp. B327*
<220>
   <221> CDS
   <222> (2)..(301)
<400> 1
   c gtc gac ggc ttc cgc atc gac acg gcc aag cac atc ccg gcg acc gac 49
     Val Asp Gly Phe Arg Ile Asp Thr Ala Lys His Ile Pro Ala Thr Asp
       1               5                  10                  15
   ctc gcc aac atc aag tcg cgt ctg acg aac ccc tcc gtc tac tgg aag   97
   Leu Ala Asn Ile Lys Ser Arg Leu Thr Asn Pro Ser Val Tyr Trp Lys
                20                  25                  30
   cag gag gtc atc tac ggc agc ggg gag gcc gtc cag ccc acc gag tac   145
   Gln Glu Val Ile Tyr Gly Ser Gly Glu Ala Val Gln Pro Thr Glu Tyr
            35                  40                  45
   acg ggc aac ggg gac gtc cag gag ttc cgc tac gcc tac gac ctc aag   193
   Thr Gly Asn Gly Asp Val Gln Glu Phe Arg Tyr Ala Tyr Asp Leu Lys
        50                  55                  60
   cgc gtc ttc aac aac gag aac ctc gcc tat ctg aag aac tac ggc gag   241
   Arg Val Phe Asn Asn Glu Asn Leu Ala Tyr Leu Lys Asn Tyr Gly Glu
    65                  70                  75                  80
   ggc tgg ggg tac ctg aac agc tcg gtg gcc ggc gtc tac gtc gac aac   289
   Gly Trp Gly Tyr Leu Asn Ser Ser Val Ala Gly Val Tyr Val Asp Asn
                85                  90                  95
   cac gac acc gag c                                                 302
   His Asp Thr Glu
               100
<210> 2
   <211> 100
   <212> PRT
   <213> Streptomyces sp. B327*
<400> 2
   Val Asp Gly Phe Arg Ile Asp Thr Ala Lys His Ile Pro Ala Thr Asp
    1               5                  10                  15
   Leu Ala Asn Ile Lys Ser Arg Leu Thr Asn Pro Ser Val Tyr Trp Lys
                20                  25                  30
   Gln Glu Val Ile Tyr Gly Ser Gly Glu Ala Val Gln Pro Thr Glu Tyr
            35                  40                  45
   Thr Gly Asn Gly Asp Val Gln Glu Phe Arg Tyr Ala Tyr Asp Leu Lys
        50                  55                  60
   Arg Val Phe Asn Asn Glu Asn Leu Ala Tyr Leu Lys Asn Tyr Gly Glu
    65                  70                  75                  80
   Gly Trp Gly Tyr Leu Asn Ser Ser Val Ala Gly Val Tyr Val Asp Asn
                85                  90                  95
   His Asp Thr Glu
               100
<210> 3
   <211> 293
   <212> DNA
   <213> Streptomyces sp. B400B
<220>
   <221> CDS
   <222> (2)..(292)
<400> 3
   c gtc gac ggc ttc cgc atc gac gcc gcc aag cac atg tcc gcc gac gac 49
     Val Asp Gly Phe Arg Ile Asp Ala Ala Lys His Met Ser Ala Asp Asp
     1               5                   10                  15
   gtc gcc gcg atc aag ggg aag atg agc gac ccg ggg ttc tgg gtc acc   97
   Val Ala Ala Ile Lys Gly Lys Met Ser Asp Pro Gly Phe Trp Val Thr
               20                  25                  30
   gag gtc atc cac ggc ggg gga gag gcg gtc cag ccg gag gag tac acc   145
   Glu Val Ile His Gly Gly Gly Glu Ala Val Gln Pro Glu Glu Tyr Thr
           35                  40                  45
   tcg atc ggg gac gcc gac gag ttc cgt tac ggc ggc cac ctc aag tcc   193
   Ser Ile Gly Asp Ala Asp Glu Phe Arg Tyr Gly Gly His Leu Lys Ser
       50                  55                  60
   gcc ttc cag ggc ggc ggc ctg ccc ggc ctg aag tcg atc gcc gac ggc   241
   Ala Phe Gln Gly Gly Gly Leu Pro Gly Leu Lys Ser Ile Ala Asp Gly
   65                  70                  75                  80
   aaa ctg gcc ggc gcc tcc gcc cgt acg tac gtg gac aac cac gac acc   289
   Lys Leu Ala Gly Ala Ser Ala Arg Thr Tyr Val Asp Asn His Asp Thr
                   85                  90                  95
   gag c                                                             293
   Glu
<210> 4
   <211> 97
   <212> PRT
   <213> Streptomyces sp. B400B
<400> 4
   Val Asp Gly Phe Arg Ile Asp Ala Ala Lys His Met Ser Ala Asp Asp
   1               5                   10                  15
   Val Ala Ala Ile Lys Gly Lys Met Ser Asp Pro Gly Phe Trp Val Thr
               20                  25                  30
   Glu Val Ile His Gly Gly Gly Glu Ala Val Gln Pro Glu Glu Tyr Thr
           35                  40                  45
   Ser Ile Gly Asp Ala Asp Glu Phe Arg Tyr Gly Gly His Leu Lys Ser
       50                  55                  60
   Ala Phe Gln Gly Gly Gly Leu Pro Gly Leu Lys Ser Ile Ala Asp Gly
   65                  70                  75                  80
   Lys Leu Ala Gly Ala Ser Ala Arg Thr Tyr Val Asp Asn His Asp Thr
                   85                  90                  95
   Glu
<210> 5
   <211> 1386
   <212> DNA
   <213> Streptomyces sp. B327*
<220>
   <221> CDS
   <222> (1)..(1386)
<220>
   <221> misc_feature
   <222> (1).. (3)
   <223> INIT_MET
<220>
   <221> mat_peptide
   <222> (91)
<400> 5
   gtg ata tcg aga tgg acc gca tcc gct gtc gcc acg gcc gcc gcg ttc    48
   Val Ile Ser Arg Trp Thr Ala Ser Ala Val Ala Thr Ala Ala Ala Phe
   -30                 -25                 -20                 -15
   gcc gcc gcg gtg gcc ctg ccg gcc ccc cag gcc gcc tac gcc tcc ccg    96
   Ala Ala Ala Val Ala Leu Pro Ala Pro Gln Ala Ala Tyr Ala Ser Pro
                   -10                 -5                  -1   1
   ccc ggt acc aag gac gtc acc gcc gtc ctc ttc gag tgg aac ttc gcc   144
   Pro Gly Thr Lys Asp Val Thr Ala Val Leu Phe Glu Trp Asn Phe Ala
                5                  10                  15
   tcc gtc gcc aag gag tgc acc aac acc ctc ggc ccc gcc gga tac ggc   192
   Ser Val Ala Lys Glu Cys Thr Asn Thr Leu Gly Pro Ala Gly Tyr Gly
        20                  25                  30
   tcc gtg cag gtc tcc ccg ccc gcc gag cac atc cag ggc tca cag tgg   240
   Ser Val Gln Val Ser Pro Pro Ala Glu His Ile Gln Gly Ser Gln Trp
    35                  40                  45                  50
   tgg acc tcg tac cag ccg gtc agc tac aag atc gcg ggg cgc ctc ggt   288
   Trp Thr Ser Tyr Gln Pro Val Ser Tyr Lys Ile Ala Gly Arg Leu Gly
                    55                  60                  65
   gac gcc acc gcc ttc aag aac atg gtc ggc acc tgc cac gcg gcc ggg   336
   Asp Ala Thr Ala Phe Lys Asn Met Val Gly Thr Cys His Ala Ala Gly
                70                  75                  80
   gtg aag gtc gtc gtc gac acc gtc atc aac cac atg tcc gcg ggc agc   384
   Val Lys Val Val Val Asp Thr Val Ile Asn His Met Ser Ala Gly Ser
            85                  90                  95
   ggc acc ggt acc ggc gga tcg tcg tac acg aag tac aac tac ccc ggc   432
   Gly Thr Gly Thr Gly Gly Ser Ser Tyr Thr Lys Tyr Asn Tyr Pro Gly
       100                 105                 110
   ctg tac tcc tcg tac gac atg gac gac tgc acg tcg acc atc acc gac   480
   Leu Tyr Ser Ser Tyr Asp Met Asp Asp Cys Thr Ser Thr Ile Thr Asp
   115                 120                 125                 130
   tac acc aac cgc ggc aac gtc cag aac tgc gaa ctc gtc ggc ctc gcc   528
   Tyr Thr Asn Arg Gly Asn Val Gln Asn Cys Glu Leu Val Gly Leu Ala
                   135                 140                 145
   gac ctg gac acc ggc gag gag tac gtc cgc gcc acc atc gcc ggc tac   576
   Asp Leu Asp Thr Gly Glu Glu Tyr Val Arg Ala Thr Ile Ala Gly Tyr
               150                 155                 160
   ctg aac tcg ctg ctc ggc tac ggc gtc gac ggc ttc cgc atc gac gcg   624
   Leu Asn Ser Leu Leu Gly Tyr Gly Val Asp Gly Phe Arg Ile Asp Ala
           165                 170                 175
   gcc aag cac atc ccg gcg acc gac ctc gcc aac atc aag tcg cgt ctg   672
   Ala Lys His Ile Pro Ala Thr Asp Leu Ala Asn Ile Lys Ser Arg Leu
       180                 185                 190
   acg aac ccc tcc gtc tac tgg aag cag gag gtc atc tac ggc agc ggg   720
   Thr Asn Pro Ser Val Tyr Trp Lys Gln Glu Val Ile Tyr Gly Ser Gly
   195                 200                 205                 210
   gag gcc gtc cag ccc acc gag tac acg ggc aac ggg gac gtc cag gag   768
   Glu Ala Val Gln Pro Thr Glu Tyr Thr Gly Asn Gly Asp Val Gln Glu
                   215                 220                 225
   ttc cgc tac gcc tac gac ctc aag cgc gtc ttc aac aac gag aac ctc     816
   Phe Arg Tyr Ala Tyr Asp Leu Lys Arg Val Phe Asn Asn Glu Asn Leu
               230                 235                 240
   gcc tat ctg aag aac tac ggc gag ggc tgg ggg tac ctg aac agc tcg     864
   Ala Tyr Leu Lys Asn Tyr Gly Glu Gly Trp Gly Tyr Leu Asn Ser Ser
           245                 250                 255
   gtg gcc ggc gtc ttc gtc gac aac cac gac acc gag cgc aac ggc tcc     912
   Val Ala Gly Val Phe Val Asp Asn His Asp Thr Glu Arg Asn Gly Ser
       260                 265                 270
   acg ctc aac tac aag gac ggc gcg aac tac acc ctc gcc aac gtc ttc     960
   Thr Leu Asn Tyr Lys Asp Gly Ala Asn Tyr Thr Leu Ala Asn Val Phe
   275                 280                 285                 290
   atg ctc gcc tac ccg tac ggc gcc ccg gac atc aac tca ggc tac gag     1008
   Met Leu Ala Tyr Pro Tyr Gly Ala Pro Asp Ile Asn Ser Gly Tyr Glu
                   295                 300                 305
   tgg tcc gac acg gac gcg ggc ccg ccc aac aac ggc tcc gtg agc gcc     1056
   Trp Ser Asp Thr Asp Ala Gly Pro Pro Asn Asn Gly Ser Val Ser Ala
               310                 315                 320
   tgt tgg cag gac ggc tgg aag tgt cag cac gcc tgg ccg gag atc ctc     1104
   Cys Trp Gln Asp Gly Trp Lys Cys Gln His Ala Trp Pro Glu Ile Leu
           325                 330                 335
   cgc atg gtc gcc ttc cgc aac gcc acc cgc ggt gag tcg gtc acc aac     1152
   Arg Met Val Ala Phe Arg Asn Ala Thr Arg Gly Glu Ser Val Thr Asn
       340                 345                 350
   tgg tgg gac aac ggc ggc gac gcg atc gcc ttc ggg cgg ggc gcc aag     1200
   Trp Trp Asp Asn Gly Gly Asp Ala Ile Ala Phe Gly Arg Gly Ala Lys
   355                 360                 365                 370
   ggc tat gtc gcc atc aac cac gag tcc ggc agc ctg agc cgg acc tac     1248
   Gly Tyr Val Ala Ile Asn His Glu Ser Gly Ser Leu Ser Arg Thr Tyr
                   375                 380                 385
   cag acc tcg ctg ccc gcc ggc acg tac tgc aac gtg cag aac aac acg     1296
   Gln Thr Ser Leu Pro Ala Gly Thr Tyr Cys Asn Val Gln Asn Asn Thr
               390                 395                 400
   agc gtg acg gtc ggc tcc aac ggt cag ttc acg gcc acg ctc ggg tcc     1344
   Ser Val Thr Val Gly Ser Asn Gly Gln Phe Thr Ala Thr Leu Gly Ser
           405                 410                 415
   aac acg gca ctg gcg atc tac gcg ggc aag gcc aac tgc tga             1386
   Asn Thr Ala Leu Ala Ile Tyr Ala Gly Lys Ala Asn Cys
       420                 425                 430
<210> 6
   <211> 461
   <212> PRT
   <213> Streptomyces sp. B327*
<400> 6
   Val Ile Ser Arg Trp Thr Ala Ser Ala Val Ala Thr Ala Ala Ala Phe
   1 5 10 15
   Ala Ala Ala Val Ala Leu Pro Ala Pro Gln Ala Ala Tyr Ala Ser Pro
   20 25 30
   Pro Gly Thr Lys Asp Val Thr Ala Val Leu Phe Glu Trp Asn Phe Ala
   35 40 45
   Ser Val Ala Lys Glu Cys Thr Asn Thr Leu Gly Pro Ala Gly Tyr Gly
   50 55 60
   Ser Val Gln Val Ser Pro Pro Ala Glu His Ile Gln Gly Ser Gln Trp
   65 70 75 80
   Trp Thr Ser Tyr Gln Pro Val Ser Tyr Lys Ile Ala Gly Arg Leu Gly
   85 90 95
   Asp Ala Thr Ala Phe Lys Asn Met Val Gly Thr Cys His Ala Ala Gly
   100 105 110
   Val Lys Val Val Val Asp Thr Val Ile Asn His Met Ser Ala Gly Ser
   115 120 125
   Gly Thr Gly Thr Gly Gly Ser Ser Tyr Thr Lys Tyr Asn Tyr Pro Gly
   130 135 140
   Leu Tyr Ser Ser Tyr Asp Met Asp Asp Cys Thr Ser Thr Ile Thr Asp
   145 150 155 160
   Tyr Thr Asn Arg Gly Asn Val Gln Asn Cys Glu Leu Val Gly Leu Ala
   165 170 175
   Asp Leu Asp Thr Gly Glu Glu Tyr Val Arg Ala Thr Ile Ala Gly Tyr
   180 185 190
   Leu Asn Ser Leu Leu Gly Tyr Gly Val Asp Gly Phe Arg Ile Asp Ala
   195 200 205
   Ala Lys His Ile Pro Ala Thr Asp Leu Ala Asn Ile Lys Ser Arg Leu
   210 215 220
   Thr Asn Pro Ser Val Tyr Trp Lys Gln Glu Val Ile Tyr Gly Ser Gly
   225 230 235 240
   Glu Ala Val Gln Pro Thr Glu Tyr Thr Gly Asn Gly Asp Val Gln Glu
   245 250 255
   Phe Arg Tyr Ala Tyr Asp Leu Lys Arg Val Phe Asn Asn Glu Asn Leu
   260 265 270
   Ala Tyr Leu Lys Asn Tyr Gly Glu Gly Trp Gly Tyr Leu Asn Ser Ser
   275 280 285
   Val Ala Gly Val Phe Val Asp Asn His Asp Thr Glu Arg Asn Gly Ser
   290 295 300
   Thr Leu Asn Tyr Lys Asp Gly Ala Asn Tyr Thr Leu Ala Asn Val Phe
   305 310 315 320
   Met Leu Ala Tyr Pro Tyr Gly Ala Pro Asp Ile Asn Ser Gly Tyr Glu
   325 330 335
   Trp Ser Asp Thr Asp Ala Gly Pro Pro Asn Asn Gly Ser Val Ser Ala
   340 345 350
   Cys Trp Gln Asp Gly Trp Lys Cys Gln His Ala Trp Pro Glu Ile Leu
   355 360 365
   Arg Met Val Ala Phe Arg Asn Ala Thr Arg Gly Glu Ser Val Thr Asn
   370 375 380
   Trp Trp Asp Asn Gly Gly Asp Ala Ile Ala Phe Gly Arg Gly Ala Lys
   385 390 395 400
   Gly Tyr Val Ala Ile Asn His Glu Ser Gly Ser Leu Ser Arg Thr Tyr
   405 410 415
   Gln Thr Ser Leu Pro Ala Gly Thr Tyr Cys Asn Val Gln Asn Asn Thr
   420 425 430
   Ser Val Thr Val Gly Ser Asn Gly Gln Phe Thr Ala Thr Leu Gly Ser
   435 440 445
   Asn Thr Ala Leu Ala Ile Tyr Ala Gly Lys Ala Asn Cys
   450 455 460
<210> 7
   <211> 1377
   <212> DNA
   <213> Streptomyces sp. B400B
<220>
   <221> CDS
   <222> (1)..(1377)
<220>
   <221> misc_feature
   <222> (1)..(3)
   <223> INIT_MET
<220>
   <221> mat_peptide
   <222> (88)
<400> 7
   ttg agt tcc cgc gcc gca cgc gca acc ctg gcg ggc ctg ctc gcc gcc    48
   Leu Ser Ser Arg Ala Ala Arg Ala Thr Leu Ala Gly Leu Leu Ala Ala
                   -25                 -20                 -15
   ggt ggc ctc acg gtc ctc gcc ccc tgg cct tcc cag gcg acc cca ccg    96
   Gly Gly Leu Thr Val Leu Ala Pro Trp Pro Ser Gln Ala Thr Pro Pro
               -10                 -5                  -1   1
   ggc gag aag acc gtc acc gcc acc ctc ttc gag tgg aag tac gac gcg    144
   Gly Glu Lys Thr Val Thr Ala Thr Leu Phe Glu Trp Lys Tyr Asp Ala
       5                   10                  15
   gtg gcc acc gcc tgc acc gac acc ctg ggc ccg gcc ggc tac ggc tac    192
   Val Ala Thr Ala Cys Thr Asp Thr Leu Gly Pro Ala Gly Tyr Gly Tyr
   20                  25                  30                  35
   gtc gag gtc tcg ccc gcc acc gag cac atc cag ggc gac cag tgg tgg    240
   Val Glu Val Ser Pro Ala Thr Glu His Ile Gln Gly Asp Gln Trp Trp
                   40                  45                  50
   acc tcg tac cag ccg gtc agc tac cgg atc gcg ggc cgt ctc ggt gac    288
   Thr Ser Tyr Gln Pro Val Ser Tyr Arg Ile Ala Gly Arg Leu Gly Asp
               55                  60                  65
   cgg gac tcc ttc gcc gcg atg gtg gag agc tgc cac gcg gcc ggg gtc    336
   Arg Asp Ser Phe Ala Ala Met Val Glu Ser Cys His Ala Ala Gly Val
           70                  75                  80
   cgg gtc gtg gcg gac gcc gtg atc aac cac atg gcc gcc ggc tcc ggg    384
   Arg Val Val Ala Asp Ala Val Ile Asn His Met Ala Ala Gly Ser Gly
       85                  90                  95
   acc ggg acg ggc ggc acg tcg tac acc aag tac gac tac ccc ggc acg    432
   Thr Gly Thr Gly Gly Thr Ser Tyr Thr Lys Tyr Asp Tyr Pro Gly Thr
   100                 105                 110                 115
   ttc cag gac cag gac ttc cac gcc tgc cgc aag gac atc gcg aac tac    480
   Phe Gln Asp Gln Asp Phe His Ala Cys Arg Lys Asp Ile Ala Asn Tyr
                   120                 125                 130
   ggc gac cgc ggc gac gtc cag aac tgc gaa ctg gtc ggc ctc gcg gac      528
   Gly Asp Arg Gly Asp Val Gln Asn Cys Glu Leu Val Gly Leu Ala Asp
               135                 140                 145
   ctg gac acc ggc agc gac gcc gta cgc acg acg atc gcc gcc tac ctc      576
   Leu Asp Thr Gly Ser Asp Ala Val Arg Thr Thr Ile Ala Ala Tyr Leu
           150                 155                 160
   tcc gac ctc cgc tcc ctg ggc gtc gac ggc ttc cgc atc gac gcc gcc      624
   Ser Asp Leu Arg Ser Leu Gly Val Asp Gly Phe Arg Ile Asp Ala Ala
       165                 170                 175
   aag cac atg tcc gcc gac gac gtc gcc gcg atc aag ggg aag atg agc      672
   Lys His Met Ser Ala Asp Asp Val Ala Ala Ile Lys Gly Lys Met Ser
   180                 185                 190                 195
   gac ccg ggg ttc tgg gtc acc gag gtc atc cac ggc ggg gga gag gcg      720
   Asp Pro Gly Phe Trp Val Thr Glu Val Ile His Gly Gly Gly Glu Ala
                   200                 205                 210
   gtc cag ccg gag gag tac acc tcg atc ggg gac gtc gac gag ttc cgt      768
   Val Gln Pro Glu Glu Tyr Thr Ser Ile Gly Asp Val Asp Glu Phe Arg
               215                 220                 225
   tac ggc ggc cac ctc aag tcc gcc ttc cag ggc ggc ggc ctg ccc ggc      816
   Tyr Gly Gly His Leu Lys Ser Ala Phe Gln Gly Gly Gly Leu Pro Gly
           230                 235                 240
   ctg aag tcg atc gcc gac ggc aaa ctg gcc ggc gcc tcc gcc cgt acg      864
   Leu Lys Ser Ile Ala Asp Gly Lys Leu Ala Gly Ala Ser Ala Arg Thr
       245                 250                 255
   ttc gtc gac aac tgg gac acc gag cgc aac ggc tcc acc ctc acc cac      912
   Phe Val Asp Asn Trp Asp Thr Glu Arg Asn Gly Ser Thr Leu Thr His
   260                 265                 270                 275
   aag gac ggc gcc gcc tac acc ctg gcc aac gtc ttc atg ctg gcc tcg      960
   Lys Asp Gly Ala Ala Tyr Thr Leu Ala Asn Val Phe Met Leu Ala Ser
                   280                 285                 290
   ccc tac ggc tcc ccg aac gtc ttc tcc ggc tac acc tgg acc gac aag     1008
   Pro Tyr Gly Ser Pro Asn Val Phe Ser Gly Tyr Thr Trp Thr Asp Lys
               295                 300                 305
   gac gcc ggc ccg ccg aac ggc ggc gcg gcc gac tgc ggc tcc ggc gcg     1056
   Asp Ala Gly Pro Pro Asn Gly Gly Ala Ala Asp Cys Gly Ser Gly Ala
           310                 315                 320
   tgg acc tgc acg cac gcc cag cag gcg gtc acc ggc atg gtc ggc ttc     1104
   Trp Thr Cys Thr His Ala Gln Gln Ala Val Thr Gly Met Val Gly Phe
       325                 330                 335
   cac aac gcc gtc gcg ggc gcg gag ctg acc gac tgg tgg gac gac ggc     1152
   His Asn Ala Val Ala Gly Ala Glu Leu Thr Asp Trp Trp Asp Asp Gly
   340                 345                 350                 355
   tcc tcc gcc ctc gcc ttc gcc cgc gcg ggc aag ggc ttt gtc gcc gtc     1200
   Ser Ser Ala Leu Ala Phe Ala Arg Ala Gly Lys Gly Phe Val Ala Val
                   360                 365                 370
   aac aac ggc gac gcg gag ctg aac cgc acc ttc acg acg acg ctg ccg     1248
   Asn Asn Gly Asp Ala Glu Leu Asn Arg Thr Phe Thr Thr Thr Leu Pro
               375                 380                 385
   gcg ggc acg tac tgc aat gtg gtc gcc gcg gcc ccc gac tcc tgc gac     1296
   Ala Gly Thr Tyr Cys Asn Val Val Ala Ala Ala Pro Asp Ser Cys Asp
           390                 395                 400
   ggc aac ggg acc acg gtc gcg gac gac ggc acg gcg acg atc acg gtc     1344
   Gly Asn Gly Thr Thr Val Ala Asp Asp Gly Thr Ala Thr Ile Thr Val
       405                 410                 415
   ccg gcg cgc gga gcg gtg gcg ctg cac acc tag                         1377
   Pro Ala Arg Gly Ala Val Ala Leu His Thr
   420                 425                 430
<210> 8
   <211> 458
   <212> PRT
   <213> Streptomyces sp. B400B
<400> 8
   Leu Ser Ser Arg Ala Ala Arg Ala Thr Leu Ala Gly Leu Leu Ala Ala
   1               5                   10                  15
   Gly Gly Leu Thr Val Leu Ala Pro Trp Pro Ser Gln Ala Thr Pro Pro
               20                  25                  30
   Gly Glu Lys Thr Val Thr Ala Thr Leu Phe Glu Trp Lys Tyr Asp Ala
           35                  40                  45
   Val Ala Thr Ala Cys Thr Asp Thr Leu Gly Pro Ala Gly Tyr Gly Tyr
       50                  55                  60
   Val Glu Val Ser Pro Ala Thr Glu His Ile Gln Gly Asp Gln Trp Trp
   65                  70                  75                  80
   Thr Ser Tyr Gln Pro Val Ser Tyr Arg Ile Ala Gly Arg Leu Gly Asp
                   85                  90                  95
   Arg Asp Ser Phe Ala Ala Met Val Glu Ser Cys His Ala Ala Gly Val
               100                 105                 110
   Arg Val Val Ala Asp Ala Val Ile Asn His Met Ala Ala Gly Ser Gly
           115                 120                 125
   Thr Gly Thr Gly Gly Thr Ser Tyr Thr Lys Tyr Asp Tyr Pro Gly Thr
       130                 135                 140
   Phe Gln Asp Gln Asp Phe His Ala Cys Arg Lys Asp Ile Ala Asn Tyr
   145                 150                 155                 160
   Gly Asp Arg Gly Asp Val Gln Asn Cys Glu Leu Val Gly Leu Ala Asp
                   165                 170                 175
   Leu Asp Thr Gly Ser Asp Ala Val Arg Thr Thr Ile Ala Ala Tyr Leu
               180                 185                 190
   Ser Asp Leu Arg Ser Leu Gly Val Asp Gly Phe Arg Ile Asp Ala Ala
           195                 200                 205
   Lys His Met Ser Ala Asp Asp Val Ala Ala Ile Lys Gly Lys Met Ser
       210                 215                 220
   Asp Pro Gly Phe Trp Val Thr Glu Val Ile His Gly Gly Gly Glu Ala
   225                 230                 235                 240
   Val Gln Pro Glu Glu Tyr Thr Ser Ile Gly Asp Val Asp Glu Phe Arg
                   245                 250                 255
   Tyr Gly Gly His Leu Lys Ser Ala Phe Gln Gly Gly Gly Leu Pro Gly
               260                 265                 270
   Leu Lys Ser Ile Ala Asp Gly Lys Leu Ala Gly Ala Ser Ala Arg Thr
           275                 280                 285
   Phe Val Asp Asn Trp Asp Thr Glu Arg Asn Gly Ser Thr Leu Thr His
       290                 295                 300
   Lys Asp Gly Ala Ala Tyr Thr Leu Ala Asn Val Phe Met Leu Ala Ser
   305                 310                 315                 320
   Pro Tyr Gly Ser Pro Asn Val Phe Ser Gly Tyr Thr Trp Thr Asp Lys
                   325                 330                 335
   Asp Ala Gly Pro Pro Asn Gly Gly Ala Ala Asp Cys Gly Ser Gly Ala
               340                 345                 350
   Trp Thr Cys Thr His Ala Gln Gln Ala Val Thr Gly Met Val Gly Phe
           355                 360                 365
   His Asn Ala Val Ala Gly Ala Glu Leu Thr Asp Trp Trp Asp Asp Gly
       370                 375                 380
   Ser Ser Ala Leu Ala Phe Ala Arg Ala Gly Lys Gly Phe Val Ala Val
   385                 390                 395                 400
   Asn Asn Gly Asp Ala Glu Leu Asn Arg Thr Phe Thr Thr Thr Leu Pro
                   405                 410                 415
   Ala Gly Thr Tyr Cys Asn Val Val Ala Ala Ala Pro Asp Ser Cys Asp
               420                 425                 430
   Gly Asn Gly Thr Thr Val Ala Asp Asp Gly Thr Ala Thr Ile Thr Val
           435                 440                 445
   Pro Ala Arg Gly Ala Val Ala Leu His Thr
       450                 455
<210> 9
   <211> 24
   <212> DNA
   <213> Künstliche Sequenz
<220>
   <223> PCR-Primer (GEX024)
<400> 9
   cgtcgacggc ttccgsatcg acrc 24
<210> 10
   <211> 25
   <212> DNA
   <213> Künstliche Sequenz
<220>
   <223> PCR-Primer (GEX026)
<400> 10
   gctcggtgtc gtggttgtcs acgwa 25
<210> 11
   <211> 24
   <212> DNA
   <213> Künstliche Sequenz
<220>
   <223> PCR-Primer (GEX029)
<400> 11
   cggcgtcgac ggctkscgbn tsga 24

<210> 12
   <211> 25
   <212> DNA
   <213> Künstliche Sequenz
<220>
   <223> PCR-Primer (GEX031)
<400> 12
   gctgggtgtc gtggttgtcs acsma 25
<210> 13
   <211> 22
   <212> DNA
   <213> Künstliche Sequenz
<220>
   <223> PCR-Primer (GEX015)
<400> 13
   ggtggacgtc ctaccagccs gt 22
<210> 14
   <211> 23
   <212> DNA
   <213> Künstliche Sequenz
<220>
   <223> PCR-Primer (GEX016)
<400> 14
   gcacccgcag gagcacrycs agg 23
<210> 15
   <211> 18
   <212> DNA
   <213> Künstliche Sequenz
<220>
   <223> PCR-Primer (GEX017)
<400> 15
   cgcggccggc gtsaagrt 18
<210> 16
   <211> 22
   <212> DNA
   <213> Künstliche Sequenz
<220>
   <223> PCR-Primer (GEX018)
<400> 16
   tggtcaacac ctgccacgms gc 22
<210> 17
   <211> 24
   <212> DNA
   <213> Künstliche Sequenz
<220>
   <223> PCR-Primer (GEX019)
<400> 17
   cgcggcgtcg atgckgaagm mgtc 24
<210> 18
   <211> 26
   <212> DNA
   <213> Künstliche Sequenz
<220>
   <223> PCR-Primer (GEX020)
<400> 18
   ggagccgtac ggccasgcsa gcatga 26
<210> 19
   <211> 23
   <212> DNA
   <213> Künstliche Sequenz
<220>
   <223> PCR-Primer (GEX021)
<400> 19
   cccttgtcgc cgcgsscgaa sgc 23
<210> 20
   <211> 24
   <212> DNA
   <213> Künstliche Sequenz
<220>
   <223> PCR-Primer (GEX022)
<400> 20
   ggcgtcctcg tggttgawsg csac 24
<210> 21
   <211> 27
   <212> DNA
   <213> Künstliche Sequenz
<220>
   <223> PCR-Primer (GEX023)
<400> 21
   ggtctacgcc gacgtcgtsw wcaacca 27
<210> 22
   <211> 21
   <212> DNA
   <213> Künstliche Sequenz
<220>
   <223> PCR-Primer (GEX025)
<400> 22
   ggcgtcgatg cggaasccgt c 21
<210> 23
   <211> 31
   <212> DNA
   <213> Künstliche Sequenz
<220>
   <223> PCR-Primer (GEX027)
<400> 23
   gcgtccaggt ctacgtcgac rysgtshtsa a 31
<210> 24
   <211> 29
   <212> DNA
   <213> Künstliche Sequenz
<220>
   <223> PCR-Primer (GEX028)
<400> 24
   caggtctacg tcgacgtcgt shtsaacca 29
<210> 25
   <211> 27
   <212> DNA
   <213> Künstliche Sequenz
<220>
   <223> PCR-Primer (GEX030)
<400> 25
   cggcggggat gtgctksrcs rmgtcsa 27
<210> 26
   <211> 24
   <212> DNA
   <213> Künstliche Sequenz
<220>
   <223> PCR-Primer (GEX036)
<400> 26
   gtacgccgac gccgtnwtha ayca 24
<210> 27
   <211> 26
   <212> DNA
   <213> Künstliche Sequenz
<220>
   <223> PCR-Primer (GEX037)
<400> 27
   gtacgccgac gccgtnwtha aycaya 26
<210> 28
   <211> 24
   <212> DNA
   <213> Künstliche Sequenz
<220>
   <223> PCR-Primer (GEX038)
<400> 28
   ggcggcgtcg atcckraanc crtc 24
<210> 29
   <211> 24
   <212> DNA
   <213> Künstliche Sequenz
<220>
   <223> PCR-Primer (GEX039)
<400> 29
   cttggcggcg tcgatnckra ancc 24
<210> 30
   <211> 25
   <212> DNA
   <213> Künstliche Sequenz
<220>
   <223> PCR-Primer (GEX040)
<400> 30
   tcttgctcgg cgtggayggn ttymg 25
<210> 31
   <211> 24
   <212> DNA
   <213> Künstliche Sequenz
<220>
   <223> PCR-Primer (GEX041)
<400> 31
   ccggatcgac gccgynaarc ayat 24
<210> 32
   <211> 26
   <212> DNA
   <213> Künstliche Sequenz
<220>
   <223> PCR-Primer (GEX042)
<400> 32
   cgctcggtgt cgtggttntc nacvma 26
<210> 33
   <211> 28
   <212> DNA
   <213> Künstliche Sequenz
<220>
   <223> PCR-Primer (GEX043)
<400> 33
   cgttccgctc ggtgtcgyrr ttntcnac 28
<210> 34
   <211> 100
   <212> PRT
   <213> Streptomyces sp. B1002
<400> 34
   Val Asp Gly Phe Arg Ile Asp Ala Ala Lys His Met Pro Ala Ala Asp
   1               5                   10                  15
   Leu Ala Asn Ile Lys Ser Arg Leu Gly Asp Pro Asn Val Tyr Trp Lys
               20                  25                  30
   Gln Glu Ala Ile Tyr Gly Ala Gly Glu Ala Val Ser Pro Asp Glu Tyr
           35                  40                  45
   Thr Gly Thr Gly Asp Val Gln Glu Phe Arg Tyr Ala Arg Gly Leu Lys
       50                  55                  60
   Gln Val Phe Asn Asn Glu Asn Leu Ala Tyr Leu Lys Asn Tyr Gly Glu
   65                  70                  75                  80
   Gly Trp Gly Phe Met Pro Ser Ser Lys Ala Ala Val Tyr Val Asp Asn
                   85                  90                  95
   His Asp Thr Glu
                   100
<210> 35
   <211> 100
   <212> PRT
   <213> Streptomyces sp. B1003B
<400> 35
   Val Asp Gly Phe Arg Ile Asp Thr Ala Lys His Met Pro Ala Ala Asp
   1 5 10 15
   Leu Ala Asn Ile Lys Ser Arg Leu Gly Asp Pro Asn Val Tyr Trp Lys
   20 25 30
   Gln Glu Ala Ile Tyr Gly Ala Gly Glu Ala Val Ser Pro Asp Glu Tyr
   35 40 45
   Thr Gly Thr Gly Asp Val Gln Glu Phe Arg Tyr Ala Arg Gly Leu Lys
   50 55 60
   Gln Val Phe Asn Asn Glu Asn Leu Ala Tyr Leu Lys Asn Tyr Gly Glu
   65 70 75 80
   Gly Trp Gly Phe Met Pro Ser Ser Lys Ala Ala Val Tyr Val Asp Asn
   85 90 95
   His Asp Thr Glu
   100
<210> 36
   <211> 97
   <212> PRT
   <213> Streptomyces sp. B1006
<400> 36
   Val Asp Gly Phe Arg Ile Asp Ala Ala Lys His Met Ser Ala Ala Asp
   1 5 10 15
   Val Ala Ala Val Lys Gly Lys Met Arg Asp Pro Gly Tyr Trp Val Gln
   20 25 30
   Glu Val Ile Tyr Gly Ala Gly Glu Ala Val Gln Pro Asp Glu Tyr Thr
   35 40 45
   Gly Ile Gly Asp Val Asp Glu Phe Arg Tyr Gly Ser His Leu Lys Ser
   50 55 60
   Ala Phe Gln Gly Gly Asn Ile Ala Gln Leu Lys Ser Val Ala Asp Gly
   65 70 75 80
   Lys Leu Gly Ser Asp Lys Ala Arg Thr Tyr Val Asp Asn His Asp Thr
   85 90 95
   Glu
<210> 37
   <211> 100
   <212> PRT
   <213> Streptomyces sp. B1008A1
<400> 37
   Val Asp Gly Phe Arg Ile Asp Ala Ala Lys His Ile Pro Glu Asp Asp
   1               5                   10                  15
   Leu Ala Ala Ile Lys Ser Arg Leu Ser Asn Pro Gly Val Tyr Trp Lys
               20                  25                  30
   Gln Glu Thr Ile Tyr Gly Ala Gly Glu Ala Val Gln Pro Thr Glu Tyr
           35                  40                  45
   Leu Thr Thr Gly Asp Ala Gln Glu Phe Arg Tyr Ser Trp Asp Leu Lys
       50                  55                  60
   Arg Val Phe Thr Ser Glu Lys Leu Ala Tyr Leu Lys Asn Phe Gly Glu
   65                  70                  75                  80
   Gly Trp Gly Tyr Met Ala Gly Gly Lys Ala Ser Val Tyr Val Asp Asn
                   85                  90                  95
   His Asp Thr Glu
               100
<210> 38
   <211> 100
   <212> PRT
   <213> Streptomyces sp. B1009A
<400> 38
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 39
   <211> 100
   <212> PRT
   <213> Streptomyces sp. B1010
<400> 39
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 40
   <211> 100
   <212> PRT
   <213> Streptomyces sp. B1011
<400> 40
   Val Asp Gly Phe Arg Ile Asp Ala Ala Lys His Ile Pro Ala Ala Asp
   1               5                   10                  15
   Leu Ala Asn Ile Lys Ser Arg Leu Thr Lys Pro Asp Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Arg Asn Glu Arg Leu Ala Tyr Leu Lys Asn Tyr Gly Glu
   65                  70                  75                  80
   Gly Trp Gly Tyr Met Asn Ser Ser Val Ala Gly Val Tyr Val Asp Asn
                   85                  90                  95
   His Asp Thr Glu
               100
<210> 41
   <211> 100
   <212> PRT
   <213> Streptomyces sp. B1012B
<400> 41
   Val Asp Gly Phe Arg Ile Asp Thr Ala Lys His Ile Pro Ala Ala Asp
    1               5                  10                  15
   Leu Ala Asn Ile Lys Ser Arg Leu Thr Lys Pro Asp Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Arg Asn Glu Arg Leu Ala Tyr Leu Lys Asn Tyr Gly Glu
    65                  70                  75                  80
   Gly Trp Gly Tyr Met Asn Ser Ser Val Ala Gly Val Tyr Val Asp Asn
                   85                  90                  95
   His Asp Thr Glu
               100
<210> 42
   <211> 100
   <212> PRT
   <213> Streptomyces sp. B1014A1
<400> 42
   Val Asp Gly Phe Arg Ile Asp Ala Ala Lys His Ile Pro Ala Ala Asp
    1               5                  10                  15
   Leu Ala Asn Ile Lys Ser Arg Leu Thr Lys Pro Asp Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Arg Asn Glu Arg Leu Ala Tyr Leu Lys Asn Tyr Gly Glu
    65                  70                  75                  80
   Gly Trp Gly Tyr Met Asn Ser Ser Val Ala Gly Val Phe Val Asp Asn
                   85                  90                  95
   His Asp Thr Glu
               100
<210> 43
   <211> 100
   <212> PRT
   <213> Streptomyces sp. B1017C
<400> 43
   Val Asp Gly Phe Arg Ile Asp Ala Ala Lys His Ile Pro Ala Ala Asp
   1 5 10 15
   Leu Ala Asn Ile Lys Ser Arg Leu Thr Lys Pro Asp Val Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Arg Asn Glu Arg Leu Ala Tyr Leu Lys Asn Tyr Gly Glu
   65 70 75 80
   Gly Trp Gly Tyr Met Asn Ser Ser Val Ala Gly Val Phe Val Asp Asn
   85 90 95
   His Asp Thr Glu
   100
<210> 44
   <211> 100
   <212> PRT
   <213> Streptomyces sp. B1019
<400> 44
   Val Asp Gly Phe Arg Ile Asp Ala Ala Lys His Ile Pro Ala Ala Asp
   1 5 10 15
   Leu Ala Asn Ile Lys Ser Arg Leu Thr Lys Pro Asp Val Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Arg Asn Glu Lys Leu Ala Tyr Leu Lys Asn Tyr Gly Glu
   65 70 75 80
   Gly Trp Gly Tyr Met Asn Ser Ser Val Ala Gly Val Tyr Val Asp Asn
   85 90 95
   His Asp Thr Glu
   100
<210> 45
   <211> 100
   <212> PRT
   <213> Streptomyces sp. B101A
<400> 45
   Val Asp Gly Phe Arg Ile Asp Thr Ala Lys His Met Ala Ala Ala Asp
   1 5 10 15
   Leu Ala Asp Ile Lys Ser Arg Leu Ser Asp Pro Asp Val Tyr Trp Lys
   20 25 30
   His Glu Ala Ile His Gly Ala Gly Glu Ala Val Ser Pro Ser Glu Tyr
   35 40 45
   Leu Gly Ser Gly Asp Val Gln Glu Phe Arg Tyr Gly Arg Asp Leu Lys
   50 55 60
   Arg Ile Phe Thr Gly Glu Asn Leu Ala Tyr Leu Lys Asn Val Gly Glu
   65 70 75 80
   Ala Trp Gly Tyr Met Pro Ser Asp Lys Ser Asn Val Phe Val Asp Asn
   85 90 95
   His Asp Thr Glu
   100
<210> 46
   <211> 100
   <212> PRT
   <213> Streptomyces sp. B101B
<400> 46
   Val Asp Gly Phe Arg Ile Asp Ala Ala Lys His Met Pro Ala Ala Asp
   1 5 10 15
   Leu Ala Asn Ile Lys Ser Arg Leu Ser Asn Pro Ser Ala Tyr Trp Lys
   20 25 30
   Gln Glu Val Ile Phe Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Met Asn Ser Ser Val Ser Gly Val Tyr Val Asp Asn
   85 90 95
   His Asp Thr Glu
   100
<210> 47
   <211> 84
   <212> PRT
   <213> Streptomyces sp. B102
<400> 47
   Gly Ser Lys His Met Pro Ala Ala Asp Ile Ala Ala Ile Lys Ala Lys
   1 5 10 15
   Leu Asn Arg Ser Ala Tyr Leu Val Gln Glu Val Asn Tyr Gly Ala Gly
   20 25 30
   Glu Pro Ile Gln Pro Thr Glu Tyr Thr Gly Asn Gly Asp Val His Glu
   35 40 45
   Phe Arg Tyr Gly Lys Asp Leu Ala Arg Met Phe Asn Asn Glu Arg Leu
   50 55 60
   Ala Tyr Leu Arg Asn Phe Gly Glu Ser Trp Gly Tyr Leu Ser Ser Ala
   65 70 75 80
   Lys Ala Val Val
<210> 48
   <211> 100
   <212> PRT
   <213> Streptomyces sp. B1020C
<400> 48
   Val Asp Gly Phe Arg Ile Asp Ala Ala Lys His Ile Pro Ala Glu Asp
   1 5 10 15
   Leu Ala Asn Ile Lys Ser Arg Leu Ser Asn Pro Asn Ala Tyr Trp Lys
   20 25 30
   Gln Glu Val Ile Tyr Gly Ala Gly Glu Ala Val Gln Pro Gly Glu Tyr
   35 40 45
   Thr Gly Thr Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Thr Thr Ala Gly Val Phe Val Asp Asn
   85 90 95
   His Asp Thr Glu
   100
<210> 49
   <211> 100
   <212> PRT
   <213> Streptomyces sp. B1022A
<400> 49
   Val Asp Gly Phe Arg Ile Asp Ala Ala Lys His Met Ala Ala Gly Asp
   1               5                   10                  15
   Leu Ala Pro Ile Lys Ser Arg Leu Ser Asn Thr Asn Val Tyr Trp Lys
               20                  25                  30
   Gln Glu Val Ile His Gly Ala Gly Glu Ala Val Gln Pro Gly Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Ser Glu
   65                  70                  75                  80
   Gly Trp Gly Tyr Leu Asn Ser Ser Val Ser Gly Val Tyr Val Asp Asn
                   85                  90                  95
   His Asp Thr Glu
               100
<210> 50
   <211> 100
   <212> PRT
   <213> Streptomyces sp. B1028
<400> 50
   Val Asp Gly Phe Arg Ile Asp Ala Ala Lys His Ile Ala Thr Glu Asp
   1               5                   10                  15
   Leu Ala Ala Val Lys Ala Lys Leu Ser Lys Pro Asp Val Tyr Trp Lys
               20                  25                  30
   Gln Glu Thr Ile Tyr Ser Ala Gly Glu Ala Val Gln Pro Gln Glu Tyr
           35                  40                  45
   Leu Gly Asn Gly Asp Val Gln Glu Phe Arg Tyr Ala Arg Asp Leu Lys
       50                  55                  60
   Arg Val Phe Gln Ser Glu Arg Leu Ala Tyr Leu Lys Asn Tyr Gly Glu
   65                  70                  75                  80
   Gly Trp Gly Tyr Leu Pro Gly Asp Arg Ala Ser Val Tyr Val Asp Asn
                   85                  90                  95
   His Asp Thr Glu
               100
<210> 51
   <211> 100
   <212> PRT
   <213> Streptomyces sp. B1029
<400> 51
   Val Asp Gly Phe Arg Ile Asp Thr Ala Lys His Met Pro Ala Gly Asp
   1               5                   10                  15
   Leu Ala Asn Ile Lys Ser Arg Leu Ser Asn Pro Gly Val Tyr Trp Lys
               20                  25                  30
   Gln Glu Ala Ile Tyr Gly Ala Gly Glu Ala Val Ser Pro Asp Glu Tyr
           35                  40                  45
   Ala Gly Asn Gly Asp Val Gln Glu Phe Arg Tyr Ala Arg Ser Leu Lys
       50                  55                  60
   Gln Val Phe Asn Asn Glu Asn Leu Ala Asn Leu Lys Asn Phe Gly Glu
   65                  70                  75                  80
   Gly Trp Gly Phe Met Pro Ser Ser Lys Ala Ala Val Tyr Val Asp Asn
                   85                  90                  95
   His Asp Thr Glu
               100
<210> 52
   <211> 100
   <212> PRT
   <213> Streptomyces sp. B1030A
<400> 52
   Val Asp Gly Phe Arg Ile Asp Ala Ala Lys His Met Pro Ala Gly Asp
   1               5                   10                  15
   Leu Ala Asn Ile Lys Ser Arg Leu Ser Asn Pro Gly Val Tyr Trp Lys
               20                  25                  30
   Gln Glu Ala Ile Tyr Gly Ala Gly Glu Ala Val Ser Pro Asp Glu Tyr
           35                  40                  45
   Ala Gly Asn Gly Asp Val Gln Glu Phe Arg Tyr Ala Arg Ser Leu Lys
       50                  55                  60
   Gln Val Phe Asn Asn Glu Asn Leu Ala Asn Leu Lys Asn Phe Gly Glu
   65                  70                  75                  80
   Gly Trp Gly Phe Met Pro Ser Ser Lys Ala Ala Val Tyr Val Asp Asn
                   85                  90                  95
   His Asp Thr Glu
               100
<210> 53
   <211> 100
   <212> PRT
   <213> Streptomyces sp. B1035B
<400> 53
   Val Asp Gly Phe Arg Ile Asp Ala Ala Lys His Ile Ala Glu Asp Asp
   1 5 10 15
   Leu Ala Asn Ile Lys Ser Arg Leu Thr Asp Pro Gly Val Tyr Trp Lys
   20 25 30
   Gln Glu Thr Ile Gly Ala Ala Gly Glu Ala Val Gln Pro Ser Glu Tyr
   35 40 45
   Tyr Asn Thr Gly Asp Val Gln Glu Phe His Tyr Ala Tyr Asp Leu Lys
   50 55 60
   Arg Val Phe Thr Gly Glu Lys Leu Ala Glu Leu Lys Asn Phe Gly Glu
   65 70 75 80
   Ala Trp Gly Tyr Val Pro Ser Gly Lys Ala Ser Val Phe Val Asp Asn
   85 90 95
   His Asp Thr Glu
   100
<210> 54
   <211> 100
   <212> PRT
   <213> Streptomyces sp. B1036
<400> 54
   Val Asp Gly Phe Arg Ile Asp Ala Ala Lys His Ile Ala Ala Ala Asp
   1 5 10 15
   Leu Ala Asn Ile Lys Ser Arg Leu Thr Asn Pro Asn Ala Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Arg Tyr Gly Tyr Asp Leu Lys
   50 55 60
   Arg Val Phe Asn Ser Glu Lys Leu Ala Tyr Leu Asn Asn Phe Gly Glu
   65 70 75 80
   Gly Trp Gly Tyr Leu Pro Gly Asn Val Ala Gly Val Tyr Val Asp Asn
   85 90 95
   His Asp Thr Glu
   100
<210> 55
   <211> 100
   <212> PRT
   <213> Streptomyces sp. B1037A
<400> 55
   Val Asp Gly Phe Arg Ile Asp Thr Ala Lys His Ile Ala Ala Ala Asp
   1               5                   10                  15
   Leu Ala Asn Ile Lys Ser Arg Leu Ser Asn Pro Asn Ala Tyr Trp Lys
               20                  25                  30
   Gln Glu Ala Ile Tyr Gly Ala Gly Glu Ala Val Ser Pro Ser Glu Tyr
           35                  40                  45
   Leu Gly Thr Gly Asp Val Gln Glu Phe Arg Tyr Ala Arg Asp Leu Lys
       50                  55                  60
   Arg Val Phe Gln Asn Glu Asn Leu Ala Tyr Leu Lys Asn Tyr Gly Glu
   65                  70                  75                  80
   Gly Trp Gly Tyr Met Ala Ser Gly Gln Ser Ala Val Phe Val Asp Asn
                   85                  90                  95
   His Asp Thr Glu
               100
<210> 56
   <211> 100
   <212> PRT
   <213> Streptomyces sp. B1039A
<400> 56
   Val Asp Gly Phe Arg Ile Asp Ala Ala Lys His Met Ala Ala Gly Asp
   1               5                   10                  15
   Leu Ala Ala Ile Lys Ser Arg Leu Thr Asn Pro Asn Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Phe Gly Ser Glu Asn Leu Ala Tyr Leu Lys Asn Phe Gly Glu
   65                  70                  75                  80
   Ser Trp Gly Tyr Met Pro Ser Gly Gln Ser Ala Val Tyr Val Asp Asn
                   85                  90                  95
   His Asp Thr Glu
               100

<210> 57
   <211> 100
   <212> PRT
   <213> Streptomyces sp. B103A
<400> 57
   Val Asp Gly Phe Arg Ile Asp Ala Ala Lys His Met Pro Ala Ala Asp
   1               5                   10                  15
   Leu Ala Asn Ile Lys Ser Arg Leu Ser Asn Pro Gly Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Gly Arg Gly Leu Lys
       50                  55                  60
   Gln Val Phe Asn Asn Glu Asn Leu Ala His Leu Lys Asn Phe Gly Glu
   65                  70                  75                  80
   Gly Trp Gly Phe Met Glu Ser Gly Lys Ser Ala Val Phe Val Asp Asn
                   85                  90                  95
   His Asp Thr Glu
               100
<210> 58
   <211> 100
   <212> PRT
   <213> Streptomyces sp. B1041A1
<400> 58
   Val Asp Gly Phe Arg Ile Asp Ala Ala Lys His Ile Pro Ala Ala Asp
   1               5                   10                  15
   Leu Ala Asn Ile Lys Ser Arg Leu Thr Lys Pro Asp Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Arg Asn Glu Arg Leu Ala Tyr Leu Lys Asn Tyr Gly Glu
   65                  70                  75                  80
   Gly Trp Gly Tyr Met Asn Ser Ser Val Ala Gly Val Phe Val Asp Asn
                   85                  90                  95
   His Asp Thr Glu
               100
<210> 59
   <211> 100
   <212> PRT
   <213> Streptomyces sp. B1043A
<400> 59
   Val Asp Gly Phe Arg Ile Asp Ala Ala Lys His Met Ala Ala Ala Asp
   1               5                   10                  15
   Leu Ala Ala Ile Lys Ser Arg Leu Ser Asn Pro Asn Val Tyr Trp Lys
               20                  25                  30
   His Glu Ala Ile Tyr Gly Ala Gly Glu Thr Val Ser Pro Thr Glu Tyr
           35                  40                  45
   Val Gly Ser Gly Asp Val Gln Glu Phe Arg Tyr Ala Arg Asp Leu Lys
       50                  55                  60
   Arg Val Phe Asn Gly Glu Asn Leu Ala Tyr Leu Lys Asn Phe Gly Glu
   65                  70                  75                  80
   Ala Trp Gly His Leu Pro Ser Asp Glu Ala Ala Val Tyr Val Asp Asn
                   85                  90                  95
   His Asp Thr Glu
               100
<210> 60
   <211> 100
   <212> PRT
   <213> Streptomyces sp. B1044C
<400> 60
   Val Asp Gly Phe Arg Ile Asp Ala Ala Lys His Ile Ala Thr Glu Asp
   1               5                   10                  15
   Leu Ala Ala Val Lys Ala Lys Leu Ser Lys Pro Asp Val Tyr Trp Lys
               20                  25                  30
   Gln Glu Thr Ile Tyr Gly Ala Gly Glu Ala Val Gln Pro Gln Glu Tyr
           35                  40                  45
   Leu Gly Asn Gly Asp Val Gln Glu Phe Arg Tyr Ala Arg Asp Leu Lys
       50                  55                  60
   Arg Val Phe Gln Ser Glu Arg Leu Ala Tyr Leu Lys Asn Tyr Gly Glu
   65                  70                  75                  80
   Gly Trp Gly Tyr Leu Pro Gly Asp Arg Ala Ser Val Tyr Val Asp Asn
                   85                  90                  95
   His Asp Thr Glu
               100
<210> 61
   <211> 100
   <212> PRT
   <213> Streptomyces sp. B1045
<400> 61
   Val Asp Gly Phe Arg Ile Asp Thr Ala Lys His Ile Pro Ala Thr Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Ser Val Ala Gly Val Phe Val Asp Asn
   85 90 95
   His Asp Thr Glu
   100
<210> 62
   <211> 100
   <212> PRT
   <213> Streptomyces sp. B1046A
<400> 62
   Val Asp Gly Phe Arg Ile Asp Ala Ala Lys His Met Ala Ala Ala Asp
   1 5 10 15
   Leu Ala Asn Ile Lys Ser Arg Leu Thr Asp Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asp Leu Ala Tyr Leu Lys Asn Phe Gly Glu
   65 70 75 80
   Ala Trp Gly Tyr Leu Pro Ser Asp Gln Ala Ala Val Phe Val Asp Asn
   85 90 95
   His Asp Thr Glu
   100
<210> 63
   <211> 100
   <212> PRT
   <213> Streptomyces sp. B1047A1
<400> 63
   Val Asp Gly Phe Arg Ile Asp Ala Ala Lys His Met Ala Ala Ala Asp
   1 5 10 15
   Leu Ala Ala Ile Lys Ser Arg Leu Ser Asn Pro Asn Val Tyr Trp Lys
   20 25 30
   His Glu Ala Met Tyr Gly Ala Gly Glu Ala Val Ser Pro Thr Glu Tyr
   35 40 45
   Val Gly Ser Gly Asp Val Gln Glu Phe Arg Tyr Ala Arg Asp Leu Lys
   50 55 60
   Arg Val Phe Asn Gly Glu Asn Leu Ala Tyr Leu Lys Asn Phe Gly Glu
   65 70 75 80
   Ala Trp Gly His Leu Pro Ser Asp Glu Ala Ala Val Tyr Val Asp Asn
   85 90 95
   His Asp Thr Glu
   100
<210> 64
   <211> 100
   <212> PRT
   <213> Streptomyces sp. B1048A
<400> 64
   Val Asp Gly Phe Arg Ile Asp Thr Ala Lys His Met Xaa Ala Ala Asp
   1 5 10 15
   Leu Ala Ala Ile Lys Ser Arg Leu Ser Asn Pro Asn Val Tyr Trp Lys
   20 25 30
   His Glu Ala Met Tyr Gly Ala Gly Glu Ala Val Ser Pro Thr Glu Tyr
   35 40 45
   Val Gly Ser Gly Asp Val Gln Glu Phe Arg Tyr Ala Arg Asp Leu Lys
   50 55 60
   Arg Val Phe Asn Gly Glu Asn Leu Ala Tyr Leu Lys Asn Phe Gly Glu
   65 70 75 80
   Ala Trp Gly His Leu Pro Ser Asp Glu Ala Ala Val Phe Val Asp Asn
   85 90 95
   His Asp Thr Glu
   100
<210> 65
   <211> 100
   <212> PRT
   <213> Streptomyces sp. B1049A
<400> 65
   Val Asp Gly Phe Arg Ile Asp Ala Ala Lys His Met Ala Ala Gly Asp
   1               5                   10                  15
   Leu Ala Ala Ile Lys Ser Arg Leu Thr Asn Pro Asn Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Phe Gly Ser Glu Asn Leu Ala Tyr Leu Lys Asn Phe Gly Glu
   65                  70                  75                  80
   Ser Trp Gly Tyr Met Pro Ser Gly Gln Ser Ala Val Tyr Val Asp Asn
                   85                  90                  95
   His Asp Thr Glu
               100
<210> 66
   <211> 100
   <212> PRT
   <213> Streptomyces sp. B1050A
<400> 66
   Val Asp Gly Phe Arg Ile Asp Thr Ala Lys His Met Ala Ala Gly Asp
   1               5                   10                  15
   Leu Ala Ala Ile Lys Ser Arg Leu Thr Asn Pro Asn Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Phe Gly Ser Glu Asn Leu Ala Tyr Leu Lys Asn Phe Gly Glu
   65                  70                  75                  80
   Ser Trp Gly Tyr Met Pro Ser Gly Gln Ser Ala Val Tyr Val Asp Asn
                   85                  90                  95
   His Asp Thr Glu
               100
<210> 67
   <211> 100
   <212> PRT
   <213> Streptomyces sp. B1052A2
<400> 67
   Val Asp Gly Phe Arg Ile Asp Thr Ala Lys His Met Ala Ala Gly Asp
   1               5                   10                  15
   Leu Ala Asn Ile Lys Ser Arg Leu Thr Asn Pro Asn Val Tyr Trp Lys
               20                  25                  30
   His Glu Ala Ile Tyr Gly Ala Gly Glu Ala Val Ser Pro Ala Glu Tyr
           35                  40                  45
   Leu Gly Ser Gly Asp Val Gln Glu Phe Arg Tyr Ala Arg Asp Leu Lys
       50                  55                  60
   Arg Val Phe Asn Asn Glu Asn Leu Ala Tyr Leu Lys Asn Phe Gly Glu
   65                  70                  75                  80
   Ser Trp Gly Tyr Leu Pro Ser Asp Gln Ala Ala Val Tyr Val Asp Asn
                   85                  90                  95
   His Asp Thr Glu
               100
<210> 68
   <211> 100
   <212> PRT
   <213> Streptomyces sp. B1053
<400> 68
   Val Asp Gly Phe Arg Ile Asp Thr Ala Lys His Met Pro Ala Ala Asp
   1               5                   10                  15
   Leu Thr Ala Ile Lys Ala Lys Val Gly Asn Gly Ser Thr Tyr Trp Lys
               20                  25                  30
   Gln Glu Ala Ile His Gly Ala Gly Glu Ala Val Gln Pro Ser Glu Tyr
           35                  40                  45
   Leu Gly Thr Gly Asp Val Gln Glu Phe Arg Tyr Ala Arg Asp Leu Lys
       50                  55                  60
   Arg Val Phe Gln Asn Glu Asn Leu Ala His Leu Lys Xaa Phe Gly Glu
   65                  70                  75                  80
   Asp Trp Gly Tyr Met Ala Ser Gly Lys Ser Ala Val Tyr Val Asp Asn
                   85                  90                  95
   His Asp Thr Glu
               100
<210> 69
   <211> 97
   <212> PRT
   <213> Streptomyces sp. B1059
<400> 69
   Val Asp Gly Phe Arg Ile Asp Ala Ala Lys His Met Ser Ala Asp Asp
   1               5                   10                  15
   Val Ala Ala Ile Lys Gly Lys Met Ser Asp Pro Gly Phe Trp Val Thr
               20                  25                  30
   Glu Val Ile His Gly Gly Gly Glu Ala Val Gln Pro Glu Glu Tyr Thr
           35                  40                  45
   Ser Ile Gly Asp Val Asp Glu Phe Arg Tyr Gly Gly His Leu Lys Ser
       50                  55                  60
   Ala Phe Gln Gly Gly Gly Leu Pro Gly Leu Lys Ser Ile Ala Asp Gly
   65                  70                  75                  80
   Lys Leu Ala Gly Ala Ser Ala Arg Thr Tyr Val Asp Asn His Asp Thr
                   85                  90                  95
   Glu
<210> 70
   <211> 100
   <212> PRT
   <213> Streptomyces sp. B1060
<400> 70
   Val Asp Gly Phe Arg Ile Asp Ala Ala Lys His Met Pro Ala Gly Asp
   1               5                   10                  15
   Leu Ala Asn Ile Lys Ser Arg Leu Ser Asn Pro Asn Val Tyr Trp Lys
               20                  25                  30
   His Glu Ala Ile Phe Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Ala Val Tyr Val Asp Asn
                   85                  90                  95
   His Asp Thr Glu
               100
<210> 71
   <211> 100
   <212> PRT
   <213> Streptomyces sp. B1061B
<400> 71
   Val Asp Gly Phe Arg Ile Asp Thr Ala Lys His Met Pro Ala Ala Asp
   1 5 10 15
   Leu Ala Asn Ile Lys Ser Arg Pro Ser Asn Pro Gly Val Tyr Trp Lys
   20 25 30
   His Glu Ala Ile Tyr Gly Ala Gly Glu Ala Val Ser Pro Ser Glu Tyr
   35 40 45
   Leu Gly Ser Gly Asp Val Gln Glu Phe Arg Tyr Gly Arg Gly Leu Lys
   50 55 60
   Gln Val Phe Asn Asn Glu Asn Leu Ala Tyr Leu Lys Asn Phe Gly Glu
   65 70 75 80
   Gly Trp Gly Phe Met Glu Ser Gly Arg Ser Ala Val Tyr Val Asp Asn
   85 90 95
   His Asp Thr Glu
   100
<210> 72
   <211> 100
   <212> PRT
   <213> Streptomyces sp. B1065
<400> 72
   Val Asp Gly Phe Arg Ile Asp Thr Ala Lys His Met Pro Ala Gly Asp
   1 5 10 15
   Leu Ala Asn Ile Lys Ser Arg Leu Ser Asn Pro Asn Val Tyr Trp Lys
   20 25 30
   His Glu Ala Ile Phe Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 73
   <211> 100
   <212> PRT
   <213> Streptomyces sp. B1067A
<400> 73
   Val Asp Gly Phe Arg Ile Asp Thr Ala Lys His Met Pro Ala Ala Asp
   1               5                   10                  15
   Leu Ala Asn Ile Lys Ser Arg Leu Ser Asn Pro Gly Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Gly Arg Gly Leu Lys
       50                  55                  60
   Gln Val Phe Asn Asn Glu Asn Leu Ala Tyr Leu Lys Asn Phe Gly Glu
   65                  70                  75                  80
   Gly Trp Gly Phe Met Glu Ser Gly Arg Ser Ala Val Tyr Val Asp Asn
                   85                  90                  95
   His Asp Thr Glu
               100
<210> 74
   <211> 100
   <212> PRT
   <213> Streptomyces sp. B1068
<400> 74
   Val Asp Gly Phe Arg Ile Asp Thr Ala Lys His Met Pro Ala Gly Asp
   1               5                   10                  15
   Leu Ala Asn Ile Lys Ser Arg Leu Ser Asn Pro Asn Val Tyr Trp Lys
               20                  25                  30
   His Glu Ala Ile Phe Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Ala Val Tyr Val Asp Asn
                   85                  90                  95
   His Asp Thr Glu
               100
<210> 75
   <211> 100
   <212> PRT
   <213> Streptomyces sp. B1069B
<400> 75
   Val Asp Gly Phe Arg Ile Asp Thr Ala Lys His Met Pro Ala Ala Asp
   1               5                   10                  15
   Leu Thr Ala Ile Lys Ala Lys Val Gly Asp Gly Gly Thr Tyr Trp Lys
               20                  25                  30
   Gln Glu Ala Ile His Gly Ala Gly Glu Ala Val Gln Pro Ser Glu Tyr
           35                  40                  45
   Leu Gly Thr Gly Asp Val Gln Glu Phe Arg Tyr Ala Arg Asp Leu Lys
       50                  55                  60
   Arg Val Phe Gln Asn Glu Asn Leu Ala His Leu Lys Asn Phe Asp Glu
   65                  70                  75                  80
   Asp Trp Gly His Met Gln Ser Gly Arg Ser Ala Val Tyr Val Asp Asn
                   85                  90                  95
   His Asp Thr Glu
               100
<210> 76
   <211> 100
   <212> PRT
   <213> Streptomyces sp. B106C
<400> 76
   Val Asp Gly Phe Arg Ile Asp Ala Ala Lys His Met Pro Ala Ala Asp
   1               5                   10                  15
   Leu Ala Asn Ile Lys Ser Arg Leu Thr Asp Pro Gly Val Tyr Trp Lys
               20                  25                  30
   Gln Glu Val Ile Phe Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Lys Ser Ser Ser Ala Gly Val Tyr Val Asp Asn
                   85                  90                  95
   His Asp Thr Glu
               100
<210> 77
   <211> 100
   <212> PRT
   <213> Streptomyces sp. B107
<400> 77
   Val Asp Gly Phe Arg Ile Asp Ala Ala Lys His Met Pro Ala Ala Asp
   1               5                   10                  15
   Leu Ala Asn Ile Lys Pro Arg Leu Thr Asn Pro Ser Ala Tyr Trp Lys
               20                  25                  30
   Gln Glu Val Ile Tyr Gly Ala Gly Glu Ala Val Gln Pro Gly Glu Tyr
           35                  40                  45
   Thr Gly Thr Gly Asp Val Gln Glu Phe Arg Tyr Ala Tyr Asp Leu Lys
       50                  55                  60
   Arg Val Phe Asn Asn Glu Ser Leu Ala Tyr Leu Lys Asn Tyr Gly Glu
   65                  70                  75                  80
   Gly Trp Gly Tyr Leu Lys Ser Ser Ser Ala Gly Val Tyr Val Asp Asn
                   85                  90                  95
   His Asp Thr Glu
               100
<210> 78
   <211> 100
   <212> PRT
   <213> Streptomyces sp. B1070A
<400> 78
   Val Asp Gly Phe Arg Ile Asp Thr Ala Lys His Ile Pro Ala Ala Asp
   1               5                   10                  15
   Leu Ala Asn Ile Lys Ser Arg Leu Thr Asn Pro Ser Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Ser Ser Ser Val Ala Gly Val Tyr Val Asp Asn
                   85                  90                  95
   His Asp Thr Glu
               100
<210> 79
   <211> 100
   <212> PRT
   <213> Streptomyces sp. B1071
<400> 79
   Val Asp Gly Phe Arg Ile Asp Thr Ala Lys His Met Pro Ala Gly Asp
   1 5 10 15
   Leu Ala Asn Ile Lys Ser Arg Leu Ser Asn Pro Asn Val Tyr Trp Lys
   20 25 30
   His Glu Ala Ile Phe Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 80
   <211> 100
   <212> PRT
   <213> Streptomyces sp. B1072A
<400> 80
   Val Asp Gly Phe Arg Ile Asp Ala Ala Lys His Ile Pro Ala Ala Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Ser Val Ala Gly Val Phe Val Asp Asn
   85 90 95
   His Asp Thr Glu
   100
<210> 81
   <211> 100
   <212> PRT
   <213> Streptomyces sp. B108
<400> 81
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Ser Val Phe Trp Lys
   20 25 30
   Met Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Val Leu Gln Asn Glu Lys Leu Ala His Leu Lys Asn Phe Gly Glu
   65 70 75 80
   Ser Trp Gly Tyr Met Pro Ser Gly Gln Ser Gly Val Tyr Val Asp Asn
   85 90 95
   His Asp Thr Glu
   100
<210> 82
   <211> 100
   <212> PRT
   <213> Streptomyces sp. B109A
<400> 82
   Val Asp Gly Phe Arg Ile Asp Ala Ala Lys His Met Pro Ala Ala Asp
   1 5 10 15
   Leu Ala Asn Ile Lys Ser Arg Leu Thr Lys Pro Gly Val Tyr Trp Lys
   20 25 30
   Gln Glu Ala Ile Tyr Gly Ala Gly Glu Ala Val Gln Pro Ser Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Asn Ser Gly Val Ser Gly Val Tyr Val Asp Asn
   85 90 95
   His Asp Thr Glu
   100
<210> 83
   <211> 100
   <212> PRT
   <213> Streptomyces sp. B114C
<400> 83
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 84
   <211> 100
   <212> PRT
   <213> Streptomyces sp. B115
<400> 84
   Val Asp Gly Phe Arg Ile Asp Ala Ala Lys His Ile Pro Ala Ala Asp
   1 5 10 15
   Leu Ala Asp Ile Lys Ser Arg Leu Thr Asn Pro Ser Ala Tyr Trp Lys
   20 25 30
   Gln Glu Val Ile Phe Gly Ala Gly Glu Ala Val Gln Pro Thr Glu Tyr
   35 40 45
   Thr Gly Ala Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Asn Ser Gly Ser Ala Gly Val Phe Val Asp Asn
   85 90 95
   His Asp Thr Glu
   100
<210> 85
   <211> 100
   <212> PRT
   <213> Streptomyces sp. B117A1
<400> 85
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Ser Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 86
   <211> 100
   <212> PRT
   <213> Streptomyces sp. B118
<400> 86
   Val Asp Gly Phe Arg Ile Asp Ala Ala Lys His Met Ala Val Gly Asp
   1 5 10 15
   Leu Ala Asp Ile Lys Ser Arg Leu Gly Asn Pro Asp Val His Trp Lys
   20 25 30
   His Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Val Leu Thr Gly Gly Ser Leu Ala His Leu Arg Asn Phe Gly Glu
   65 70 75 80
   Gly Trp Gly Tyr Met Ala Ser Asp Arg Ser Asn Val Phe Val Asp Asn
   85 90 95
   His Asp Thr Glu
   100
<210> 87
   <211> 100
   <212> PRT
   <213> Streptomyces sp. B119E
<400> 87
   Val Asp Gly Phe Arg Ile Asp Ala Ala Lys His Ile Pro Glu Asp Asp
   1 5 10 15
   Leu Ala Ala Ile Lys Ser Arg Leu Ser Asn Gln Gly Val Tyr Trp Lys
   20 25 30
   Gln Glu Thr Ile Tyr Gly Ala Gly Glu Ala Val Gln Pro Thr Glu Tyr
   35 40 45
   Leu Thr Thr Gly Asp Ala Gln Glu Leu Arg Tyr Ser Trp Asp Leu Lys
   50 55 60
   Arg Val Phe Thr Ser Glu Lys Leu Ala Tyr Leu Lys Asn Phe Gly Glu
   65 70 75 80
   Gly Trp Gly Tyr Met Ala Gly Gly Lys Ala Ser Val Tyr Val Asp Asn
   85 90 95
   His Asp Thr Glu
   100

<210> 88
   <211> 100
   <212> PRT
   <213> Streptomyces sp. B120alt
<400> 88
   Val Asp Gly Phe Arg Ile Asp Ala Ala Lys His Ile Pro Ala Ala Asp
   1 5 10 15
   Leu Ala Asn Ile Lys Ser Arg Leu Thr Asn Pro Ser Ala Tyr Trp Lys
   20 25 30
   His Glu Val Ile Phe Gly Ala Gly Glu Ala Val Gln Pro Thr Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Thr Ser Gly Ser Ala Gly Val Tyr Val Asp Asn
   85 90 95
   His Asp Thr Glu
   100
<210> 89
   <211> 100
   <212> PRT
   <213> Streptomyces sp. B123
<400> 89
   Val Asp Gly Phe Arg Ile Asp Thr Ala Lys His Met Pro Ala Ala Asp
   1 5 10 15
   Leu Thr Ala Ile Lys Ala Xaa Val Gly Asp Gly Gly Thr Tyr Trp Lys
   20 25 30
   Gln Glu Ala Ile His Gly Ala Gly Glu Ala Val Gln Pro Ser Glu Tyr
   35 40 45
   Leu Gly Thr Cys Asp Val Gln Glu Phe Arg Tyr Ala Arg Asp Leu Lys
   50 55 60
   Arg Val Phe Gln Asn Glu Asn Leu Ala His Leu Lys Asn Phe Gly Glu
   65 70 75 80
   Asp Trp Gly His Met Gln Ser Gly Arg Ser Ala Val Phe Val Asp Asn
   85 90 95
   His Asp Thr Glu
   100
<210> 90
   <211> 100
   <212> PRT
   <213> Streptomyces sp. B124
<400> 90
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Phe Val Asp Asn
   85 90 95
   His Asp Thr Glu
   100
<210> 91
   <211> 100
   <212> PRT
   <213> Streptomyces sp. B125C
<400> 91
   Val Asp Gly Phe Arg Ile Asp Ala Ala Lys His Met Ala Ala Gly Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Phe Gly Glu
   65 70 75 80
   Ser Trp Gly Tyr Leu Pro Ser Asp Gln Ala Ala Val Tyr Val Asp Asn
   85 90 95
   His Asp Thr Glu
   100
<210> 92
   <211> 100
   <212> PRT
   <213> Streptomyces sp. B126A
<400> 92
   Val Asp Gly Phe Arg Ile Asp Thr Ala Lys His Ile Pro Ala Ala Asp
   1 5 10 15
   Leu Ala Asp Ile Lys Ser Arg Leu Thr Asn Pro Ser Ala Tyr Trp Lys
   20 25 30
   His Glu Val Ile Phe Gly Ala Gly Glu Ala Val Gln Pro Thr Glu Tyr
   35 40 45
   Thr Gly Ala Gly Asp Val Gln Glu Phe Arg Tyr Ala Tyr Asp Leu Lys
   50 55 60
   Arg Val Phe Asn Asn Glu Asn Leu Thr Tyr Leu Lys Asn Tyr Gly Glu
   65 70 75 80
   Gly Trp Gly Tyr Leu Asn Ser Gly Phe Ala Gly Val Tyr Val Asp Asn
   85 90 95
   His Asp Thr Glu
   100
<210> 93
   <211> 100
   <212> PRT
   <213> Streptomyces sp. B127A
<400> 93
   Val Asp Gly Phe Arg Ile Asp Thr Ala Lys His Met Pro Ala Ala Asp
    1              5                   10                  15
   Leu Ala Asn Ile Lys Ser Arg Leu Thr Lys Pro Gly Val Tyr Trp Lys
               20                  25                  30
   Gln Glu Ala Ile Tyr Gly Ala Gly Glu Ala Val Gln Pro Ser Glu Tyr
           35                  40                  45
   Thr Gly Thr Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Asn Ser Gly Val Ser Gly Val Tyr Val Asp Asn
                   85                  90                  95
   His Asp Thr Glu
               100
<210> 94
   <211> 100
   <212> PRT
   <213> Streptomyces sp. B128B
<400> 94
   Val Asp Gly Phe Arg Ile Asp Thr Ala Lys His Ile Pro Ala Ala Asp
    1              5                   10                  15
   Leu Ala Asn Ile Lys Ser Arg Leu Thr Asn Pro Ser Ala Tyr Trp Lys
               20                  25                  30
   His Glu Val Ile Phe Gly Ala Gly Glu Ala Val Gln Pro Thr Glu Tyr
           35                  40                  45
   Thr Gly Thr Gly Asp Val Gln Glu Phe Arg Tyr Ala Tyr Asp Leu Lys
       50                  55                  60
   Arg Val Phe Asn Xaa Glu Asn Leu Ala Tyr Leu Lys Asn Tyr Gly Glu
   65                  70                  75                  80
   Gly Trp Gly Tyr Leu Thr Ser Gly Ser Ala Gly Val Tyr Val Asp Asn
                   85                  90                  95
   His Asp Thr Glu
               100
<210> 95
   <211> 100
   <212> PRT
   <213> Streptomyces sp. B130B
<400> 95
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Asn Ala Tyr Trp Lys
   20 25 30
   Leu Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Val Leu Gln Ser Glu Lys Leu Ala His Leu Lys Asn Phe Gly Glu
   65 70 75 80
   Ala Trp Gly Tyr Met Pro Ser Ala Gln Ser Gly Val Phe Val Asp Asn
   85 90 95
   His Asp Thr Glu
   100
<210> 96
   <211> 100
   <212> PRT
   <213> Streptomyces sp. B131
<400> 96
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Ser Val Phe Trp Lys
   20 25 30
   Met Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Val Leu Gln Asn Glu Lys Leu Ala His Leu Lys Asn Phe Gly Glu
   65 70 75 80
   Ser Trp Gly Tyr Met Pro Ser Gly Gln Ser Gly Val Tyr Val Asp Asn
   85 90 95
   His Asp Thr Glu
   100
<210> 97
   <211> 100
   <212> PRT
   <213> Streptomyces sp. B134
<400> 97
   Val Asp Gly Phe Arg Ile Asp Ala Ala Lys His Met Pro Ala Ala Asp
   1               5                   10                  15
   Leu Ala Asn Ile Lys Ser Arg Leu Thr Asn Pro Asn Ala Tyr Trp Lys
               20                  25                  30
   Leu Glu Ala Ile His Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Ile Val Gln Ser Glu Lys Leu Ala Tyr Leu Lys Asn Phe Gly Glu
   65                  70                  75                  80
   Ala Trp Gly Tyr Met Pro Ser Gly Gln Ser Gly Val Tyr Val Asp Asn
                   85                  90                  95
   His Asp Thr Glu
               100
<210> 98
   <211> 100
   <212> PRT
   <213> Streptomyces sp. B135A
<400> 98
   Val Asp Gly Phe Arg Ile Asp Ala Ala Lys His Ile Pro Ala Ala Asp
   1               5                   10                  15
   Leu Ala Asn Ile Lys Ser Arg Leu Ser Asp Pro Gly Val Tyr Trp Lys
               20                  25                  30
   His Glu Val Ile His Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Ser Ser Gly Ala Ala Gly Val Tyr Val Asp Asn
                   85                  90                  95
   His Asp Thr Glu
               100
<210> 99
   <211> 100
   <212> PRT
   <213> Streptomyces sp. B137
<400> 99
   Val Asp Gly Phe Arg Ile Asp Ala Ala Lys His Met Pro Ala Gly Asp
   1 5 10 15
   Pro Ala Asn Ile Lys Ser Arg Leu Ser Asn Pro Asn Val Tyr Trp Lys
   20 25 30
   His Glu Ala Ile Phe Gly Ala Gly Glu Ala Val Ser Pro Ser Glu His
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 100
   <211> 100
   <212> PRT
   <213> Streptomyces sp. B138
<400> 100
   Val Asp Gly Phe Arg Ile Asp Thr Ala Lys His Met Pro Ala Ala Asp
   1 5 10 15
   Leu Ala Asn Ile Lys Ser Arg Leu Ser Asn Pro Asp Ala Tyr Trp Lys
   20 25 30
   Gln Glu Val Ile His Gly Ala Gly Glu Ala Val Gln Pro Gly Glu Tyr
   35 40 45
   Thr Gly Asn Gly Asp Val Gln Glu Phe Arg Tyr Ala Tyr Asp Leu Lys
   50 55 60
   Arg Val Phe His Asn Glu Asn Leu Ala Tyr Leu Lys Asn Tyr Gly Glu
   65 70 75 80
   Gly Trp Gly Tyr Leu Lys Asn Ser Ser Ala Gly Val Tyr Val Asp Asn
   85 90 95
   His Asp Thr Glu
   100
<210> 101
   <211> 100
   <212> PRT
   <213> Streptomyces sp. B138A
<400> 101
   Val Asp Gly Phe Arg Ile Asp Thr Ala Lys His Met Pro Ala Ala Asp
   1               5                   10                  15
   Leu Ala Asn Ile Lys Ser Arg Leu Ser Asn Pro Asp Ala Tyr Trp Lys
               20                  25                  30
   Gln Glu Val Ile His Gly Ala Gly Glu Ala Val Gln Pro Gly Glu Tyr
           35                  40                  45
   Thr Gly Asn Gly Asp Val Gln Glu Phe Arg Tyr Ala Tyr Asp Leu Lys
       50                  55                  60
   Arg Val Phe His Asn Glu Asn Leu Ala Tyr Leu Lys Asn Tyr Gly Glu
   65                  70                  75                  80
   Gly Trp Gly Tyr Leu Lys Asn Ser Ser Ala Gly Val Tyr Val Asp Asn
                   85                  90                  95
   His Asp Thr Glu
               100
<210> 102
   <211> 100
   <212> PRT
   <213> Streptomyces sp. B138A2
<400> 102
   Val Asp Gly Phe Arg Ile Asp Ala Ala Lys His Met Pro Ala Ala Asp
   1               5                   10                  15
   Leu Ala Asn Ile Lys Ser Arg Leu Ser Asn Pro Asp Ala Tyr Trp Lys
               20                  25                  30
   Gln Glu Val Ile His Gly Ala Gly Glu Ala Val Gln Pro Gly Glu Tyr
           35                  40                  45
   Thr Gly Asn Gly Asp Val Gln Glu Phe Arg Tyr Ala Tyr Asp Leu Lys
       50                  55                  60
   Arg Val Phe His Asn Glu Asn Leu Ala Tyr Leu Lys Asn Tyr Gly Glu
   65                  70                  75                  80
   Gly Trp Gly Tyr Leu Lys Asn Ser Ser Ala Gly Val Tyr Val Asp Asn
                   85                  90                  95
   His Asp Thr Glu
               100
<210> 103
   <211> 100
   <212> PRT
   <213> Streptomyces sp. B140
<400> 103
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Asn Ala Tyr Trp Lys
   20 25 30
   Leu Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Ile Val Gln Ser Glu Lys Leu Ala Tyr Leu Lys Asn Phe Gly Glu
   65 70 75 80
   Ala Trp Gly Tyr Met Pro Ser Gly Gln Ser Gly Val Tyr Val Asp Asn
   85 90 95
   His Asp Thr Glu
   100
<210> 104
   <211> 100
   <212> PRT
   <213> Streptomyces sp. B141
<400> 104
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Asn Ala Tyr Trp Lys
   20 25 30
   Leu Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Ile Val Gln Ser Glu Lys Leu Ala Tyr Leu Lys Asn Phe Gly Glu
   65 70 75 80
   Ala Trp Gly Tyr Met Pro Ser Gly Gln Ser Gly Val Tyr Val Asp Asn
   85 90 95
   His Asp Thr Glu
   100
<210> 105
   <211> 100
   <212> PRT
   <213> Streptomyces sp. B142
<400> 105
   Val Asp Gly Phe Arg Ile Asp Ala Ser Lys His Met Pro Ala Asp Asp
   1 5 10 15
   Leu Ala Ala Ile Lys Gly Lys Leu Ala Asn Pro Gly Val Tyr Trp Lys
   20 25 30
   His Glu Ala Ile His Gly Ala Gly Glu Ala Val Ser Pro Ser Glu Tyr
   35 40 45
   Leu Gly Ser Gly Asp Val Gln Glu Phe Arg Tyr Gly Arg Gly Leu Lys
   50 55 60
   Gln Val Phe Thr Gly Gly Ser Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 106
   <211> 100
   <212> PRT
   <213> Streptomyces sp. B143
<400> 106
   Val Asp Gly Phe Arg Ile Asp Ala Ala Lys His Met Pro Ala Ala Gly
   1 5 10 15
   Leu Ala Asn Ile Lys Ser Arg Leu Thr Asn Pro Asn Ala Tyr Trp Lys
   20 25 30
   Leu Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Ile Val Gln Ser Glu Lys Leu Ala Tyr Leu Lys Asn Phe Gly Glu
   65 70 75 80
   Ala Trp Gly Tyr Met Pro Ser Gly Gln Ser Gly Val Phe Val Asp Asn
   85 90 95
   His Asp Thr Glu
   100
<210> 107
   <211> 100
   <212> PRT
   <213> Streptomyces sp. B148A
<400> 107
   Val Asp Gly Phe Arg Ile Asp Thr Ala Lys His Ile Pro Ala Ala Asp
   1               5                   10                  15
   Leu Ala Asp Ile Lys Ser Arg Leu Thr Asn Pro Ser Ala Tyr Trp Lys
               20                  25                  30
   Gln Glu Val Ile Xaa Gly Ala Gly Glu Ala Val Gln Pro Thr Glu Tyr
           35                  40                  45
   Thr Gly Ala Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Asn Ser Gly Phe Ala Gly Val Tyr Val Asp Asn
                   85                  90                  95
   His Asp Thr Glu
               100
<210> 108
   <211> 100
   <212> PRT
   <213> Streptomyces sp. B152A
<400> 108
   Val Asp Gly Phe Arg Ile Asp Thr Ala Lys His Met Pro Ala Ala Asp
   1               5                   10                  15
   Leu Ala Asn Ile Lys Ser Arg Leu Ser Asn Pro Gly Val Tyr Trp Lys
               20                  25                  30
   Gln Glu Ala Ile His Gly Ala Gly Glu Ala Val Ser Pro Ser Glu Tyr
           35                  40                  45
   Leu Gly Asn Gly Asp Val Gln Glu Phe Arg Tyr Ala Arg Asp Leu Lys
       50                  55                  60
   Arg Ile Phe Gly Ser Glu Lys Leu Ser His Leu Ser Thr Phe Gly Glu
   65                  70                  75                  80
   Ser Trp Gly Tyr Met Ala Ser Gly Arg Ser Gly Val Tyr Val Asp Asn
                   85                  90                  95
   His Asp Thr Glu
               100
<210> 109
   <211> 100
   <212> PRT
   <213> Streptomyces sp. B153(B)
<400> 109
   Val Asp Gly Phe Arg Ile Asp Thr Ala Lys His Met Ala Ala Ala Asp
   1 5 10 15
   Leu Ala Asn Ile Lys Ser Arg Leu Thr Asp Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Phe Gly Glu
   65 70 75 80
   Ala Trp Gly Tyr Leu Pro Ser Asp Gln Ala Ala Val Tyr Val Asp Asn
   85 90 95
   His Asp Thr Glu
   100
<210> 110
   <211> 100
   <212> PRT
   <213> Streptomyces sp. B154A
<400> 110
   Val Asp Gly Phe Arg Ile Asp Ala Ala Lys His Met Asp Ala Ala Asp
   1 5 10 15
   Leu Ala Asp Ile Lys Ser Arg Leu Ser Asn Pro Ser Val Tyr Trp Lys
   20 25 30
   Gln Glu Val Ile Tyr Gly Ser Gly Glu Ala Val Gln Pro Thr Glu Tyr
   35 40 45
   Thr Ser Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Gly Val Ser Gly Val Tyr Val Asp Asn
   85 90 95
   His Asp Thr Glu
   100
<210> 111
   <211> 100
   <212> PRT
   <213> Streptomyces sp. B156B
<400> 111
   Val Asp Gly Phe Arg Ile Asp Thr Ala Lys His Met Ala Ala Ala Asp
   1 5 10 15
   Leu Ala Asn Ile Lys Ser Arg Leu Thr Asp Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Phe Gly Glu
   65 70 75 80
   Ala Trp Gly Tyr Leu Pro Ser Asp Gln Ala Ala Val Tyr Val Asp Asn
   85 90 95
   His Asp Thr Glu
   100
<210> 112
   <211> 100
   <212> PRT
   <213> Streptomyces sp. B157C
<400> 112
   Val Asp Gly Phe Arg Ile Asp Ala Ala Lys His Met Asp Ala Ala Asp
   1 5 10 15
   Leu Ala Asp Ile Lys Ser Arg Leu Ser Asn Pro Ser Val Tyr Trp Lys
   20 25 30
   Gln Glu Val Ile Tyr Gly Ser Gly Glu Ala Val Gln Pro Thr Glu Tyr
   35 40 45
   Thr Ser Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Gly Val Ser Gly Val Phe Val Asp Asn
   85 90 95
   His Asp Thr Glu
   100
<210> 113
   <211> 100
   <212> PRT
   <213> Streptomyces sp. B158A
<400> 113
   Val Asp Gly Phe Arg Ile Asp Thr Ala Lys His Met Pro Ala Ala Asp
   1               5                   10                  15
   Leu Ala Asn Ile Lys Ser Arg Leu Ser Asn Pro Gly Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 114
   <211> 100
   <212> PRT
   <213> Streptomyces sp. B159
<400> 114
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ala Gly Val Tyr Val Asp Asn
                   85                  90                  95
   His Asp Thr Glu
               100
<210> 115
   <211> 100
   <212> PRT
   <213> Streptomyces sp. B160B
<400> 115
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 116
   <211> 100
   <212> PRT
   <213> Streptomyces sp. B161A
<400> 116
   Val Asp Gly Phe Arg Ile Asp Thr Ala Lys His Met Ala Ala Ala Asp
   1 5 10 15
   Leu Ala Asn Ile Lys Ser Arg Leu Thr Asp Pro Asn Val Tyr Trp Lys
   20 25 30
   His Glu Ala Ile Tyr Gly Ala Gly Glu Ala Val Gln Pro Ser Glu Tyr
   35 40 45
   Leu Gly Thr Gly Asp Val Gln Glu Phe Arg Tyr Ala Arg Asp Leu Lys
   50 55 60
   Arg Val Phe Gln Asn Glu Asn Leu Ala His Leu Lys Asn Phe Gly Glu
   65 70 75 80
   Asp Trp Gly Tyr Met Ala Ser Gly Lys Ser Ala Val Phe Val Asp Asn
   85 90 95
   His Asp Thr Glu
   100
<210> 117
   <211> 100
   <212> PRT
   <213> Streptomyces sp. B166B
<400> 117
   Val Asp Gly Phe Arg Ile Asp Ala Ala Lys His Ile Pro Ala Ala Asp
   1 5 10 15
   Leu Ala Asn Ile Lys Ser Arg Leu Asn Asn Pro Ser Ala Tyr Trp Lys
   20 25 30
   Gln Glu Val Ile Tyr Gly Ala Gly Glu Ala Val Gln Pro Gly Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Lys Ser Gly Ser Ala Gly Val Tyr Val Asp Asn
   85 90 95
   His Asp Thr Glu
   100
<210> 118
   <211> 100
   <212> PRT
   <213> Streptomyces sp. B168
<400> 118
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 119
   <211> 100
   <212> PRT
   <213> Streptomyces sp. B179
<400> 119
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 120
   <211> 100
   <212> PRT
   <213> Streptomyces sp. B181C
<400> 120
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Phe Val Asp Asn
   85 90 95
   His Asp Thr Glu
   100

<210> 121
   <211> 100
   <212> PRT
   <213> Streptomyces sp. B183B
<400> 121
   Val Asp Gly Phe Arg Ile Asp Ala Ala Lys His Met Pro Ala Ala Asp
   1 5 10 15
   Leu Thr Ala Ile Lys Ala Lys Val Gly Asn Gly Ser Thr Tyr Trp Lys
   20 25 30
   Gln Glu Ala Ile His Gly Ala Gly Glu Ala Val Gln Pro Ser Glu Tyr
   35 40 45
   Leu Gly Thr Gly Asp Val Gln Glu Phe Arg Tyr Ala Arg Asp Leu Lys
   50 55 60
   Arg Val Phe Gln Asn Glu Asn Leu Ala Tyr Leu Lys Asn Phe Gly Glu
   65 70 75 80
   Gly Trp Gly Tyr Met Ala Ser Gly Lys Ser Ala Val Tyr Val Asp Asn
   85 90 95
   His Asp Thr Glu
   100
<210> 122
   <211> 100
   <212> PRT
   <213> Streptomyces sp. B184
<400> 122
   Val Asp Gly Phe Arg Ile Asp Ala Ala Lys His Met Pro Ala Ala Asp
   1 5 10 15
   Leu Thr Ala Ile Lys Ala Lys Val Gly Asn Gly Ser Thr Tyr Trp Lys
   20 25 30
   Gln Glu Ala Ile His Gly Ala Gly Glu Ala Val Gln Pro Ser Glu Tyr
   35 40 45
   Leu Gly Thr Gly Asp Val Gln Glu Phe Arg Tyr Ala Arg Asp Leu Lys
   50 55 60
   Arg Val Phe Gln Asn Gly Asn Leu Ala Tyr Leu Lys Asn Phe Gly Glu
   65 70 75 80
   Gly Trp Gly Tyr Met Ala Ser Gly Lys Ser Ala Val Tyr Val Asp Asn
   85 90 95
   His Asp Thr Glu
   100
<210> 123
   <211> 100
   <212> PRT
   <213> Streptomyces sp. B185(B)
<400> 123
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 124
   <211> 100
   <212> PRT
   <213> Streptomyces sp. B186A
<400> 124
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Gly Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg His Ala Arg Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Asn Ser Gly Ser Ala Gly Val Tyr Val Asp Asn
   85 90 95
   His Asp Thr Glu
   100
<210> 125
   <211> 100
   <212> PRT
   <213> Streptomyces sp. B187A
<400> 125
   Val Asp Gly Phe Arg Ile Asp Thr Ala Lys His Met Ala Ala Gly Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Phe Gly Glu
   65 70 75 80
   Ser Trp Gly Tyr Leu Pro Ser Asp Gln Ala Ala Val Tyr Val Asp Asn
   85 90 95
   His Asp Thr Glu
   100
<210> 126
   <211> 100
   <212> PRT
   <213> Streptomyces sp. B187A2
<400> 126
   Val Asp Gly Phe Arg Ile Asp Thr Ala Lys His Met Ala Ala Gly Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Phe Gly Glu
   65 70 75 80
   Ser Trp Gly Tyr Leu Pro Ser Asp Gln Ala Ala Val Tyr Val Asp Asn
   85 90 95
   His Asp Thr Glu
   100
<210> 127
   <211> 97
   <212> PRT
   <213> Streptomyces sp. B194A
<400> 127
   Val Asp Gly Phe Arg Ile Asp Ala Ala Lys His Ile Pro Ala Ser Asp
   1 5 10 15
   Leu Glu Ala Ile Lys Ala Arg Met Ser Asn Pro Asn Val Phe Trp Val
   20 25 30
   His Glu Val Ile Gly Ala Ala Gly Glu Pro Ile Gln Pro Ser Glu Tyr
   35 40 45
   Leu Gly Ser Gly Asp Ser His Glu Phe Asp Tyr Ala Arg Gln Leu Lys
   50 55 60
   Arg Asp Phe Asp Gly Gln Ile Lys Asn Leu Arg Tyr Ile Gly Asp Gly
   65 70 75 80
   Lys Leu Pro Tyr Asp Arg Ala Gly Val Phe Val Asp Asn His Asp Thr
   85 90 95
   Glu
<210> 128
   <211> 100
   <212> PRT
   <213> Streptomyces sp. B194B1
<400> 128
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 129
   <211> 100
   <212> PRT
   <213> Streptomyces sp. B196A2C
<400> 129
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Asp
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 130
   <211> 100
   <212> PRT
   <213> Streptomyces sp. B196B
<400> 130
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Asp
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 131
   <211> 100
   <212> PRT
   <213> Streptomyces sp. B197B
<400> 131
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Xaa Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Asp
   65 70 75 80
   Gly Trp Gly Tyr Leu Asn Ser Gly Val Ser Gly Val Phe Val Asp Asn
   85 90 95
   His Asp Thr Glu
   100
<210> 132
   <211> 100
   <212> PRT
   <213> Streptomyces sp. B198C2
<400> 132
   Val Asp Gly Phe Arg Ile Asp Ala Ala Lys His Ile Pro Ala Ala Asp
   1 5 10 15
   Leu Ala Asn Ile Lys Ser Arg Leu Thr Asn Pro Ser Ala Tyr Trp Lys
   20 25 30
   Gln Glu Val Ile Tyr Gly Ala Gly Glu Ala Val Gln Pro Thr Glu Tyr
   35 40 45
   Thr Gly Ala Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Asn Ser Gly Ser Ala Gly Val Phe Val Asp Asn
   85 90 95
   His Asp Thr Glu
   100
<210> 133
   <211> 100
   <212> PRT
   <213> Streptomyces sp. B200B
<400> 133
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 134
   <211> 100
   <212> PRT
   <213> Streptomyces sp. B201A
<400> 134
   Val Asp Gly Phe Arg Ile Asp Ala Ala Lys His Ile Pro Ala Ala Asp
   1 5 10 15
   Leu Ala Asp Ile Lys Ser Arg Leu Ser Asp Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Ser Ser Gly Ala Ala Gly Val Tyr Val Asp Asn
   85 90 95
   His Asp Thr Glu
   100
<210> 135
   <211> 100
   <212> PRT
   <213> Streptomyces sp. B202A
<400> 135
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Xaa Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 136
   <211> 100
   <212> PRT
   <213> Streptomyces sp. B202B
<400> 136
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 137
   <211> 100
   <212> PRT
   <213> Streptomyces sp. B206A
<400> 137
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Xaa Asp Asn
   85 90 95
   His Asp Thr Glu
   100
<210> 138
   <211> 100
   <212> PRT
   <213> Streptomyces sp. B207
<400> 138
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ala Gly Val Phe Val Asp Asn
   85 90 95
   His Asp Thr Glu
   100
<210> 139
   <211> 100
   <212> PRT
   <213> Streptomyces sp. B208B
<400> 139
   Val Asp Gly Phe Arg Ile Asp Thr Ala Lys His Met Ala Ala Gly Asp
   1               5                   10                  15
   Leu Ala Ala Ile Lys Ser Arg Leu Thr Asn Pro Asn Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Phe Gly Ser Glu Asn Leu Ala Tyr Leu Lys Asn Phe Gly Glu
   65                  70                  75                  80
   Ser Trp Gly Tyr Met Pro Ser Gly Gln Ser Ala Val Tyr Val Asp Asn
                   85                  90                  95
   His Asp Thr Glu
               100
<210> 140
   <211> 100
   <212> PRT
   <213> Streptomyces sp. B209B
<400> 140
   Val Asp Gly Phe Arg Ile Asp Thr Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 141
   <211> 100
   <212> PRT
   <213> Streptomyces sp. B210
<400> 141
   Val Asp Gly Phe Arg Ile Asp Ala Ala Lys His Met Ala Ala Gly Asp
    1              5                   10                  15
   Leu Ala Asn Ile Lys Ser Arg Leu Thr Asn Pro Asn Val Tyr Trp Lys
               20                  25                  30
   His Glu Ala Ile Tyr Gly Ala Gly Glu Ala Val Ser Pro Ala Glu Tyr
           35                  40                  45
   Leu Gly Ser Gly Asp Val Gln Glu Phe Arg Tyr Ala Arg Asp Leu Lys
       50                  55                  60
   Arg Val Phe Asn Asn Glu Asn Leu Ala Tyr Leu Lys Asn Phe Gly Glu
   65                  70                  75                  80
   Ser Trp Gly Tyr Leu Pro Ser Asp Gln Ala Ala Val Tyr Val Asp Asn
                   85                  90                  95
   His Asp Thr Glu
               100
<210> 142
   <211> 100
   <212> PRT
   <213> Streptomyces sp. B211A
<400> 142
   Val Asp Gly Phe Arg Ile Asp Ala Ala Lys His Ile Pro Ala Ala Asp
    1              5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 143
   <211> 100
   <212> PRT
   <213> Streptomyces sp. B212B1
<400> 143
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 144
   <211> 100
   <212> PRT
   <213> Streptomyces sp. B213
<400> 144
   Val Asp Gly Phe Arg Ile Asp Ala Ala Lys His Ile Pro Ala Ala Asp
   1 5 10 15
   Leu Ala Asn Ile Lys Ser Arg Leu Ser Asn Arg Gly Val Tyr Trp Lys
   20 25 30
   His Glu Val Thr Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 145
   <211> 100
   <212> PRT
   <213> Streptomyces sp. B214B
<400> 145
   Val Asp Gly Phe Arg Ile Asp Thr Ala Lys His Met Xaa Ala Ala Asp
   1               5                   10                  15
   Leu Ala Asn Ile Lys Ser Arg Leu Thr Asn Pro Asn Val Tyr Trp Lys
               20                  25                  30
   His Glu Ala Ile Tyr Trp Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Ala Val Phe Val Asp Asn
                   85                  90                  95
   His Asp Thr Glu
               100
<210> 146
   <211> 100
   <212> PRT
   <213> Streptomyces sp. B214C
<400> 146
   Val Asp Gly Phe Arg Ile Asp Ala Ala Lys His Met Pro Ala Ala Asp
   1               5                   10                  15
   Leu Ala Asn Ile Lys Ser Arg Leu Thr Asn Pro Asn Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Ala Val Phe Val Asp Asn
                   85                  90                  95
   His Asp Thr Glu
               100
<210> 147
   <211> 100
   <212> PRT
   <213> Streptomyces sp. B215
<400> 147
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Phe Val Asp Asn
   85 90 95
   His Asp Thr Glu
   100
<210> 148
   <211> 100
   <212> PRT
   <213> Streptomyces sp. B218D2
<400> 148
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Val
   65 70 75 80
   Gly Trp Gly Tyr Leu Asn Ser Gly Val Ser Gly Val Tyr Val Asp Asn
   85 90 95
   His Asp Thr Glu
   100
<210> 149
   <211> 100
   <212> PRT
   <213> Streptomyces sp. B219A
<400> 149
   Val Asp Gly Phe Arg Ile Asp Thr Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Phe Val Asp Asn
                   85                  90                  95
   His Asp Thr Glu
               100
<210> 150
   <211> 100
   <212> PRT
   <213> Streptomyces sp. B220B
<400> 150
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
           100
<210> 151
   <211> 100
   <212> PRT
   <213> Streptomyces sp. B221A
<400> 151
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Asp Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Phe Val Asp Asn
                   85                  90                  95
   His Asp Thr Glu
               100

<210> 152
   <211> 100
   <212> PRT
   <213> Streptomyces sp. B222(B)
<400> 152
   Val Asp Gly Phe Arg Ile Asp Ala Ala Lys His Met Ala Ala Gly Asp
   1               5                   10                  15
   Leu Ala Asn Ile Lys Ser Arg Leu Thr Asn Pro Asn Val Tyr Trp Lys
               20                  25                  30
   His Glu Ala Ile Tyr Gly Ala Gly Glu Ala Val Ser Pro Ala Glu Tyr
           35                  40                  45
   Leu Gly Ser Gly Asp Val Gln Glu Phe Arg Tyr Ala Arg Asp Leu Lys
       50                  55                  60
   Arg Val Phe Asn Asn Glu Asn Leu Ala Xaa Leu Lys Asn Phe Gly Glu
   65                  70                  75                  80
   Ser Trp Gly Tyr Leu Pro Ser Asp Gln Ala Ala Val Tyr Val Asp Asn
                   85                  90                  95
   His Asp Thr Glu
               100
<210> 153
   <211> 100
   <212> PRT
   <213> Streptomyces sp. B223A
<400> 153
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Ser Ala Tyr Trp Lys
   20 25 30
   Gln Glu Val Ile Tyr Gly Ala Gly Glu Ala Val Gln Pro Gly Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Lys Ser Ser Ser Ala Gly Val Tyr Val Asp Asn
   85 90 95
   His Asp Thr Glu
   100
<210> 154
   <211> 100
   <212> PRT
   <213> Streptomyces sp. B224(A)
<400> 154
   Val Asp Gly Phe Arg Ile Asp Ala Ala Lys His Ile Pro Ala Ala Asp
   1 5 10 15
   Leu Ala Asn Ile Lys Ser Arg Leu Ser Ser Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 155
   <211> 100
   <212> PRT
   <213> Streptomyces sp. B225B
<400> 155
   Val Asp Gly Phe Arg Ile Asp Thr Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Gly Ala Gly Glu Ala Val Gln Ser Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Tyr Val Asp Asn
   85 90 95
   His Asp Thr Glu
   100
<210> 156
   <211> 100
   <212> PRT
   <213> Streptomyces sp. B226B
<400> 156
   Val Asp Gly Phe Arg Ile Asp Thr Ala Lys His Met Ala Ala Gly Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Phe Gly Glu
   65 70 75 80
   Ser Trp Gly Tyr Leu Pro Ser Asp Gln Ala Ala Val Tyr Val Asp Asn
   85 90 95
   His Asp Thr Glu
   100
<210> 157
   <211> 100
   <212> PRT
   <213> Streptomyces sp. B227B2
<400> 157
   Val Asp Gly Phe Arg Ile Asp Ala Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Gly Ala Gly Glu Ala Val Gln Pro Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Phe Val Asp Asn
   85 90 95
   His Asp Thr Glu
   100
<210> 158
   <211> 100
   <212> PRT
   <213> Streptomyces sp. B228B
<400> 158
   Val Asp Gly Phe Arg Ile Asp Thr Ala Lys His Ile Pro Ala Ala Asp
   1 5 10 15
   Leu Ala Asn Ile Lys Ser Arg Leu Asn Asn Pro Ser Ala Tyr Trp Lys
   20 25 30
   Gln Glu Val Ile Tyr Gly Ala Gly Glu Ala Val Gln Pro Gly Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Lys Ser Gly Ser Ala Gly Val Tyr Val Asp Asn
   85 90 95
   His Asp Thr Glu
   100
<210> 159
   <211> 100
   <212> PRT
   <213> Streptomyces sp. B230B1
<400> 159
   Val Asp Gly Phe Arg Ile Asp Ala Ala Lys His Ile Ala Thr Glu Asp
   1               5                   10                  15
   Leu Ala Ala Val Lys Ala Lys Leu Ser Lys Pro Asp Val Tyr Trp Lys
               20                  25                  30
   Gln Glu Thr Ile Tyr Gly Ala Gly Glu Ala Val Gln Pro Gln Glu Tyr
           35                  40                  45
   Leu Gly Asn Gly Asp Val Gln Glu Phe Arg Tyr Ala Arg Asp Leu Lys
       50                  55                  60
   Arg Val Phe Gln Ser Glu Arg Leu Ala Tyr Leu Lys Asn Tyr Gly Glu
   65                  70                  75                  80
   Gly Trp Gly Tyr Leu Pro Gly Asp Arg Ala Ser Val Tyr Val Asp Asn
                   85                  90                  95
   His Asp Thr Glu
               100
<210> 160
   <211> 100
   <212> PRT
   <213> Streptomyces sp. B231A
<400> 160
   Val Asp Gly Phe Arg Ile Asp Thr Ala Lys His Ile Ala Thr Glu Asp
   1               5                   10                  15
   Leu Ala Ala Val Lys Ala Lys Leu Ser Lys Pro Asp Val Tyr Trp Lys
               20                  25                  30
   Gln Glu Thr Ile Tyr Gly Ala Gly Glu Ala Val Gln Pro Gln Glu Tyr
           35                  40                  45
   Leu Gly Asn Gly Asp Val Gln Glu Phe Arg Tyr Ala Arg Asp Leu Lys
       50                  55                  60
   Arg Val Phe Gln Ser Glu Arg Leu Ala Tyr Leu Lys Asn Tyr Gly Glu
   65                  70                  75                  80
   Gly Trp Gly Tyr Leu Pro Gly Asp Arg Ala Ser Val Tyr Val Asp Asn
                   85                  90                  95
   His Asp Thr Glu
               100
<210> 161
   <211> 100
   <212> PRT
   <213> Streptomyces sp. B233C
<400> 161
   Val Asp Gly Phe Arg Ile Asp Ala Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Gly Ala Gly Glu Ala Val Gln Pro Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Phe Val Asp Asn
   85 90 95
   His Asp Thr Glu
   100
<210> 162
   <211> 100
   <212> PRT
   <213> Streptomyces sp. B234
<400> 162
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 163
   <211> 100
   <212> PRT
   <213> Streptomyces sp. B235A
<400> 163
   Val Asp Gly Phe Arg Ile Asp Ala Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Gly Ala Gly Glu Ala Val Gln Pro Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Phe Val Asp Asn
   85 90 95
   His Asp Thr Glu
   100
<210> 164
   <211> 100
   <212> PRT
   <213> Streptomyces sp. B237A
<400> 164
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ala Gly Val Phe Val Asp Asn
   85 90 95
   His Asp Thr Glu
   100
<210> 165
   <211> 100
   <212> PRT
   <213> Streptomyces sp. B238A
<400> 165
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ala Gly Val Tyr Val Asp Asn
   85 90 95
   His Asp Thr Glu
   100
<210> 166
   <211> 100
   <212> PRT
   <213> Streptomyces sp. B240A
<400> 166
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 167
   <211> 100
   <212> PRT
   <213> Streptomyces sp. B241B2
<400> 167
   Val Asp Gly Phe Arg Ile Asp Ala Ala Lys His Ile Pro Ala Ala Asp
   1               5                   10                  15
   Leu Ala Asn Ile Lys Ser Arg Leu Thr Asn Pro Ser Ala Tyr Trp Lys
               20                  25                  30
   His Glu Val Ile Phe Gly Ala Gly Glu Ala Val Gln Pro Thr Glu Tyr
           35                  40                  45
   Thr Gly Thr Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Thr Ser Gly Ser Ala Gly Val Tyr Val Asp Asn
                   85                  90                  95
   His Asp Thr Glu
               100
<210> 168
   <211> 100
   <212> PRT
   <213> Streptomyces sp. B242A1
<400> 168
   Val Asp Gly Phe Arg Ile Asp Thr Ala Lys His Met Pro Ala Gly Asp
   1               5                   10                  15
   Pro Ala Asn Ile Lys Ser Arg Leu Ser Asn Pro Asn Val Tyr Trp Lys
               20                  25                  30
   His Glu Ala Ile Phe Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Ala Val Tyr Val Asp Asn
                   85                  90                  95
   His Asp Thr Glu
               100
<210> 169
   <211> 100
   <212> PRT
   <213> Streptomyces sp. B243C
<400> 169
   Val Asp Gly Phe Arg Ile Asp Ala Ala Lys His Met Pro Ala Ala Asp
   1 5 10 15
   Leu Ala Asn Ile Lys Ser Arg Leu Ser Asn Pro Asp Ala Tyr Trp Lys
   20 25 30
   Gln Glu Val Ile His Gly Ala Gly Glu Ala Val Gln Pro Gly Glu Tyr
   35 40 45
   Thr Gly Asn Gly Asp Val Gln Glu Phe Arg Tyr Ala Tyr Asp Leu Lys
   50 55 60
   Arg Val Phe His Asn Glu Asn Leu Ala Tyr Leu Lys Asn Tyr Gly Glu
   65 70 75 80
   Gly Trp Gly Tyr Leu Lys Asn Ser Ser Ala Gly Val Xaa Val Asp Asn
   85 90 95
   His Asp Thr Glu
   100
<210> 170
   <211> 100
   <212> PRT
   <213> Streptomyces sp. B244B2
<400> 170
   Val Asp Gly Phe Arg Ile Asp Ala Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Gly Ala Gly Glu Ala Val Gln Pro Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Tyr Val Asp Asn
   85 90 95
   His Asp Thr Glu
   100
<210> 171
   <211> 100
   <212> PRT
   <213> Streptomyces sp. B246
<400> 171
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ala Gly Val Tyr Val Asp Asn
   85 90 95
   His Asp Thr Glu
   100
<210> 172
   <211> 100
   <212> PRT
   <213> Streptomyces sp. B247A
<400> 172
   Val Asp Gly Phe Arg Ile Asp Thr Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Gly Ala Gly Glu Ala Val Gln Pro Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Tyr Val Asp Asn
   85 90 95
   His Asp Thr Glu
   100
<210> 173
   <211> 100
   <212> PRT
   <213> Streptomyces sp. B248B2
<400> 173
   Val Asp Gly Phe Arg Ile Asp Thr Ala Lys His Met Pro Ala Ala Asp
   1               5                   10                  15
   Leu Ala Asn Ile Lys Ser Arg Leu Ser Asn Pro Gly Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Gly Arg Gly Leu Lys
       50                  55                  60
   Gln Val Phe Asn Asn Glu Asn Leu Ala Tyr Leu Lys Asn Phe Gly Glu
   65                  70                  75                  80
   Gly Trp Gly Phe Met Glu Ser Gly Lys Ser Ala Val Tyr Val Asp Asn
                   85                  90                  95
   His Asp Thr Glu
               100
<210> 174
   <211> 100
   <212> PRT
   <213> Streptomyces sp. B249A
<400> 174
   Val Asp Gly Phe Arg Ile Asp Thr Ala Lys His Ile Ala Thr Glu Asp
   1               5                   10                  15
   Leu Ala Ala Val Lys Ala Lys Leu Ser Lys Pro Asp Val Tyr Trp Lys
               20                  25                  30
   Gln Glu Thr Ile Tyr Gly Ala Gly Glu Ala Val Gln Pro Gln Glu Tyr
           35                  40                  45
   Leu Gly Asn Gly Asp Val Gln Glu Phe Arg Tyr Ala Arg Asp Leu Lys
       50                  55                  60
   Arg Val Phe Gln Ser Glu Arg Leu Ala Tyr Leu Lys Asn Tyr Gly Glu
   65                  70                  75                  80
   Gly Trp Gly Tyr Leu Pro Gly Asp Arg Ala Ser Val Phe Val Asp Asn
                   85                  90                  95
   His Asp Thr Glu
               100
<210> 175
   <211> 100
   <212> PRT
   <213> Streptomyces sp. B249C
<400> 175
   Val Asp Gly Phe Arg Ile Asp Ala Ala Lys His Ile Pro Glu Asp Asp
    1               5                  10                  15
   Leu Ala Ala Ile Lys Ser Arg Leu Ser Asn Pro Gly Val Tyr Trp Lys
               20                  25                  30
   Gln Glu Thr Ile Tyr Gly Ala Gly Glu Ala Val Gln Pro Thr Glu Tyr
           35                  40                  45
   Leu Thr Thr Gly Asp Ala Gln Glu Phe Arg Tyr Ser Trp Asp Leu Lys
       50                  55                  60
   Arg Val Phe Thr Ser Glu Lys Leu Ala Tyr Leu Lys Asn Phe Gly Glu
   65                  70                  75                  80
   Gly Trp Gly Tyr Met Ala Gly Gly Lys Ala Ser Val Tyr Val Asp Asn
                   85                  90                  95
   His Asp Thr Glu
               100
<210> 176
   <211> 100
   <212> PRT
   <213> Streptomyces sp. B250A2
<400> 176
   Val Asp Gly Phe Arg Ile Asp Thr Ala Lys His Met Asp Ala Ala Asp
    1               5                  10                  15
   Leu Ala Asn Ile Lys Ser Arg Leu Ser Asn Pro Ser Val Tyr Trp Lys
               20                  25                  30
   Gln Glu Ala Ile Tyr Gly Ser Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Ser Ser Gly Val Ser Gly Val Tyr Val Asp Asn
                   85                  90                  95
   His Asp Thr Glu
               100
<210> 177
   <211> 100
   <212> PRT
   <213> Streptomyces sp. B251B
<400> 177
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Phe Val Asp Asn
   85 90 95
   His Asp Thr Glu
   100
<210> 178
   <211> 100
   <212> PRT
   <213> Streptomyces sp. B252A
<400> 178
   Val Asp Gly Phe Arg Ile Asp Thr Ala Lys His Met Pro Ala Ala Asp
   1 5 10 15
   Val Ala Asn Ile Lys Ser Arg Leu Thr Asn Pro Ser Ala Tyr Trp Lys
   20 25 30
   Gln Glu Val Ile Tyr Gly Ala Gly Glu Ala Val Gln Pro Gly Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Lys Ser Ser Ser Ala Gly Val Tyr Val Asp Asn
   85 90 95
   His Asp Thr Glu
   100
<210> 179
   <211> 100
   <212> PRT
   <213> Streptomyces sp. B253
<400> 179
   Val Asp Gly Phe Arg Ile Asp Thr Ala Lys His Ile Asp Thr Ala Asp
   1 5 10 15
   Leu Ala Asn Ile Lys Ser Arg Leu Thr Asn Pro Ser Ala Tyr Trp Lys
   20 25 30
   Gln Glu Val Ile Tyr Gly Ser Gly Glu Ala Val Gln Pro Thr Glu Tyr
   35 40 45
   Thr Arg Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Ser Ser Ser Ser Ala Gly Val Tyr Val Asp Asn
   85 90 95
   His Asp Thr Glu
   100
<210> 180
   <211> 100
   <212> PRT
   <213> Streptomyces sp. B253A
<400> 180
   Val Asp Gly Phe Arg Ile Asp Thr Ala Lys His Met Pro Ala Ala Asp
   1 5 10 15
   Leu Ala Asn Ile Lys Ser Arg Leu Thr Asn Pro Ser Ala Tyr Trp Lys
   20 25 30
   Gln Glu Val Ile Tyr Gly Ala Gly Glu Ala Val Gln Pro Gly Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Lys Ser Ser Ser Ala Gly Val Tyr Val Asp Asn
   85 90 95
   His Asp Thr Glu
   100
<210> 181
   <211> 100
   <212> PRT
   <213> Streptomyces sp. B255B2
<400> 181
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Phe Val Asp Asn
   85 90 95
   His Asp Thr Glu
   100
<210> 182
   <211> 100
   <212> PRT
   <213> Streptomyces sp. B256A
<400> 182
   Val Asp Gly Phe Arg Ile Asp Thr Ala Lys His Met Pro Ala Ala Asp
   1 5 10 15
   Leu Ala Asn Ile Lys Ser Arg Leu Thr Asp Pro Gly Val Tyr Trp Lys
   20 25 30
   Gln Glu Val Ile Phe Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Lys Ser Ser Ser Ala Gly Val Tyr Val Asp Asn
   85 90 95
   His Asp Thr Glu
   100
<210> 183
   <211> 100
   <212> PRT
   <213> Streptomyces sp. B259A
<400> 183
   Val Asp Gly Phe Arg Ile Asp Ala Ala Lys His Met Asp Ala Ala Asp
   1 5 10 15
   Leu Ala Asn Ile Lys Ser Arg Leu Gly Asn Pro Ser Ala Tyr Trp Lys
   20 25 30
   Gln Glu Ala Ile Phe Gly Ala Gly Glu Ala Val Gln Pro Thr Glu Tyr
   35 40 45
   Thr Gly Asn Gly Asp Val Gln Glu Phe Arg Tyr Gly Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Met Asn Ser Ser Val Ala Gly Val Tyr Val Asp Asn
   85 90 95
   His Asp Thr Glu
   100

<210> 184
   <211> 100
   <212> PRT
   <213> Streptomyces sp. B261
<400> 184
   Val Asp Gly Phe Arg Ile Asp Thr Ala Lys His Ile Pro Ala Glu Asp
   1 5 10 15
   Leu Ala Asn Ile Lys Ser Arg Leu Ser Asn Pro Asn Ala Tyr Trp Lys
   20 25 30
   Gln Glu Val Ile Tyr Gly Ala Gly Glu Ala Val Gln Pro Gly Glu Tyr
   35 40 45
   Thr Gly Thr Gly Asp Val Gln Glu Phe Arg Tyr Ala Tyr Asp Leu Lys
   50 55 60
   Arg Val Phe Thr Gln Gly Asn Leu Ala Tyr Leu Lys Asn Tyr Gly Glu
   65 70 75 80
   Asp Trp Gly Tyr Leu Ser Ser Thr Thr Ala Gly Val Tyr Val Asp Asn
   85 90 95
   His Asp Thr Glu
   100
<210> 185
   <211> 100
   <212> PRT
   <213> Streptomyces sp. B278
<400> 185
   Val Asp Gly Phe Arg Ile Asp Thr Ala Lys His Met Ala Ala Gly Asp
   1               5                   10                  15
   Leu Ala Asn Ile Lys Ser Arg Leu Thr Asn Pro Asn Val Tyr Trp Lys
               20                  25                  30
   His Glu Ala Ile Tyr Gly Ala Gly Glu Ala Val Ser Pro Ala Glu Tyr
           35                  40                  45
   Leu Gly Ser Gly Asp Val Gln Glu Phe Arg Tyr Ala Arg Asp Leu Lys
       50                  55                  60
   Arg Val Phe Asn Asn Glu Asn Leu Ala Tyr Leu Lys Asn Phe Gly Glu
   65                  70                  75                  80
   Ser Trp Gly Tyr Leu Pro Ser Asp Gln Ala Ala Val Tyr Xaa Asp Asn
                   85                  90                  95
   His Asp Thr Glu
               100
<210> 186
   <211> 100
   <212> PRT
   <213> Streptomyces sp. B279
<400> 186
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 187
   <211> 100
   <212> PRT
   <213> Streptomyces sp. B280C
<400> 187
   Val Asp Gly Phe Arg Ile Asp Ala Ala Lys His Met Ala Ala Gly Asp
   1               5                   10                  15
   Leu Ala Pro Ile Lys Ser Arg Leu Ser Asn Pro Asn Val Tyr Trp Lys
               20                  25                  30
   Gln Glu Val Ile His Gly Ala Gly Glu Ala Val Gln Pro Gly Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Asn Ser Ser Val Ser Gly Val Phe Val Asp Asn
                   85                  90                  95
   His Asp Thr Glu
               100
<210> 188
   <211> 100
   <212> PRT
   <213> Streptomyces sp. B284A
<400> 188
   Val Asp Gly Phe Arg Ile Asp Ala Ala Lys His Met Pro Ala Ala Asp
   1               5                   10                  15
   Leu Ala Asn Ile Lys Ser Arg Leu Thr Lys Pro Gly Val Tyr Trp Lys
               20                  25                  30
   Gln Glu Ala Ile Tyr Gly Ala Gly Glu Ala Val Gln Pro Ser Glu Tyr
           35                  40                  45
   Thr Gly Thr Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Asn Ser Gly Val Ser Gly Val Tyr Val Asp Asn
                   85                  90                  95
   His Asp Thr Glu
               100
<210> 189
   <211> 100
   <212> PRT
   <213> Streptomyces sp. B286A
<400> 189
   Val Asp Gly Phe Arg Ile Asp Thr Ala Lys His Met Pro Ala Ala Asp
   1               5                   10                  15
   Leu Ala Asn Ile Lys Ser Arg Leu Ser Asn Pro Ser Val Tyr Trp Lys
               20                  25                  30
   Gln Glu Val Ile Phe Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Met Asn Ser Gly Val Ser Gly Val Tyr Val Asp Asn
                   85                  90                  95
   His Asp Thr Glu
               100
<210> 190
   <211> 100
   <212> PRT
   <213> Streptomyces sp. B287
<400> 190
   Val Asp Gly Phe Arg Ile Asp Ala Ala Lys His Met Pro Ala Gly Asp
   1               5                   10                  15
   Leu Ala Asn Ile Lys Ser Arg Leu Ser Asn Pro Asn Val Tyr Trp Lys
               20                  25                  30
   Gln Glu Ala Ile His Gly Ala Gly Glu Ala Val Ser Pro Ser Glu Tyr
           35                  40                  45
   Leu Gly Thr Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Gly Val Tyr Val Asp Asn
                   85                  90                  95
   His Asp Thr Glu
               100
<210> 191
   <211> 100
   <212> PRT
   <213> Streptomyces sp. B292A
<400> 191
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Asn Ala Phe Trp Lys
   20 25 30
   Leu Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Val Leu Gln Asn Glu Lys Leu Ala Tyr Leu Lys Asn Phe Gly Glu
   65 70 75 80
   Ala Trp Gly Tyr Met Pro Ser Gly Gln Ser Gly Val Tyr Val Asp Asn
   85 90 95
   His Asp Thr Glu
   100
<210> 192
   <211> 100
   <212> PRT
   <213> Streptomyces sp. B3001org
<400> 192
   Val Asp Gly Phe Arg Ile Asp Thr Ala Lys His Ile Ala Ala Ala Asp
   1 5 10 15
   Leu Ala Asn Ile Lys Ser Arg Leu Thr Asn Pro Gly Val Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Cys Tyr Gly Tyr Asp Leu Lys
   50 55 60
   Arg Val Phe Asn Ser Glu Lys Leu Ala Tyr Leu Asn Asn Phe Gly Glu
   65 70 75 80
   Gly Trp Gly Tyr Ile Pro Gly Asn Val Ala Gly Val Tyr Val Asp Asn
   85 90 95
   His Asp Thr Glu
   100
<210> 193
   <211> 100
   <212> PRT
   <213> Streptomyces sp. B3002org
<400> 193
   Val Asp Gly Phe Arg Ile Asp Thr Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Gly Ala Gly Glu Ala Val Gln Pro Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Tyr Val Asp Asn
   85 90 95
   His Asp Thr Glu
   100
<210> 194
   <211> 100
   <212> PRT
   <213> Streptomyces sp. B3003org
<400> 194
   Val Asp Gly Phe Arg Ile Asp Thr Ala Lys His Ile Asp Thr Ala Asp
   1 5 10 15
   Leu Ala Asn Ile Lys Ser Arg Leu Thr Asn Pro Gly Val Tyr Trp Lys
   20 25 30
   Gln Glu Val Ile Tyr Gly Ser Gly Glu Ala Val Gln Pro Ala Glu Tyr
   35 40 45
   Thr Gly Asn Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Ser Asn Ser Val Ala Gly Val Tyr Val Asp Asn
   85 90 95
   His Asp Thr Glu
   100
<210> 195
   <211> 100
   <212> PRT
   <213> Streptomyces sp. B3017
<400> 195
   Val Asp Gly Phe Arg Ile Asp Ala Ser Lys His Met Pro Ala Asp Asp
   1 5 10 15
   Leu Ala Ala Ile Lys Gly Lys Leu Ala Asn Pro Gly Val Tyr Trp Lys
   20 25 30
   His Glu Ala Ile His Gly Ala Gly Glu Ala Val Ser Pro Ser Glu Tyr
   35 40 45
   Leu Gly Ser Gly Asp Val Gln Glu Phe Arg Tyr Gly Arg Gly Leu Lys
   50 55 60
   Gln Val Phe Thr Gly Gly Ser Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 196
   <211> 100
   <212> PRT
   <213> Streptomyces sp. B306
<400> 196
   Val Asp Gly Phe Arg Ile Asp Ala Ala Lys His Met Pro Ala Ala Asp
   1 5 10 15
   Leu Ala Asn Ile Lys Ser Arg Leu Thr Asn Pro Gly Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Asn Ser Gly Ser Ala Gly Val Tyr Val Asp Asn
   85 90 95
   His Asp Thr Glu
   100
<210> 197
   <211> 100
   <212> PRT
   <213> Streptomyces sp. B308
<400> 197
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 198
   <211> 100
   <212> PRT
   <213> Streptomyces sp. B311
<400> 198
   Val Asp Gly Phe Arg Ile Asp Ala Ala Lys His Met Pro Ala Gly Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 199
   <211> 100
   <212> PRT
   <213> Streptomyces sp. B315
<400> 199
   Val Asp Gly Phe Arg Ile Asp Ala Ala Lys His Ile Pro Ala Ala Asp
    1               5                  10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Asp
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 200
   <211> 100
   <212> PRT
   <213> Streptomyces sp. B317
<400> 200
   Val Asp Gly Phe Arg Ile Asp Ala Ala Lys His Met Pro Ala Ala Asp
    1               5                  10                  15
   Leu Ala Ala Ile Lys Ser Arg Leu Thr Asn Pro Asn Val Tyr Trp Lys
               20                  25                  30
   Gln Glu Ala Ile His Gly Ala Gly Glu Ala Val Ser Pro Ser Glu Tyr
           35                  40                  45
   Leu Gly Thr Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Gly Val Tyr Val Asp Asn
                   85                  90                  95
   His Asp Thr Glu
               100
<210> 201
   <211> 100
   <212> PRT
   <213> Streptomyces sp. B318
<400> 201
   Val Asp Gly Phe Arg Ile Asp Ala Ala Lys His Met Pro Ala Gly Asp
   1 5 10 15
   Leu Ala Asp Ile Lys Ser Arg Leu Ser Asn Pro Asn Val Tyr Trp Lys
   20 25 30
   Gln Glu Ala Ile Tyr Gly Ala Asn Glu Ala Val Ser Pro Thr Glu Tyr
   35 40 45
   Leu Gly Thr Gly Asp Val Gln Glu Phe Arg Tyr Ala Arg Gly Leu Lys
   50 55 60
   Gln Thr Phe Leu Gly Gly Asn Leu Ala Asp Leu Lys Asn Phe Gly Glu
   65 70 75 80
   Gly Trp Gly Phe Met Glu Ser Gly Arg Ser Ala Val Tyr Val Asp Asn
   85 90 95
   His Asp Thr Glu
   100
<210> 202
   <211> 100
   <212> PRT
   <213> Streptomyces sp. B319
<400> 202
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 203
   <211> 100
   <212> PRT
   <213> Streptomyces sp. B320A
<400> 203
   Val Asp Gly Phe Arg Ile Asp Ala Ala Lys His Ile Ala Ala Ala Asp
    1               5                  10                  15
   Leu Ala Asn Ile Lys Ser Arg Leu Thr Asn Pro Gly Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Cys Tyr Gly Tyr Asp Leu Lys
       50                  55                  60
   Arg Val Phe Asn Ser Glu Lys Leu Ala Tyr Leu Lys Asn Tyr Gly Glu
   65                  70                  75                  80
   Gly Trp Gly Tyr Met Ser Ser Ser Val Ala Gly Val Tyr Val Asp Asn
                   85                  90                  95
   His Asp Thr Glu
               100
<210> 204
   <211> 100
   <212> PRT
   <213> Streptomyces sp. B321
<400> 204
   Val Asp Gly Phe Arg Ile Asp Thr Ala Lys His Met Ala Val Gly Asp
    1               5                  10                  15
   Leu Ala Asp Ile Lys Ser Arg Leu Gly Asn Pro Asp Val His Trp Lys
               20                  25                  30
   His Glu Ala Ile His Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Leu Thr Gly Gly Ser Leu Ala His Leu Arg Asn Phe Gly Glu
   65                  70                  75                  80
   Gly Trp Gly Tyr Met Ala Ser Asp Arg Ser Asn Val Tyr Val Asp Asn
                   85                  90                  95
   His Asp Thr Glu
               100
<210> 205
   <211> 100
   <212> PRT
   <213> Streptomyces sp. B322A
<400> 205
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ala Gly Val Tyr Val Asp Asn
                   85                  90                  95
   His Asp Thr Glu
               100
<210> 206
   <211> 100
   <212> PRT
   <213> Streptomyces sp. B323
<400> 206
   Val Asp Gly Phe Arg Ile Asp Ala Ala Lys His Met Pro Ala Ala Asp
   1               5                   10                  15
   Leu Ala Asn Ile Lys Thr Arg Leu Thr Asn Pro Asn Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Gly Val Phe Val Asp Asn
                   85                  90                  95
   His Asp Thr Glu
               100
<210> 207
   <211> 100
   <212> PRT
   <213> Streptomyces sp. B326
<400> 207
   Val Asp Gly Phe Arg Ile Asp Thr Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   Gln Glu Ala Ile Phe Gly Ala Gly Glu Ala Val Gln Pro Ser Glu Tyr
   35 40 45
   Thr Gly Thr Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Gly Val Ser Gly Val Tyr Val Asp Asn
   85 90 95
   His Asp Thr Glu
   100
<210> 208
   <211> 100
   <212> PRT
   <213> Streptomyces sp. B327B
<400> 208
   Val Asp Gly Phe Xaa Ile Asp Ala Ala Lys His Met Pro Ala Ala Asp
   1 5 10 15
   Leu Thr Ala Ile Lys Ala Lys Val Gly Asp Gly Gly Thr Tyr Trp Lys
   20 25 30
   Gln Glu Ala Ile His Gly Ala Gly Glu Ala Val Gln Pro Ser Glu Tyr
   35 40 45
   Leu Gly Thr Gly Asp Val Gln Glu Phe Arg Tyr Ala Arg Asp Leu Lys
   50 55 60
   Arg Val Phe Gln Asn Glu Asn Leu Ala His Leu Lys Asn Phe Gly Glu
   65 70 75 80
   Asp Trp Gly His Met Gln Ser Gly Arg Ser Ala Val Phe Val Asp Asn
   85 90 95
   His Asp Thr Glu
   100
<210> 209
   <211> 100 <212> PRT
   <213> Streptomyces sp. B335org
<400> 209
   Val Asp Gly Phe Arg Ile Asp Ala Ala Lys His Met Pro Ala Ala Asp
   1 5 10 15
   Leu Ala Asn Ile Lys Thr Arg Leu Thr Asn Pro Asn Val Tyr Trp Lys
   20 25 30
   His Glu Ala Ile Tyr Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Gly Val Phe Val Asp Asn
   85 90 95
   His Asp Thr Glu
   100
<210> 210
   <211> 100
   <212> PRT
   <213> Streptomyces sp. B345
<400> 210
   Val Asp Gly Phe Arg Ile Asp Ala Ala Lys His Met Asp Ala Ala Asp
   1 5 10 15
   Leu Ala Asn Ile Lys Ser Arg Leu Ser Asp Pro Asn Val Tyr Trp Lys
   20 25 30
   Gln Glu Val Ile His Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Gly Asn Glu Asn Leu Ala Tyr Leu Lys Asn Tyr Gly Glu
   65 70 75 80
   Gly Trp Gly Tyr Met Asn Ser Ser Val Ala Gly Val Tyr Val Asp Asn
   85 90 95
   His Asp Thr Glu
   100
<210> 211
   <211> 100
   <212> PRT
   <213> Streptomyces sp. B346
<400> 211
   Val Asp Gly Phe Arg Ile Asp Ala Ala Lys His Met Asp Ala Ala Asp
   1               5                   10                  15
   Leu Ala Asn Ile Lys Ser Arg Leu Ser Asn Pro Ser Val Tyr Trp Lys
               20                  25                  30
   Gln Glu Ala Ile Tyr Gly Ser Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Asn Ser Ser Val Ser Gly Val Phe Val Asp Asn
                   85                  90                  95
   His Asp Thr Glu
               100
<210> 212
   <211> 100
   <212> PRT
   <213> Streptomyces sp. B347
<400> 212
   Val Asp Gly Phe Arg Ile Asp Thr Ala Lys His Met Pro Val Ala Asp
   1               5                   10                  15
   Leu Asn Ala Ile Trp Ala Lys Leu Asp Asp Thr Thr Ala Gly Gly Glu
               20                  25                  30
   Pro Tyr Ile Phe Gln Glu Val Tyr Pro Gly Ser Thr Pro Ala Ala Ser
           35                  40                  45
   Asp Tyr Tyr Ser Ala Gly Asp Val Leu Asp Phe Thr Tyr Ala Ser Arg
       50                  55                  60
   Val Lys Ser Ala Phe Gln Gly Asn Val Ser Asp Leu Glu Ser Leu Pro
   65                  70                  75                  80
   Ser Ser Gly Val Leu Pro Pro Ala Asn Ser Val Ser Phe Val Asp Asn
                   85                  90                  95
   His Asp Thr Glu
               100
<210> 213
   <211> 100
   <212> PRT
   <213> Streptomyces sp. 8348
<400> 213
   Val Asp Gly Phe Arg Ile Asp Ala Ala Arg His Ile Asp Thr Ala Asp
   1 5 10 15
   Leu Ala Asn Ile Lys Ser Arg Leu Thr Asn Pro Asn Ala Tyr Arg Lys
   20 25 30
   Gln Glu Val Ile Tyr Gly Ser Gly Glu Ala Val Gln Pro Thr Glu Tyr
   35 40 45
   Thr Arg Asn Gly Asp Val Gln Glu Phe Arg Tyr Ala Tyr Asp Leu Lys
   50 55 60
   Arg Val Phe Asn Asn Glu Asn Pro Ala Tyr Leu Lys Asn Tyr Gly Glu
   65 70 75 80
   Gly Trp Gly Tyr Leu Ser Ser Pro Ser Ala Gly Val Tyr Val Asp Asn
   85 90 95
   His Asp Thr Glu
   100
<210> 214
   <211> 100
   <212> PRT
   <213> Streptomyces sp. B350
<400> 214
   Val Asp Gly Phe Arg Ile Asp Ala Ala Lys His Met Asp Ala Ala Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Lys Ser Gly Val Ser Gly Val Tyr Val Asp Asn
   85 90 95
   His Asp Thr Glu
   100
<210> 215
   <211> 100
   <212> PRT
   <213> Streptomyces sp. B352
<400> 215
   Val Asp Gly Phe Arg Ile Asp Thr Ala Lys His Met Asp Ala Ala Asp
   1 5 10 15
   Leu Ala Asn Ile Lys Ser Arg Leu Ser Asn Pro Ser Val Tyr Trp Lys
   20 25 30
   Gln Glu Ala Ile Tyr Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Asn Ser Ser Val Ala Gly Val Tyr Val Asp Asn
   85 90 95
   His Asp Thr Glu
   100
<210> 216
   <211> 100
   <212> PRT
   <213> Streptomyces sp. B353
<400> 216
   Val Asp Gly Phe Arg Ile Asp Thr Ala Lys His Met Asp Ala Ala Asp
   1 5 10 15
   Leu Ala Asn Ile Lys Ser Arg Leu Ser Asn Pro Ser Val Tyr Trp Lys
   20 25 30
   Gln Glu Val Ile His Gly Ser Gly Glu Ala Val Gln Pro Thr Glu Tyr
   35 40 45
   Leu Thr Thr Gly Asp Ala Gln Glu Phe Arg Tyr Ser Trp Asp Leu Lys
   50 55 60
   Arg Val Phe Thr Ser Glu Lys Leu Ala Tyr Leu Lys Asn Phe Gly Glu
   65 70 75 80
   Gly Trp Gly Tyr Met Ala Gly Gly Lys Ala Ser Val Phe Val Asp Asn
   85 90 95
   His Asp Thr Glu
   100
<210> 217
   <211> 100
   <212> PRT
   <213> Streptomyces sp. B354
<400> 217
   Val Asp Gly Phe Arg Ile Asp Thr Ala Lys His Met Asp Ala Ala Asp
   1 5 10 15
   Leu Ala Asn Ile Lys Ser Arg Leu Ser Asn Pro Ser Val Tyr Trp Lys
   20 25 30
   Gln Glu Ala Ile Phe Gly Ala Gly Glu Ala Val Gln Pro Thr Glu Tyr
   35 40 45
   Thr Gly Asn Gly Asp Val Gln Glu Phe Arg Tyr Gly Tyr Asp Leu Lys
   50 55 60
   Arg Val Phe Asn Ser Glu Asn Leu Ala Tyr Leu Lys Asn Tyr Gly Glu
   65 70 75 80
   Gly Trp Gly His Met Asn Ser Ser Val Ala Gly Val Tyr Val Asp Asn
   85 90 95
   His Asp Thr Glu
   100
<210> 218
   <211> 100
   <212> PRT
   <213> Streptomyces sp. B355
<400> 218
   Val Asp Gly Phe Arg Ile Asp Ala Ala Lys His Met Asp Ala Ala Asp
   1 5 10 15
   Leu Thr Asn Ile Lys Ser Arg Leu Ser Asn Pro Ser Ala Tyr Trp Lys
   20 25 30
   Gln Glu Ala Ile Phe Gly Ala Gly Glu Ala Val Gln Pro Thr Glu Tyr
   35 40 45
   Thr Gly Asn Gly Asp Val Gln Glu Phe Arg Tyr Gly Tyr Asp Leu Lys
   50 55 60
   Arg Val Phe Asp Asn Glu Asn Leu Ala Tyr Leu Arg Asn Tyr Gly Glu
   65 70 75 80
   Gly Trp Gly Tyr Met Asn Ser Ser Val Ala Gly Val Phe Val Asp Asn
   85 90 95
   His Asp Thr Glu
   100
<210> 219
   <211> 100
   <212> PRT
   <213> Streptomyces sp. B356
<400> 219
   Val Asp Gly Phe Arg Ile Asp Ala Ala Lys His Met Asp Ala Ala Asp
   1 5 10 15
   Leu Ala Asn Ile Lys Ser Arg Leu Thr Asn Pro Ser Val Tyr Trp Lys
   20 25 30
   Gln Glu Val Ile His Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Lys Ser Ser Gly Ala Gly Val Tyr Val Asp Asn
   85 90 95
   His Asp Thr Glu
   100
<210> 220
   <211> 100
   <212> PRT
   <213> Streptomyces sp. B357
<400> 220
   Val Asp Gly Phe Arg Ile Asp Ala Ala Lys His Met Ala Val Gly Asp
   1 5 10 15
   Leu Ala Asp Ile Lys Ser Arg Leu Gly Ser Pro Asp Val His Trp Lys
   20 25 30
   His Glu Ala Ile His Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Gly Val Tyr Val Asp Asn
   85 90 95
   His Asp Thr Glu
   100
<210> 221
   <211> 100
   <212> PRT
   <213> Streptomyces sp. B358
<400> 221
   Val Asp Gly Phe Arg Ile Asp Ala Ala Lys His Met Pro Ala Ala Asp
   1 5 10 15
   Leu Ala His Ile Lys Ser Arg Leu Thr Arg Pro Asp Val Tyr Trp Lys
   20 25 30
   Gln Glu Ala Ile His Gly Asp Gly Glu Ala Val Ser Pro Gly Glu Tyr
   35 40 45
   Leu Gly Ser Gly Asp Val Gln Glu Phe Arg His Ala Arg Asp Leu Lys
   50 55 60
   Arg Val Phe Gln Arg Glu Asn Leu Ala Tyr Leu Lys Asn Tyr Gly Glu
   65 70 75 80
   Gly Trp Gly Tyr Leu Pro Ser Ala Lys Ala Gly Val Tyr Val Asp Asn
   85 90 95
   His Asp Thr Glu
   100
<210> 222
   <211> 100
   <212> PRT
   <213> Streptomyces sp. B359
<400> 222
   Val Asp Gly Phe Arg Ile Asp Thr Ala Lys His Met Asp Ala Ala Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Arg Asn Tyr Gly Glu
   65 70 75 80
   Gly Trp Gly Tyr Met Asn Ser Gly Val Ser Gly Val Tyr Val Asp Asn
   85 90 95
   His Asp Thr Glu
   100
<210> 223
   <211> 100
   <212> PRT
   <213> Streptomyces sp. B360
<400> 223
   Val Asp Gly Phe Arg Ile Asp Ala Ala Lys His Ile Asp Thr Ala Asp
   1 5 10 15
   Leu Ala Asn Ile Lys Ser Arg Leu Ser Asn Pro Asn Ala Tyr Trp Lys
   20 25 30
   Gln Glu Val Ile His Gly Ser Gly Glu Ala Val Gln Pro Gly Glu Tyr
   35 40 45
   Thr Arg Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Ser Ser Ser Ser Ala Gly Val Tyr Val Asp Asn
   85 90 95
   His Asp Thr Glu
   100
<210> 224
   <211> 100
   <212> PRT
   <213> Streptomyces sp. B361
<400> 224
   Val Asp Gly Phe Arg Ile Asp Thr Ala Lys His Met Pro Val Gly Asp
   1 5 10 15
   Leu Asn Ala Ile Arg Ala Lys Leu Asp Asp Thr Thr Ser Gly Ala Glu
   20 25 30
   Pro Tyr Ile Phe Gln Glu Val Tyr Pro Gly Ala Thr Pro Ala Ala Ser
   35 40 45
   Asp Tyr Tyr Ser Ala Gly Asp Val Leu Asp Phe Thr Tyr Ala Ser Arg
   50 55 60
   Val Lys Ser Ala Phe Gln Gly Asn Val Ser Asp Leu Glu Ser Leu Pro
   65 70 75 80
   Ser Ser Gly Val Leu Thr Pro Ala Asn Ser Val Ser Tyr Val Asp Asn
   85 90 95
   His Asp Thr Glu
   100
<210> 225
   <211> 100
   <212> PRT
   <213> Streptomyces sp. B362
<400> 225
   Val Asp Gly Phe Arg Ile Asp Ala Ala Lys His Met Asp Ala Ala Asp
    1               5                  10                  15
   Leu Ala Xaa Ile Lys Ser Arg Leu Ser Asn Pro Ser Val Tyr Trp Lys
               20                  25                  30
   Gln Glu Ala Ile Tyr Gly Ser Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Ser Ser Gly Val Ser Gly Val Tyr Val Asp Asn
                   85                  90                  95
   His Asp Thr Glu
               100

<210> 226
   <211> 100
   <212> PRT
   <213> Streptomyces sp. B363org
<400> 226
   Val Asp Gly Phe Arg Ile Asp Thr Ala Lys His Met Pro Ala Ala Asp
    1               5                  10                  15
   Leu Ala Asn Ile Lys Ser Arg Leu Thr Asn Pro Ser Ala Tyr Trp Lys
               20                  25                  30
   Gln Gly Val Ile Tyr Gly Ala Gly Glu Ala Val Gln Pro Gly Glu Tyr
           35                  40                  45
   Thr Gly Thr Gly Asp Val Gln Glu Phe Arg Tyr Ala Tyr Asp Leu Lys
       50                  55                  60
   Arg Val Phe Asn Asp Glu Asn Leu Ala Tyr Leu Lys Asn Tyr Gly Glu
   65                  70                  75                  80
   Gly Trp Gly Tyr Leu Lys Ser Ser Ser Ala Gly Val Tyr Val Asp Asn
                   85                  90                  95
   His Asp Thr Glu
               100
<210> 227
   <211> 100
   <212> PRT
   <213> Streptomyces sp. B366
<400> 227
   Val Asp Gly Phe Arg Ile Asp Ala Ala Lys His Met Asp Ala Ala Asp
   1 5 10 15
   Leu Ala Asn Ile Lys Ser Arg Leu Thr Asn Thr Ser Val Tyr Trp Lys
   20 25 30
   Gln Glu Val Ile Tyr Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Ser Glu Asn Leu Ala His Leu Lys Asn Tyr Gly Glu
   65 70 75 80
   Gly Trp Gly Tyr Met Ser Ser Ser Val Ala Gly Val Tyr Val Asp Asn
   85 90 95
   His Asp Thr Glu
   100
<210> 228
   <211> 100
   <212> PRT
   <213> Streptomyces sp. B368
<400> 228
   Val Asp Gly Phe Arg Ile Asp Thr Ala Lys His Met Asp Thr Ala Asp
   1 5 10 15
   Leu Ala Thr Ile Lys Ser Arg Leu Thr Asn Pro Asn Ala Tyr Trp Lys
   20 25 30
   Gln Glu Val Ile Tyr Gly Ser Gly Glu Ala Val Gln Pro Thr Glu Tyr
   35 40 45
   Thr Arg Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Ser Val Ala Gly Val Tyr Val Asp Asn
   85 90 95
   His Asp Thr Glu
   100
<210> 229
   <211> 100
   <212> PRT
   <213> Streptomyces sp. B370
<400> 229
   Val Asp Gly Phe Arg Ile Asp Thr Ala Lys His Met Pro Ala Gly Asp
   1 5 10 15
   Leu Ala Asn Ile Lys Ser Arg Leu Ser Asn Pro Gly Val His Trp Lys
   20 25 30
   His Glu Ala Ile Tyr Gly Ala Gly Glu Ala Val Ser Pro Thr Glu Tyr
   35 40 45
   Leu Gly Ser Gly Asp Val Gln Glu Phe Arg Tyr Ala Arg Ser Leu Lys
   50 55 60
   Gln Thr Phe Asn Asn Glu Asn Leu Ala Asn Leu Lys Asn Phe Gly Glu
   65 70 75 80
   Gly Trp Gly Phe Met Glu Ser Gly Lys Ser Ala Val Phe Val Asp Asn
   85 90 95
   His Asp Thr Glu
   100
<210> 230
   <211> 100
   <212> PRT
   <213> Streptomyces sp. B371
<400> 230
   Val Asp Gly Phe Arg Ile Asp Thr Ala Lys His Met Asp Ala Ala Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Asn Ser Gly Val Ser Gly Val Phe Val Asp Asn
   85 90 95
   His Asp Thr Glu
   100
<210> 231
   <211> 100
   <212> PRT
   <213> Streptomyces sp. B372
<400> 231
   Val Asp Gly Phe Arg Ile Asp Thr Ala Lys His Met Asp Ala Ala Asp
   1               5                   10                  15
   Leu Ala Asn Ile Lys Ser Arg Leu Ser Asn Pro Ser Val Tyr Trp Lys
               20                  25                  30
   Gln Glu Val Ile Tyr Gly Ser Gly Glu Ala Val Gln Pro Thr Glu Tyr
           35                  40                  45
   Thr Ser Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                      80
   Gly Trp Gly Tyr Met Asn Ser Gly Val Ser Gly Val Tyr Val Asp Asn
                   85                  90                  95
   His Asp Thr Glu
               100
<210> 232
   <211> 100
   <212> PRT
   <213> Streptomyces sp. B373
<400> 232
   Val Asp Gly Phe Arg Ile Asp Thr Ala Lys His Met Asp Ala Ala Asp
   1               5                   10                  15
   Leu Ala Asn Ile Lys Ser Arg Leu Ser Asn Pro Ser Val Tyr Trp Lys
               20                  25                  30
   Gln Glu Ala Ile Tyr Gly Ser Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                      80
   Gly Trp Gly Tyr Met Ser Ser Gly Val Ser Gly Val Phe Val Asp Asn
                   85                  90                  95
   His Asp Thr Glu
               100
<210> 233
   <211> 100
   <212> PRT
   <213> Streptomyces sp. B374B
<400> 233
   Val Asp Gly Phe Arg Ile Asp Ala Ala Lys His Ile Asp Thr Ala Asp
    1               5                  10                  15
   Leu Ala Asn Ile Lys Ser Arg Leu Thr Asn Pro Asn Ala Tyr Trp Lys
               20                  25                  30
   Gln Glu Val Ile Tyr Gly Ser Gly Glu Ala Val Gln Pro Thr Glu Tyr
           35                  40                  45
   Thr Arg Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                      80
   Gly Trp Gly Tyr Leu Ser Ser Ser Ser Ala Gly Val Tyr Val Asp Asn
                   85                  90                  95
   His Asp Thr Glu
               100
<210> 234
   <211> 100
   <212> PRT
   <213> Streptomyces sp. B375
<400> 234
   Val Asp Gly Phe Arg Ile Asp Thr Ala Lys His Ile Asp Ser Ala Asp
    1               5                  10                  15
   Leu Ala Thr Ile Lys Ser Arg Leu Ser Asn Pro Ser Val Tyr Trp Lys
               20                  25                  30
   Gln Glu Val Ile Tyr Gly Ser Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                      80
   Gly Trp Gly Tyr Met Ser Ser Ser Val Ala Gly Val Tyr Val Asp Asn
                   85                  90                  95
   His Asp Thr Glu
               100
<210> 235
   <211> 100
   <212> PRT
   <213> Streptomyces sp. B376
<400> 235
   Val Asp Gly Phe Arg Ile Asp Ala Ala Lys His Met Asp Ala Ala Asp
    1               5                  10                  15
   Leu Ala Asn Ile Lys Ser Arg Leu Thr Asn Pro Ser Val Tyr Trp Lys
               20                  25                  30
   Gln Glu Val Ile His Gly Ala Gly Glu Ala Val Gln Pro Ala Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
    65                 70                  75                  80
   Gly Trp Gly Tyr Leu Lys Ser Ser Gly Ala Gly Val Tyr Val Asp Asn
                   85                  90                  95
   His Asp Thr Glu
               100
<210> 236
<211> 100
<212> PRT
   <213> Streptomyces sp. B380
<400> 236
   Val Asp Gly Phe Arg Ile Asp Thr Ala Lys His Met Asp Ala Ala Asp
    1               5                  10                  15
   Leu Ala Asn Ile Lys Ser Arg Leu Ser Asn Pro Ser Val Tyr Trp Lys
               20                  25                  30
   Gln Glu Ala Ile Tyr Gly Ser Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
    65                 70                  75                  80
   Gly Trp Gly Tyr Met Ser Ser Gly Val Ser Gly Val Tyr Val Asp Asn
                   85                  90                  95
   His Asp Thr Glu
               100
<210> 237
   <211> 100
   <212> PRT
   <213> Streptomyces sp. B382
<400> 237
   Val Asp Gly Phe Arg Ile Asp Ala Ala Lys His Ile Ala Ala Ala Asp
   1 5 10 15
   Leu Ala Asn Ile Lys Ser Arg Leu Thr Asn Pro Gly Val Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Cys Tyr Gly Tyr Asp Leu Lys
   50 55 60
   Arg Val Phe Asn Ser Glu Lys Leu Ala Tyr Leu Asn Asn Phe Gly Glu
   65 70 75 80
   Gly Trp Gly Tyr Ile Pro Gly Asn Val Ala Gly Val Tyr Val Asp Asn
   85 90 95
   His Asp Thr Glu
   100
<210> 238
   ziel> 100
   <212> PRT
   <213> Streptomyces sp. B390
<400> 238
<210> 239
   <211> 100
   <212> PRT
   <213> Streptomyces sp. B392A
<400> 239
   Val Asp Gly Phe Arg Ile Asp Ala Ala Lys His Met Ala Ala Ala Asp
   1               5                   10                  15
   Leu Ala Ala Ile Lys Ser Arg Leu Ser Asn Pro Asn Val Tyr Trp Lys
               20                  25                  30
   His Glu Ala Ile Tyr Ser Ala Gly Glu Ala Val Ser Pro Thr Glu Tyr
           35                  40                  45
   Val Gly Ser Gly Asp Val Gln Glu Phe Arg Tyr Ala Arg Asp Leu Lys
       50                  55                  60
   Arg Val Phe Asn Gly Glu Asn Leu Ala Tyr Leu Lys Asn Phe Gly Glu
   65                  70                  75                  80
   Ala Trp Gly His Leu Pro Ser Asp Glu Ala Ala Val Tyr Val Asp Asn
                   85                  90                  95
   His Asp Thr Glu
               100
<210> 240
   <211> 100
   <212> PRT
   <213> Streptomyces sp. B393
<400> 240
   Val Asp Gly Phe Arg Ile Asp Ala Ala Lys His Ile Ala Thr Glu Asp
   1               5                   10                  15
   Leu Ala Ala Val Lys Ala Lys Leu Ser Lys Pro Asp Val Tyr Trp Lys
               20                  25                  30
   Gln Glu Thr Ile Tyr Ser Ala Gly Glu Ala Val Gln Pro Gln Glu Tyr
           35                  40                  45
   Leu Gly Asn Gly Asp Val Gln Glu Phe Arg Tyr Ala Arg Asp Leu Lys
       50                  55                  60
   Arg Val Phe Gln Ser Glu Arg Leu Ala Tyr Leu Lys Asn Tyr Gly Glu
   65                  70                  75                  80
   Gly Trp Gly Tyr Leu Pro Gly Asp Arg Ala Ser Val Tyr Val Asp Asn
                   85                  90                  95
   His Asp Thr Glu
               100
<210> 241
   <211> 100
   <212> PRT
   <213> Streptomyces sp. B394
<400> 241
   Val Asp Gly Phe Arg Ile Asp Ala Ala Lys His Ile Ala Thr Glu Asp
   1               5                   10                  15
   Leu Ala Ala Val Lys Ala Lys Leu Ser Lys Pro Asp Val Tyr Trp Lys
               20                  25                  30
   Gln Glu Thr Ile Tyr Gly Ala Gly Glu Ala Val Gln Pro Gln Glu Tyr
           35                  40                  45
   Leu Gly Asn Gly Asp Val Gln Glu Phe Arg Tyr Ala Arg Asp Leu Lys
       50                  55                  60
   Arg Val Phe Gln Ser Glu Arg Leu Ala Tyr Leu Lys Asn Tyr Gly Glu
   65                  70                  75                  80
   Gly Trp Gly Tyr Leu Pro Gly Asp Arg Ala Ser Val Phe Val Asp Asn
                   85                  90                  95
   His Asp Thr Glu
               100
<210> 242
   <211> 100
   <212> PRT
   <213> Streptomyces sp. B395
<400> 242
   Val Asp Gly Phe Arg Ile Asp Ala Ala Lys His Ile Asp Thr Ala Asp
   1               5                   10                  15
   Leu Ala Asn Ile Lys Ser Arg Leu Thr Asn Pro Asn Ala Tyr Trp Lys
               20                  25                  30
   Gln Glu Val Ile Tyr Gly Ser Gly Glu Ala Val Gln Pro Thr Glu Tyr
           35                  40                  45
   Thr Arg Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Ser Ser Thr Ser Ala Gly Val Tyr Val Asp Asn
                   85                  90                  95
   His Asp Thr Glu
               100
<210> 243
   <211> 100
   <212> PRT
   <213> Streptomyces sp. B396(A)
<400> 243
   Val Asp Gly Phe Arg Ile Asp Ala Ala Lys His Ile Asp Thr Ala Asp
   1 5 10 15
   Leu Ala Asn Ile Lys Ser Arg Leu Thr Lys Pro Gly Val Tyr Trp Lys
   20 25 30
   Gln Glu Ala Ile Tyr Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Lys Tyr Gly Glu
   65 70 75 80
   Gly Trp Gly Tyr Met Ser Ser Ser Val Ala Gly Val Phe Val Asp Asn
   85 90 95
   His Asp Thr Glu
   100
<210> 244
   <211> 100
   <212> PRT
   <213> Streptomyces sp. B400
<400> 244
   Val Asp Gly Phe Arg Ile Asp Ala Ala Lys His Ile Pro Ala Ala Asp
   1 5 10 15
   Leu Ala Asp Ile Lys Ser Arg Leu Thr Asn Thr Ser Val Tyr Trp Lys
   20 25 30
   Gln Glu Ala Ile Tyr Gly Ser Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Ser Val Ser Gly Val Xaa Val Asp Asn
   85 90 95
   His Asp Thr Glu
   100
<210> 245
   <211> 100
   <212> PRT
   <213> Streptomyces sp. B4006
<400> 245
   Val Asp Gly Phe Arg Ile Asp Ala Ala Lys His Ile Asp Thr Ala Asp
   1 5 10 15
   Leu Ala Asn Ile Lys Ser Arg Leu Thr Asn Pro Asn Ala Tyr Trp Lys
   20 25 30
   Gln Glu Val Ile Tyr Gly Ser Gly Glu Ala Val Gln Pro Thr Glu Tyr
   35 40 45
   Thr Arg Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Leu Ser Ser Ser Ser Ala Gly Val Tyr Val Asp Asn
   85 90 95
   His Asp Thr Glu
   100
<210> 246
   <211> 103
   <212> PRT
   <213> Streptomyces sp. B4006B
<400> 246
   Val Asp Gly Phe Arg Ile Asp Ala Ala Lys His Ile Pro Glu Gly Asp
   1 5 10 15
   Leu Gln Ala Ile Leu Ser Arg Leu Lys Asn Val Tyr Pro Ala Trp Gly
   20 25 30
   Gly Gly Lys Pro Tyr Ile Phe Gln Glu Val Ile Ala Asp Ser Thr Ile
   35 40 45
   Ser Thr Gly Ser Tyr Thr His Leu Gly Ser Val Thr Glu Ser Gln Tyr
   50 55 60
   His Arg Asp Ile Ser His Ala Phe Ala Asn Gly Asn Ile Ala His Leu
   65 70 75 80
   Thr Gly Leu Gly Ser Gly Leu Thr Pro Ser Asp Lys Ala Val Val Tyr
   85 90 95
   Val Asp Asn His Asp Thr Glu
   100
<210> 247
   <211> 100
   <212> PRT
   <213> Streptomyces sp. B400A
<400> 247
   Val Asp Gly Phe Arg Ile Asp Ala Ala Lys His Ile Pro Ala Ala Asp
   1               5                   10                  15
   Leu Ala Asn Ile Lys Ser Arg Leu Ser Asn Pro Gly Val Tyr Trp Lys
               20                  25                  30
   His Glu Val Ile Tyr Gly Ala Gly Glu Ala Val Gln Pro Thr Glu Tyr
           35                  40                  45
   Thr Gly Ser Gly Asp Val Gln Glu Phe Arg Tyr Ala Tyr Asp Leu Lys
       50                  55                  60
   Arg Val Phe Thr Asn Glu Asn Leu Ala Tyr Leu Lys Asn Tyr Gly Glu
   65                  70                  75                  80
   Gly Trp Gly Tyr Leu Asn Ser Gly Val Ser Gly Val Tyr Val Asp Asn
                   85                  90                  95
   His Asp Thr Glu
               100
<210> 248
   <211> 100
   <212> PRT
   <213> Streptomyces sp. B400A2
<400> 248
   Val Asp Gly Phe Arg Ile Asp Thr Ala Lys His Ile Pro Ala Thr Asp
   1               5                   10                  15
   Leu Ala Asn Ile Lys Ser Arg Leu Thr Asn Pro Ser Val Tyr Trp Lys
               20                  25                  30
   Gln Glu Val Ile Tyr Gly Ser Gly Glu Ala Val Gln Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Asn Ser Ser Val Ala Gly Val Tyr Val Asp Asn
                   85                  90                  95
   His Asp Thr Glu
               100
<210> 249
   <211> 100
   <212> PRT
   <213> Streptomyces sp. B400B3
<400> 249
   Val Asp Gly Phe Arg Ile Asp Ala Ala Lys His Met Pro Ala Gly Asp
   1               5                   10                  15
   Leu Ala Asn Ile Lys Ser Arg Leu Ser Asn Pro Asn Val Tyr Trp Lys
               20                  25                  30
   His Glu Ala Ile Phe Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Ala Val Tyr Val Asp Asn
                   85                  90                  95
   His Asp Thr Glu
               100
<210> 250
   <211> 100
   <212> PRT
   <213> Streptomyces sp. B400C
<400> 250
   Val Asp Gly Phe Arg Ile Asp Ala Ala Lys His Met Pro Ala Ala Asp
   1               5                   10                  15
   Leu Thr Ala Ile Lys Ala Lys Val Gly Asp Gly Gly Thr Tyr Trp Lys
               20                  25                  30
   Gln Glu Ala Ile His Gly Ala Gly Glu Ala Val Gln Pro Ser Glu Tyr
           35                  40                  45
   Leu Gly Thr Gly Asp Val Gln Glu Phe Arg Tyr Ala Arg Asp Leu Lys
       50                  55                  60
   Arg Val Phe Gln Asn Glu Asn Leu Ala His Leu Lys Asn Phe Gly Glu
   65                  70                  75                  80
   Asp Trp Gly His Met Gln Ser Gly Arg Ser Ala Val Tyr Val Asp Asn
                   85                  90                  95
   His Asp Thr Glu
               100
<210> 251
   <211> 100
   <212> PRT
   <213> Streptomyces sp. B400C2
<400> 251
   Val Asp Gly Phe Arg Ile Asp Thr Ala Lys His Met Pro Ala Ala Asp
   1 5 10 15
   Leu Thr Ala Ile Lys Ala Lys Val Gly Asp Gly Gly Thr Tyr Trp Lys
   20 25 30
   Gln Gly Ala Ile His Gly Ala Gly Glu Ala Val Gln Ser Ser Glu Tyr
   35 40 45
   Leu Gly Thr Gly Asp Val Gln Glu Phe Arg Tyr Ala Arg Asp Leu Lys
   50 55 60
   Arg Val Phe Gln Asn Glu Asn Leu Ala His Leu Lys Asn Phe Gly Glu
   65 70 75 80
   Asp Trp Gly Arg Met Gln Ser Gly Arg Ser Ala Val Tyr Val Asp Asn
   85 90 95
   His Asp Thr Glu
   100
<210> 252
   <211> 100
   <212> PRT
   <213> Streptomyces sp. B400D
<400> 252
   Val Asp Gly Phe Arg Ile Asp Thr Ala Lys His Met Pro Ala Ala Asp
   1 5 10 15
   Leu Ala Asn Ile Lys Ser Arg Leu Thr Asp Pro Gly Ala Tyr Trp Lys
   20 25 30
   Gln Glu Ala Ile His Gly Ala Gly Glu Ala Val Ser Pro Ser Glu Tyr
   35 40 45
   Leu Gly Ser Gly Asp Val Gln Glu Phe Arg Tyr Ala Arg Asp Leu Lys
   50 55 60
   Arg Val Leu Gln Asn Glu Lys Leu Ala Tyr Leu Lys Asn Phe Gly Glu
   65 70 75 80
   Ala Trp Gly His Met Pro Ser Gly Arg Ala Gly Val Tyr Val Asp Asn
   85 90 95
   His Asp Thr Glu
   100
<210> 253
   <211> 100
   <212> PRT
   <213> Streptomyces sp. B400D2
<400> 253
   Val Asp Gly Phe Arg Ile Asp Xaa Ala Lys His Met Pro Ala Ala Asp
    1               5                  10                  15
   Leu Ala Asn Ile Lys Ser Arg Leu Thr Asp Pro Gly Ala Tyr Trp Lys
               20                  25                  30
   Gln Glu Ala Ile His Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Leu Gln Asn Glu Lys Leu Ala Tyr Leu Lys Asn Phe Gly Glu
   65                  70                  75                  80
   Ala Trp Gly His Met Pro Ser Gly Arg Ala Gly Val Tyr Val Asp Asn
                   85                  90                  95
   His Asp Thr Glu
               100
<210> 254
   <211> 100
   <212> PRT
   <213> Streptomyces sp. B400E
<400> 254
   Val Asp Gly Phe Arg Ile Asp Thr Ala Lys His Met Pro Ala Gly Asp
    1               5                  10                  15
   Leu Ala Asn Ile Lys Ser Arg Leu Ser Asn Pro Asn Val Tyr Trp Lys
               20                  25                  30
   His Glu Ala Ile Phe Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Ser Ala Val Tyr Val Asp Asn
                   85                  90                  95
   His Asp Thr Glu
               100
<210> 255
   <211> 100
   <212> PRT
   <213> Streptomyces sp. B400G
<400> 255
   Val Asp Gly Phe Arg Ile Asp Ala Ala Lys His Ile Pro Ala Ala Asp
   1 5 10 15
   Leu Ala Asn Ile Glu Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 256
   <211> 100
   <212> PRT
   <213> Streptomyces sp. B400G2
<400> 256
   Val Asp Gly Phe Arg Ile Asp Xaa Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Tyr Val Asp Asn
   85 90 95
   His Asp Thr Glu
   100
<210> 257
   <211> 100
   <212> PRT
   <213> Streptomyces sp. B400I
<400> 257
   Val Asp Gly Phe Arg Ile Asp Thr Ala Lys His Met Pro Ala Ala Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Leu Asn Glu Asn Leu Ala His Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100

<210> 258
   <211> 100
   <212> PRT
   <213> Streptomyces sp. B400J
<400> 258
   Val Asp Gly Phe Arg Ile Asp Ala Ala Lys His Met Pro Val Ala Asp
   1 5 10 15
   Leu Asn Ala Ile Trp Ala Lys Leu Asp Asn Thr Thr Ser Gly Ala Glu
   20 25 30
   Pro Tyr Ile Phe Gln Glu Val Tyr Pro Gly Ser Thr Pro Ala Ala Ser
   35 40 45
   Asp Tyr Tyr Ser Ala Gly Asp Val Leu Asp Phe Ser Tyr Ala Ser Lys
   50 55 60
   Val Lys Ser Ser Phe Gln Gly Asn Ile Ser Asp Leu Glu Ser Leu Pro
   65 70 75 80
   Ser Ser Gly Ala Leu Thr Pro Ala Asn Ser Val Ser Tyr Val Asp Asn
   85 90 95
   His Asp Thr Glu
   100
<210> 259
   <211> 100
   <212> PRT
   <213> Streptomyces sp. B400K
<400> 259
   Val Asp Gly Phe Arg Ile Asp Ala Ala Lys His Met Pro Val Ala Asp
   1 5 10 15
   Leu Asn Ala Ile Trp Ala Lys Leu Asp Asn Thr Thr Ser Gly Ala Glu
   20 25 30
   Pro Tyr Ile Phe Gln Glu Val Tyr Pro Gly Ser Thr Pro Ala Ala Ser
   35 40 45
   Asp Tyr Tyr Ser Ala Gly Asp Val Leu Asp Phe Ser Tyr Ala Ser Lys
   50 55 60
   Val Lys Ser Ser Phe Gln Gly Asn Ile Ser Asp Leu Glu Ser Leu Pro
   65 70 75 80
   Ser Ser Gly Ala Leu Thr Pro Ala Asn Ser Val Ser Tyr Val Asp Asn
   85 90 95
   His Asp Thr Glu
   100
<210> 260
   <211> 100
   <212> PRT
   <213> Streptomyces sp. B400L
<400> 260
   Val Asp Gly Phe Arg Ile Asp Ala Ala Lys His Met Pro Val Ala Asp
   1 5 10 15
   Leu Asn Ala Ile Trp Ala Lys Leu Asp Asn Thr Thr Ser Gly Ala Glu
   20 25 30
   Pro Tyr Ile Phe Gln Glu Val Tyr Pro Gly Ser Thr Pro Ala Ala Ser
   35 40 45
   Asp Tyr Tyr Ser Ala Gly Asp Val Leu Asp Phe Ser Tyr Ala Ser Lys
   50 55 60
   Val Lys Ser Ser Phe Gln Gly Asn Ile Ser Asp Leu Glu Ser Leu Pro
   65 70 75 80
   Ser Ser Gly Ala Leu Thr Pro Ala Asn Ser Val Ser Tyr Val Asp Asn
   85 90 95
   His Asp Thr Glu
   100
<210> 261
   <211> 100
   <212> PRT
   <213> Streptomyces sp. B902.
<400> 261
   Val Asp Gly Phe Arg Ile Asp Ala Ala Lys His Met Pro Ala Ala Asp
   1               5                   10                  15
   Leu Ala Asn Ile Lys Ser Arg Leu Thr Asp Pro Gly Ala Tyr Trp Lys
               20                  25                  30
   Gln Glu Ala Ile His Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Leu Gln Asn Glu Lys Leu Ala Tyr Leu Lys Asn Phe Gly Glu
   65                  70                  75                  80
   Ala Trp Gly His Met Pro Ser Gly Arg Ala Gly Val Tyr Val Asp Asn
                   85                  90                  95
   His Asp Thr Glu
               100
<210> 262
   <211> 100
   <212> PRT
   <213> Streptomyces sp. B907
<400> 262
   Val Asp Gly Phe Arg Ile Asp Ala Ala Lys His Met Pro Ala Ala Asp
   1               5                   10                  15
   Leu Ala Asn Ile Lys Thr Arg Leu Ala Asn Pro Asn Val Tyr Trp Lys
               20                  25                  30
   His Glu Ala Ile Tyr Gly Ala Gly Glu Ala Val Ser Pro Ser Glu Tyr
           35                  40                  45
   Leu Gly Ser Gly Asp Val Gln Glu Phe Arg Tyr Gly Arg Ser Leu Lys
       50                  55                  60
   Gln Val Phe Leu Asn Glu Asn Leu Ala His Leu Lys Asn Phe Gly Glu
   65                  70                  75                  80
   Gly Trp Gly Phe Met Glu Ser Gly Lys Pro Gly Val Tyr Val Asp Asn
                   85                  90                  95
   His Asp Thr Glu
               100
<210> 263
   <211> 100
   <212> PRT
   <213> Künstliche Sequenz
<220>
   <221> VARIANT
   <222> (6)
   <223> Ile or Leu
<220>
   <221> VARIANT
   <222> (9)
   <223> Ala or Ser
<220>
   <221> VARIANT
   <222> (10)
   <223> Lys or Arg
<220>
   <221> VARIANT
   <222> (12)
   <223> Met or Ile
<220>
   <221> VARIANT
   <222> (13)
   <223> Ser or Pro or Asp or Ala
<220>
   <221> VARIANT
   <222> (14)
   <223> Ala or Thr or Ser or Glu or Val
<220>
   <221> VARIANT
   <222> (15)
   <223> Asp or Ala or Glu or Thr or Gly or Ser
<220>
   <221> VARIANT
   <222> (16)
   <223> Asp or Gly
<220>
   <221> VARIANT
   <222> (17)
   <223> Val or Leu or Pro or Ile
<220>
   <221> VARIANT
   <222> (18)
   <223> Ala or Thr or Glu or Asn or Gln
<220>
   <221> VARIANT
   <222> (19)
   <223> Ala or Asn or Asp or His or Thr or Pro
<220>
   <221> VARIANT
   <222> (20)
   <223> Ile or Val
<220>
   <221> VARIANT
   <222> (21)
   <223> Lys or Glu or Trp or Arg or Leu
<220>
   <221> VARIANT
   <222> (22)
   <223> Gly or Ser or Pro or Ala or Thr
<220>
   <221> VARIANT
   <222> (23)
   <223> Lys or Arg
<220>
   <221> VARIANT
   <222> (24)
   <223> Met or Leu or Val or Pro
<220>
   <221> VARIANT
   <222> (25)
   <223>
<220>
   <221> VARIANT
   <222> (26)
   <223> Asp or Asn or Ser or Lys or Arg
<220>
   <221> VARIANT
   <222> (27)
   <223> Pro or Arg or Thr or Gln or Gly or Val
<220>
   <221> VARIANT
   <222> (28)
   <223>
<220>
   <221> VARIANT
   <222> (29)
   <223> Val or Ala or Thr or Pro or none
<220>
   <221> VARIANT
   <222> (30)
   <223> Phe or Tyr or His
<220>
   <221> VARIANT
   <222> (31)
   <223> Trp or Arg or Ile or Leu
<220>
   <221> VARIANT
   <222> (32)
   <223> Val or Lys or Phe
<220>
   <221> VARIANT
   <222> (33)
   <223> Thr or Gln or His or Leu or Met
<220>
   <221> VARIANT
   <222> (34)
   <223> Glu or Gly
<220>
   <221> VARIANT
   <222> (35)
   <223> Val or Ala or Thr
<220>
   <221> VARIANT
   <222> (36)
   <223> Ile or Thr or Met or Tyr or Asn
<220>
   <221> VARIANT
   <222> (37)
   <223> His or Tyr or Phe or Gly or Pro or Ala
<220>
   <221> VARIANT
   <222> (38)
   <223> Gly or Ala or Ser or Trp or Asp
<220>
   <221> VARIANT
   <222> (39)
   <223> Gly or Ala or Asp or Ser
<220>
   <221> VARIANT
   <222> (40)
   <223> Gly or Asn or Thr
<220>
   <221> VARIANT
   <222> (41)
   <223> Glu or Pro or Ile
<220>
   <221> VARIANT
   <222> (42)
   <223> Ala or Thr or Pro or Ser
<220>
   <221> VARIANT
   <222> (43)
   <223> Val or Ile or Ala or Thr
<220>
   <221> VARIANT
   <222> (44)
   <223> Gln or Ser or Gly
<220>
   <221> VARIANT
   <222> (45)
   <223> Pro or Ser or none
<220>
   <221> VARIANT
   <222> (46)
   <223>
<220>
   <221> VARIANT
   <222> (47)
   <223> Glu or Asp or Ser
<220>
   <221> VARIANT
   <222> (48)
   <223> Tyr or His
<220>
   <221> VARIANT
   <222> (49)
   <223> Thr or Leu or Tyr or Val or Ala
<220>
   <221> VARIANT
   <222> (50)
   <223> Ser or Gly or Asp or Arg or Thr or Asn or His
<220>
   <221> VARIANT
   <222> (51)
   <223> Ile or Ser or Asn or Ala or Thr or Leu
<220>
   <221> VARIANT
   <222> (52)
   <223> Gly or Cys
<220>
   <221> VARIANT
   <222> (53)
   <223> Asp or Ser
<220>
   <221> VARIANT
   <222> (54)
   <223> Val or Ala or Ser
<220>
   <221> VARIANT
   <222> (55)
   <223> Asp or Gln or His or Leu or Thr
<220>
   <221> VARIANT
   <222> (56)
   <223> Glu or Asp
<220>
   <221> VARIANT
   <222> (57)
   <223> Phe or Leu or Ser
<220>
   <221> VARIANT
   <222> (58)
   <223> Arg or Ser or Cys or His or Asp or Thr or Gln
<220>
   <221> VARIANT
   <222> (59)
   <223> Tyr or His
<220>
   <221> VARIANT
   <222> (60)
   <223> Gly or Ala or Ser or His
<220>
   <221> VARIANT
   <222> (61)
   <223> Gly or Ser or Tyr or Arg or Trp or Lys
<220>
   <221> VARIANT
   <222> (62)
   <223> His or Asp or Ser or Gly or Gln or Arg or Lys
<220>
   <221> VARIANT
   <222> (63)
   <223> Leu or Val or Ile
<220>
   <221> VARIANT
   <222> (64)
   <223> Lys or Ser or Ala
<220>
   <221> VARIANT
   <222> (65)
   <223> Ser or Arg or Gln or His
<220>
   <221> VARIANT
   <222> (66)
   <223> Ala or Val or Ile or Thr or Ser or Asp or Met
<220>
   <221> VARIANT
   <222> (67)
   <223> Phe or Val or Leu
<220>
   <221> VARIANT
   <222> (68)
   <223>
<220>
   <221> VARIANT
   <222> (69)
   <223> Gly or Asn or Gln or Arg or Ser or Asp or none
<220>
   <221> VARIANT
   <222> (70)
   <223> Gly or Glu or none
<220>
   <221> VARIANT
   <222> (71)
   <223>
<220>
   <221> VARIANT
   <222> (72)
   <223> Leu or Pro or Ile or Val
<220>
   <221> VARIANT
   <222> (73)
   <223> Pro or Ala or Thr or Ser or Lys
<220>
   <221> VARIANT
   <222> (74)
   <223> Gly or Gln or Tyr or His or Glu or Asp or Asn
<220>
   <221> VARIANT
   <222> (76)
   <223> Arg or Asn or Ser or Glu or Thr
<220>
   <221> VARIANT
   <222> (77)
   <223> Ser or Asn or Lys or Thr or Tyr or Gly
<220>
   <221> VARIANT
   <222> (78)
   <223> Ile or Val or Tyr or Phe or Leu
<220>
   <221> VARIANT
   <222> (79)
   <223> Ala or Gly or Ser or Asp or Pro
<220>
   <221> VARIANT
   <222> (80)
   <223> Asp or Glu or Val or Ser
<220>
   <221> VARIANT
   <222> (81)
   <223> Gly or Asp or Ala or Ser or none
<220>
   <221> VARIANT
   <222> (82)
   <223> Gly or Trp or none
<220>
   <221> VARIANT
   <222> (83)
   <223> Gly or Val or Ala or none
<220>
   <221> VARIANT
   <222> (84)
   <223> Lys or Tyr or His or Arg or Phe or Leu
<220>
   <221> VARIANT
   <222> (85)
   <223> Leu or Met or Ile or Val or Pro or Thr
<220>
   <221> VARIANT
   <222> (86)
   <223>
<220>
   <221> VARIANT
   <222> (87)
   <223> Gly or Ser or Asn or Tyr or Ala
<220>
   <221> VARIANT
   <222> (88)
   <223> Ala or Asp or Gly or Ser or Thr or Pro or Asn
<220>
   <221> VARIANT
   <222> (89)
   <223>
<220>
   <221> VARIANT
   <222> (90)
   <223> Ala or Ser or Pro
<220>
   <221> VARIANT
   <222> (91)
   <223> Arg or Gly or Ser or Asn or Ala or Val
<220>
   <221> VARIANT
   <222> (92)
   <223> Thr or Val or Ser
<220>
   <221> VARIANT
   <222> (93)
   <223> Tyr or Phe or Trp
<220>
   <221> VARIANT
   <222> (100)
   <223> Glu or Gln
<220>
   <221> VARIANT
   <222> (8)
   <223> Ala or Thr or Gly
<220>
   <223> Consensus-Sequenz
<400> 263
   Val Asp Gly Phe Arg Xaa Asp Xaa Xaa Xaa His Xaa Xaa Xaa Xaa Xaa
    1               5                  10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Leu Xaa Xaa Xaa Xaa Xaa
   65                  70                  75                  80
   Xaa Xaa Xaa Xaa Xaa Xaa Xaa Xaa Xaa Xaa Xaa Xaa Xaa Val Asp Asn
                   85                  90                  95
   His Asp Thr Xaa
               100

## Beschreibung

Die vorliegende Erfindung betrifft eine neue Gruppe von α-Amylasen, die einem gemeinsamen Sequenzraum angehören, sowie zu diesen α-Amylasen hinreichend ähnliche Proteine mit amylolytischer Funktion, Verfahren zu deren Herstellung und diverse Einsatzmöglichkeiten für diese amylolytischen Proteine, insbesondere in Wasch- und Reinigungsmitteln.

α-Amylasen (E.C. 3.2.1.1) hydrolysieren im Polymerinneren gelegene α-1,4-glycosidische Bindungen von Stärke und stärkeähnlichen Polymeren, wie beispielsweise Amylose, Amylopektin oder Glykogen, unter Bildung von Dextrinen und β-1,6-verzweigten Oligosacchariden. Sie gehören zu den wichtigsten industriell genutzten Enzymen überhaupt Dies aus zwei Gründen: Zum einen werden sie zumeist wie viele substratabbauende Enzyme von Mikroorganismen in das umgebende Medium abgegeben, so daß sie durch Fermentation und Aufreinigung aus dem Kulturmedium mit vergleichsweise geringem Aufwand in industriellem Maßstab gewonnen werden können. Zum anderen werden Amylasen für ein breites Anwendungsspektrum benötigt

An erster Stelle der technischen Verwendungen von α-Amylase steht die Herstellung von Glucosesirup. Andere Verwendungen sind beispielsweise die als aktiven Komponenten in Wasch- und Reinigungsmitteln, zur Behandlung von Rohmaterialien in der Textilherstellung, zur Herstellung von Klebstoffen oder zur Herstellung von zuckerhaltigen Lebensmitteln oder Lebensmittelbestandteilen.

Ein Beispiel für eine technisch besonders intensiv eingesetzte Amylase ist die α-Amylase aus *Bacillus licheniformis,* die von der Firma Novo Nordisk A/S, Bagsvaerd, Dänemark, unter dem Handelsnamen Termamyl^{Ⓡ} angeboten wird. Die aus *B. subtilis,* beziehungsweise *B. amyloliquefaciens* gewonnene und in der US-Anmeldung US 1 227 374 offenbarte Amylase wird von derselben Firma unter dem Namen BAN^{Ⓡ} vertrieben.

Dieses Amylase-Molekül, beziehungsweise dessen nahen Verwandte, sind in zahlreichen Erfindungen weiterentwickelt worden, denen die Aufgabe zugrunde gelegen hat, mithilfe diverser molekularbiologischer Modifikationen ihre enzymatischen Eigenschaften auf spezifische Anwendungen hin zu optimieren. Solche Optimierungen können beispielsweise die Substratspezifitäten, die Stabilität des Enzyms unter verschiedenen Reaktionsbedingungen oder die enzymatische Aktivität selbst betreffen. Beispielhaft für solche Optimierungen seien folgende Anmeldungen genannt: EP 0410498 für das Schlichten von Textilien und WO 96/02633 zur Stärkeverflüssigung.

Da Entwicklungen, die lediglich in Optimierungen von nur wenigen bekannten Ausgangsenzymen bestehen, möglicherweise in den erzielbaren Ergebnissen beschränkt sind, findet parallel dazu eine intensive Suche nach vergleichbaren Enzymen aus anderen natürlichen Quellen statt. Identifiziert wurden stärkespaltende Enzyme beispielsweise aus *Pimelobacter, Pseudomonas* und *Thermus* für die Lebensmittetherstellung, Kosmetik und Pharmaka (EP 0 636 693), ebensolche aus *Rhizobium, Arthrobacter, Brevibacterium* und *Micrococcus* (EP 0 628 630), aus *Pyrococcus* (WO 94/19454) und *Sulfolobus* zur Stärkeverflüssigung bei hohen Temperaturen, beziehungsweise stark sauren Reaktionsbedingungen (EP 0 727 485 und WO 96/02633). Für den Einsatz bei alkalischen pH-Werten sind Amylasen aus *Bacillus sp.* (WO 95/26397 und WO 97/00324) gefunden worden. Wegen ihrer geringen Empfindlichkeit gegenüber Detergenzien eignen sich andere Amylasen aus verschiedenen *Bacilli* (EP 0 670 367) zur Verwendung in Wasch- oder Reinigungsmitteln.

Weitere Optimierungen der aus natürlichen Quellen isolierten Enzyme für das jeweilige Anwendungsgebiet können beispielsweise über molekularbiologische Methoden (etwa gemäß US 5171673 oder WO 99/20768) oder über chemische Modifikationen vorgenommen werden (DE 4013142). In der Patentanmeldung WO 99/43793, beispielsweise, wird eine Weiterenhvicklung der bekannten Novamyl^{Ⓡ}-α-Amylase beschrieben. Darin werden Sequenzähnlichkeiten zwischen Novamyl^{®} und bekannten Cyclodextringlucanotransferasen (CGTasen) ausgenutzt, um mithilfe molekularbiologischer Techniken eine Schar verwandter Moleküle zu konstruieren. Bei diesen handelt es sich um α-Amylasen mit zusätzlichen CGTase-spezifischen Consensus-Sequenzen (Boxen) und Funktionen oder, umgekehrt, um CGTasen mit zusätzlichen für α-Amylasen typischen Bereichen und Funktionen oder um Chimären beider Moleküle. Der Sinn dieser Entwicklung besteht darin, Novamyl^{®} für diese Anwendungen zu optimieren.

Die Anmeldung WO 99/57250, beispielsweise, gibt eine Lehre an die Hand, für dies Verwendung in Wasch- und Reinigungsmitteln geeignete Enzyme über einen chemischen Linker mit einer Bindungsdomäne zu verknüpfen, welche die effektive Enzymkonzentration auf dem Reinigungsgut erhöht.

Eine moderne Richtung der Enzymentwicklung besteht darin, Elemente aus bekannten, miteinander verwandten Proteinen über statistische Verfahren zu neuen Enzymen zu kombinieren, die bislang nicht erreichte Eigenschaften aufweisen. Solche Verfahren werden auch unter dem Oberbegriff Directed Evolution zusamengefaßt. Dazu gehören beispielsweise folgende Verfahren: Die StEP-Methode (Zhao et al. (1998), Nat. Biotechnol., Band 16, S. 258-261), Random priming recombination (Shao et al., (1998), Nucleic Acids Res., Band 26, S. 681-683), DNA-Shuffling (Stemmer, W.P.C. (1994), Nature, Band 370, S. 389-391) oder RACHITT (Coco, W.M. et al. (2001), Nat. Biotechnol., Band 19, S. 354-359).

Voraussetzung zur Rekombination über derartige Verfahren ist, daß auf den eingesetzten Nukleinsäuren jeweils genügend lange Bereiche vorhanden sind, um unter den jeweiligen Bedingungen eine Hybridisierung zu erreichen. Für eine erfolgreiche Hybridisierung sind bei den Ausgangssequenzen Identitäten von mehr als 45% auf Aminosäure-Ebene zueinander als praktikabel anzusehen, zur Forcierung der homologen Neukombination von mehr 50%. Wenigstens zwei verschiedene, zueinander homologe Sequenzen definieren bereits einen Sequenzraum. Dieser enthält alle über Rekombination aus den Ausgangssequenzen theoretisch ableitbaren neuen Sequenzen. Sie können hierfür auch Homologiewerte von weniger als 45% aufweisen, sofern die von ihnen abgeleiteten Nukleinsäuren nach einer der im Stand der Technik etablierten Methoden zur Rekombination gebracht werden können.

Trotz all dieser Entwicklungen besteht aber unverändert die Aufgabe, neben den wenigen natürlichen amylolytischen Enzymen, die unverändert oder in Gestalt von Weiterentwicklungen tatsächlich industriell genutzt werden, weitere aufzufinden, die a *priori* ein breites Anwendungsspektrum aufweisen und als Ausgangspunkt für anwendungsspezifische Weiterentwicklungen, insbesondere für statistische Rekombinationsverfahren dienen können.

Die genetische Vielfalt der gram-positiven Bakterien-Ordnung der Actinomycetales, insbesondere der Gattung Streptomyces ist bislang kaum auf amylolytische Proteine hin untersucht worden, die für technische Zwecke geeignet sind. Lediglich zwei japanische Patentanmeldungen sind in diesem Zusammenhang zu betrachten. In der Anmeldung JP-A 62-143999 wird eine für die Verwendung in Wasch- oder Reinigungsmitteln geeignete α-Amylase aus einem Vertreter der Gattung Streptomyces offenbart. Dieser Organismus *Streptomyces sp.* KSM-9 oder FERM P-7620 stammt aus einem natürlichen Habitat und wächst im alkalischen Medium. Das amylolytische Enzym wird in dieser Schrift lediglich über enzymatischen Parameter und seine Eignung für den Einsatz in Wasch- und Reinigungsmitteln beschrieben, nicht jedoch über seine DNA- oder Aminosäuresequenz.

Das Enzym, das in der Anmeldung JP-A 2000-60546 offenbart wird und aus *Streptomyces sp.* TOTO-9805 oder FERM BP-6359 stammt, weist ähnliche enzymatische Eigenschaften wie das Enzym aus *Streptomyces sp.* KSM-9 auf. Es wird in dieser Anmeldung aber auch lediglich über enzymatische Parameter und nicht über die Aminosäure- oder Nukleotidsequenz charakterisiert. Hierdurch stehen beide Amylasen weder für eine heterologe Expression und Gewinnung noch für eine anwendungsspezifische Auswahl und Optimierung zur Verfügung. Denn sowohl klassische Mutageneseverfahren als auch Methoden der gerichteten Evolution (EP-PCR, Sequence-Shuffling, Family-Shuffling) beruhen auf den zugehörigen Nukleinsäuresequenzen.

Der vorliegenden Erfindung hat somit primär die Aufgabe zugrunde gelegen, natürliche, bislang noch nicht beschriebene α-Amylasen zu identifizieren, welche selbst für technische Einsatzmöglichkeiten geeignet sind oder als Grundlage für anwendungsspezifische Weiterentwicklungen dienen können.

Vorzugsweise sollte die Lösung dieser Aufgabe mit dem Auffinden mehrerera-Amylasen oder von Teilsequenzen mehrerer α-Amylasen als erreicht angesehen werden, die miteinander verwandt sind und homologisiert werden können. Denn aus einer solchen Homologisierung kann ein Sequenzraum abgeleitet werden, welcher seinerseits als Ausgangspunkt für die Erzeugung weiterer Enzyme dienen kann,

Eine Teilaufgabe bestand darin, die für derartige α-Amylasen codierenden Nukleinsäuren zu erhalten, da diese sowohl für die biotechnologische Herstellung als auch für die Weiterentwicklung dieser Enzyme unerläßlich sind.

Eine weitere Teilaufgabe bestand darin, die Organismen aufzufinden, welche die betreffenden α-Amylasen natürlichetweise produzieren.

Eine weitere Teilaufgabe bestand darin, ein Verfahren zu finden, nach welchem ein derartiger Pool an α-Amylasen bereitgestellt werden kann.

Als weitere Teilaufgabe sollten die erhaltenen α-Amylasen, α-Amylasefragmente oder α-Amylasegene oder deren Fragmente zur Auffindung oder Entwicklung neuer Enyzme genutzt werden können.

Eine weitere Teilaufgabe bestand darin, die biotechnologische Produktion der gefundenen oder ableitbaren α-Amylasen zu ermöglichen.

Eine weitere Teilaufgabe bestand darin, technische Einsatzmöglichkeiten für die gefundenen α-Amylasen zu definieren.

Die Lösung der ersten Aufgabe und damit den ersten Erfindungsgegenstand stellen amylolytische Proteine dar, wie in den Ansprüchen 1 und 2 definiert.

Hierzu gehören amylolytlsche Proteine mit den im Sequenzprotokoil unter SEQ ID NO.208 angegebenen Aminosäuresequenzen. sowie Enzyme, die zu diesen hinreichende Ahnlicheit aufweisen oder mit an sich bekannten Methoden von diesen abgeleitet werden können. Bevorzugte Vertreter lassen sich aus natürlichen Organismen, insbesondere denen der Ordnung Actinomycetales isolieren.

Aus diesen Sequenzen läßt sich über Homologisierung ein Sequenzraum ableiten, welcher seinerseits als Ausgangspunkt für die Erzeugung weiterer Enzyme dienen kann.

Den zweiten Erfindungsgegenstand bilden Nukleinsäuren, gemäß Anspruch 7 oder 8..

Hierzu gehören entsprechend bevorzugt die Nukleinsäuren, die für die jeweiligen Proteine des ersten Erfindungsgegenstands codieren,

Einen weiteren Erfindungsgegenstand bilden Vektoren mit den Nukleinsäuren des zweiten Erfindungsgegenstands, mit derartigen Vektoren transformierte Wirtszellen und alle biotechnologischen Verfahren zur Herstellung eines Proteins nach dem ersten Erfindungsgegenstand.

Einen weiteren Erfindungsgegenstand bilden die technischen Einsatzmöglichkeiten für die gefundenen α-Amylasen. Hierzu gehören Wasch- oder Reinigungsmittel, die dadurch gekennzeichnet sind, daß sie ein Protein gemäß dem ersten Erfindungsgegenstand enthalten, Verfahren zur Stärkevemüssigung, insbesondere zur Ethanolproduktion, temporäre Kiebeverfahren und diverse Verwendungsmöglichkeiten, insbesondere zur Behandlung von Rohmaterialien oder Zwischenprodukten in der Textilherstellung, insbesondere zum Entschlichten von Baumwolle, zur Herstellung von linearen und/oder kurzkettigen Oligosacchariden, zur Hydrolyse von Cyclodextrinen, zur Freisetzung von niedermolekularen Verbindungen aus Polysaccharidträgem oder Cyclodextrinen, zur Herstellung von Lebensmitteln und/oder Lebensmittelbestandtellen, zur Herstellung von Tierfutter und/oder Tierfutterbestandteilen und zur Auflösung stärkehaitiger Klebeverbindungen.

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet.

Unter einem Enzym ist im Sinne der vorliegenden Anmeldung ein Protein zu verstehen, das eine bestimmte biochemische Funktion ausübt. Unter amylolytischen Proteinen oder Enzymen mit amylolytischer Funktion sind solche zu verstehen, die α-1,4-glykosidische Bindungen von Polysacchariden hydrolysieren, insbesondere solche, die im Inneren der Polysaccharide liegen. Sie werden auch als α-1,4-Amylasen (E.C. 3.2.1.1) oder kurz: α-Amylasen bezeichnet.

Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedigungen durch eine Signalpeptidase vom übrigen Protein abgespalten, so daß dieses seine eigentliche katalytische Aktivität ohne die zunächst vorhandenen N-terminalen Aminosäuren ausübt. Die native α-Amylase aus *Streptomyces sp.* B327*, beispielsweise, ist, wie in SEQ ID NO. 6 gezeigt ist, 461 Aminosäuren lang. Wie in SEQ ID NO. 5 dargestellt ist, umfaßt das Signalpeptid dieses Enzyms 30 Aminosäuren, so daß sich für das **mature Enzym** eine Länge von 431 Aminosäuren ergibt.

Für technische Anwendungen sind aufgrund ihrer enzymatischen Aktivität die **maturen Peptide,** das heißt die nach ihrer Herstellung prozessierten Enzyme gegenüber den Präproteinen bevorzugt.

**Pro-Proteine** sind inaktive Vorstufen von Proteinen. Deren Vorläufer mit Signalsequenz werden als **Prä-Pro-Proteine** bezeichnet.

Unter **Nukleinsäuren** sind im Sinne der vorliegenden Anmeldung die natürlicherweise aus Nukleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzeistrang oder als Doppelstrang vorliegen. Als der natürlicherweise dauerhaftere informationsträger ist die Nukleinsäure **DNA** für molekularbiologische Arbeiten bevorzugt. Demgegenüber wird für die Realisierung der Erfindung in natürlicher Umgebung, wie beispielsweise in einer exprimierenden Zelle, eine **RNA** gebildet, weshalb erfindungswesentliche RNA-Moleküle ebenfalls Ausführungsformen der vorliegenden Erfindung darstellen.

Bei DNA sind die Sequenzen beider komplementärer Stränge in jeweils allen drei möglichen Leserastern zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann **(Degeneriertheit des genetischen Codes).** Außerdem weisen verschiedene Organismen Unterschiede im **Gebrauch dieser Codons** auf. Aus diesen Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als **Gen** bezeichnet.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Änderungen der Nukleotidsequenz, wie sie beispielsweise durch an sich bekannte molekularbiologische Methoden herbeigeführt werden können, werden als **Mutationen** bezeichnet. Je nach Art der Änderung kennt man beispielsweise Deletions-, **Insertions-** oder **Substitutionsmutationen** oder solche, bei denen verschiedene Gene oder Teile von Genen miteinander **fusioniert (shuffling)** werden; dies sind Genmutationen. Die zugehörigen Organismen werden als **Mutanten** bezeichnet. Die von mutierten Nukleinsäuren abgeleiteten Proteine werden als **Varianten** bezeichnet. So führen beispielsweise Deletions-, Insertions- Substitutionsmutationen oder Fusionen zu deletions-, insertions- substitutionsmutierten oder Fusionsgenen und auf Proteinebene zu entsprechenden Deletions-, Insertions- oder Substitutionsvarianten, beziehungsweise Fusionsproteinen.

Unter **Fragmenten** werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Den Fragmenten entsprechen auf Nukleinsäure-Ebene die **Teilsequenzen.**

Unter **chimären** oder **hybriden** Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch **Shuffling** oder **Fusionsmutagenese** genannt. Der Sinn einer solchen Fusion kann beispielsweise darin bestehen, mithilfe des heranfusionierten Proteinteils eine bestimmte enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können. Zur Realisierung der letztgenannten Alternative ist es beispielsweise möglich, posttranslational oder erst nach einem Aufreinigungsschritt durch eine gezielte proteolytische Spaltung eine einzelne chimäre Polypeptidkette in mehrere zu zerlegen.

Unter durch **Insertionsmutation** erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten. Proteinen ist der Anteil an Fremdprotein geringer als in chimären Proteinen.

**Inversionsmutagenese,** also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifizierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Ebenso ist unter Derivatisierung die kovalente Bindung an einen makromolekularen Träger zu verstehen.

Proteine können auch über die Reaktion mit einem Antiserum oder einem bestimmten Antikörper zu Gruppen **immunologisch verwandter** Proteine zusammengefaßt werden. Die Angehörigen einer Gruppe zeichnen sich dadurch aus, daß sie dieselbe, von einem Antikörper erkannte antigene Determinante aufweisen.

Im Sinne der vorliegenden Erfindung werden alle Enzyme, Proteine, Fragmente und Derivate, sofern sie nicht explizit als solche angesprochen zu werden brauchen, unter dem **Oberbegriff Proteine** zusammengefaßt.

Unter **Vektoren** werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten **Klonierungsvektoren,** und andererseits denen, die die Funktion erfüllien, das interessierende Gen in der Wirtszelle zu realisieren, das heißt, die Expression des betreffenden Proteins zu ermöglichen. Diese Vektoren werden als **Expressionsvektoren** bezeichnet.

Durch Vergleich mit bekannten Enzymen, die beispielsweise in allgemein zugänglichen Datenbanken hinterlegt sind, läßt sich aus der Aminosäure- oder Nukleotid-Sequenz die **enzymatische Aktivität** eines betrachteten Enzyms folgern. Diese kann durch andere Bereiche des Proteins, die nicht an der eigentlichen Reaktion beteiligt sind, qualitativ oder quantitativ modifiziert werden. Dies könnte beispielsweise die Enzymstabilität, die Aktivität, die Reaktionsbedingungen oder die Substratspezifität betreffen.

Solch ein Vergleich geschieht dadurch, daß ähnliche Abfolgen in den Nukleotid- oder Aminosäuresequenzen der betrachteten Proteine einander zugeordnet werden. Dies nennt man **Homologisierung.** Eine tabellarische Zuordnung der betreffenden Positionen wird als **Alignment** bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben.

Eine Zusammenstellung aller in den verglichenen Sequenzen übereinstimmenden Positionen wird als **Consensus-Sequenz** bezeichnet.

Solch ein Vergleich erlaubt auch eine Aussage über die Ähnlichkeit oder**Homologie** der verglichenen Sequenzen zueinander. Diese wird in **Prozent Identität,** das heißt dem Anteil der identischen Nukleotide oder Aminosäurereste an denselben Positionen widergegeben. Ein weiter gefaßter Homologiebegriff bezieht die konservierten Aminosäure-Austausche in diesen Wert mit ein. Es ist dann von **Prozent Ähnlichkeit** die Rede. Solche Aussagen können über ganze Proteine oder Gene oder nur über einzelne Bereiche getroffen werden.

Die Erstellung eines Alignments ist der erste Schritt zur Definition eines**Sequenzraums**.

Dieser hypothetische Raum umfaßt sämtliche durch Permutation in Einzelpositionen abzuleitenden Sequenzen, die sich unter Berücksichtigung aller in den betreffenden Einzelpositionen des Alignments auftretenden Variationen ergeben. Jedes hypothetisch mögliche Proteinmolekül bildet einen Punkt in diesem Sequenzraum. Beispielsweise begründen zwei Aminosäuresequenzen, die bei weitgehender Identität an lediglich zwei verschiedenen Stellen jeweils zwei verschiedene Aminosäuren aufweisen, somit einen Sequenzraum von vier verschiedenen Aminosäuresequenzen. Ein sehr großer Sequenzraum wird erhalten, wenn zu einzelnen Sequenzen eines Raums jeweils weitere homologe Sequenzen gefunden werden. Über solche, jeweils paarweise bestehenden hohen Homologien können auch sehr niedrig homologe Sequenzen als einem Sequenzraum zugehörig erkannt werden.

**Homologe Bereiche** von verschiedenen Proteinen sind solche mit gleichen Funktionen, die sich durch Übereinstimmungen in der primären Aminosäuresequenz erkennen lassen. Sie geht bis zu völligen Identitäten in kleinsten Bereichen, sogenannten Boxen, die nur wenige Aminosäuren umfassen und meist für die Gesamtaktivität essentielle Funktionen ausüben. Unter den **Funktionen der homologen Bereiche** sind kleinste Teilfunktionen der vom, gesamten Protein ausgeübten Funktion zu verstehen, wie beispielsweise die Ausbildung einzelner Wasserstoffbrückenbindungen zur Komplexierung eines Substrats oder Übergangskomplexes.

Unter dem Begriff eines **erfindungsgemäßen amylolytischen Proteins** ist somit nicht allein eines mit der reinen, die Hydrolyse von α-1,4-glycosidischen Bindungen durchführenden Funktion zu verstehen, die auf die wenigen Aminosäurereste eines vermutlichen katalytisch aktiven Zentrums zurückzuführen sind. Er umfaßt darüberhinaus alle die Hydrolyse einer α-1,4-glycosidischen Bindung unterstützende Funktionen. Solche können beispielsweise von einzelnen Peptiden als auch von einem oder mehreren einzelnen Teilen eines Proteins durch Einwirken auf die eigentlich katalytisch aktiven Bereiche erreicht werden. Der Begriff der amylolytischen Funktion umfaßt auch allein solche modifizierenden Funktionen. Denn einerseits muß nicht notwendigerweise genau bekannt sein, welche Aminosäurereste des erfindungsgemäßen Proteins tatsächlich die Hydrolyse katalysieren, und andererseits können nicht von vornherein bestimmte Einzelfunktionen definitiv von der Beteiligung an der Katalyse ausgenommen werden. Zu den Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische Aktivität. Dabei kann es sich beispielsweise auch um die Ausbildung eines Strukturelements handeln, das fern vom aktiven Zentrum liegt, oder um ein Signalpeptid, dessen Funktion die Ausschleusung des gebildeten Proteins aus der Zelle und/oder dessen korrekte Faltung betrifft und ohne das *in vivo* in der Regel kein funktionsfähiges Enzym gebildet wird. Allerdings muß sich insgesamt eine Hydrolyse von α-1,4-glycosidischen Bindungen von Stärke oder stärkeähnlichen Polymeren ergeben.

In diesem Sinne sind beispielsweise die Fragmente der α-Amylasen, die im Sequenzprotokoll unter SEQ ID NO. 34 bis 262 angegeben sind, als amylolytische Proteine anzusehen. Denn sie sind naturgemäß Bestandteile größerer Proteine, die insgesamt die α-1,4-glycosidischen Bindungen von Stärke oder stärkeähnlichen Polymeren zu hydrolysieren vermögen.

Im Rahmen der vorliegenden Anmeldung ist zwischen **Screening** (Hybridisierungs-Screening oder DNA-Screening) und **Aktivitätstest** zu unterscheiden. Im allgemeinen wird unter dem "Screening" von Transformanten eine Nachweisreaktion verstanden, die dazu geeignet ist, solche Klone zu erkennen, bei denen das gewünschte Transformationsereignis stattgefunden hat. Es richtet sich, wie beispielsweise bei der geläufigen Blau-weiß-Selektion meist auf den Nachweis einer biochemischen Aktivität, die die Transformanten erhalten haben oder die nach erfolgter Rekombination nicht mehr vorhanden ist. Diese Art von biochemischer Nachweisreaktion ist im Sinne der vorliegenden Anmeldung als Aktüvitätstest bezeichnet.

Screening bezeichnet das Durchsuchen einer Genbank mit bestimmten Nukleinsäuren und das damit mögliche Identifizieren von hinreichend ähnlichen NukleinsäureSequenzen. Dies geschieht beispielsweise über Southem- oder Northem-Blot Hybridisierungen, wie sie aus dem Stand der Technik hinlänglich bekannt sind. Dieser Begriff schließt beispielsweise aber auch erfindungsgemäße PCR-basierte Verfahren zur Identifizierung und/oder Gewinnung neuer Gene aus einer Sammlung von Organismen oder Nukleinsäuren ein; die dadurch gekennzeichnet sind, daß PCR-Primer mit einer variablen 3'-Region und einer 5'-Region mit hoher Homologie zu entsprechenden Bereichen aus bekannten Genen verwendet werden.

Unter der **Leistung eines Enzyms** wird dessen Wirksamkeit im jeweils betrachteten technischen Bereich verstanden. Diese basiert auf der eigentlichen enzymatischen Aktivität, hängt darüberhinaus aber von weiteren, für den jeweiligen Prozeß relevanten Fatoren ab. Dazu gehören beispielsweise Stabilität. Substratbindung, Wechselwirkung mit dem das Substrat tragenden Material oder Wechseiwirkungen mit anderen Inhaltsstoffen, insbesondere Synergien. So wird beispielsweise bei der Untersuchung, ob sich ein Enzym für den Einsatz in Wasch- oder Reinigungsmitteln eignet, dessen Beitrag zur Wasch- oder Reinigungsleistung eines mit weiteren Bestandteilen formulierten Mittels betrachtet. Für verschiedene technische Anwendungen kann ein Enzym über an sich bekannte molekularbiologische Techniken, insbesondere die oben genannten weiterentwickelt und optimiert werden.

Zur Lösung der gestellten Aufgabe wird erfindungsgemäß eine Vielzahl von α-Amylasen bereitgestellt, die alle als Vertreter eines bestimmten Sequenzraums anzusehen sind. Dieser ist über eine Teilsequenz dieser α-Amylasen definiert, nämlich einen Teil des für Amylasen bekannten Strukturelements (αβ)₈-Barrel. Alle amylolytischen Proteine, deren Aminosäuresequenz einen Teil enthält, der diesem spezfischen Sequenzraum angehört, sowie hinreichend ähnliche Proteine sind erfindungsgemäße Amylasen.

Dieser Sequenzraum ist dargesteil unter SEQ ID NO. 208 dargestellt.

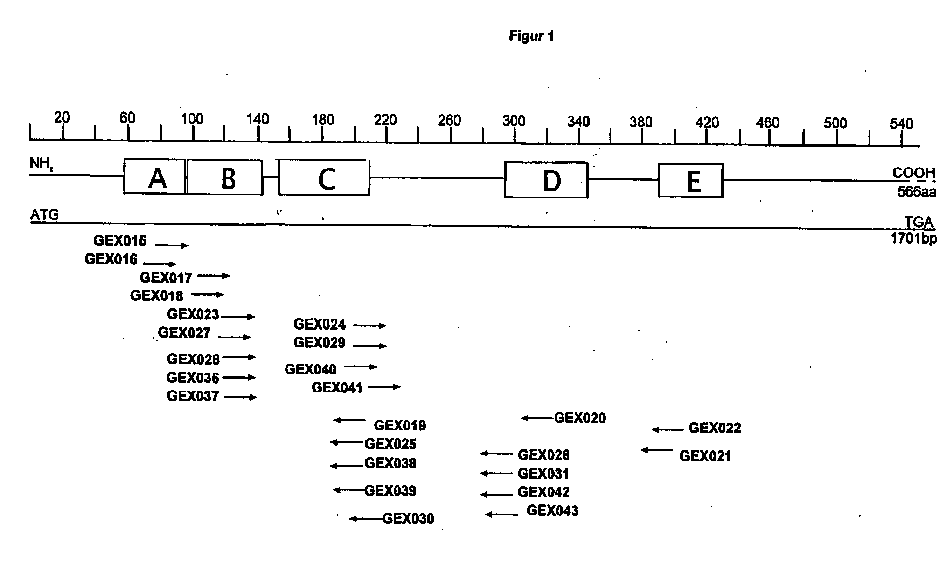

Diese Sequenz definiert eine völlig neue Gruppe von α-Amylasen. Sie bezeichnet einen Bereich, der zwischen den beiden konservierten Regionen C und D liegt, die in Figur 1 gezeigt sind. Dabei handelt es sich um ein Sekundärstrukturelement, nämlich um die Domänen β4 und β7 der (αβ)₈-Barrelstruktur, wie sie im Aufsatz von S. Janecek (1997) in Prog. Blophys. Mol. Biol. 67 (1), S. 67-97) definiert sind. Dieser Bereich wird für die Enzymfamilie der α-Amylasen als charakteristisch und als notwendiges Strukturelement angesehen. Denn es gewährleistet die optimale räumliche Anordnung der anderen funktionellen Teile des Moleküls, insbesondere des aktiven Zentrums.

Davon unabhängig ist für neu gefundene oder erzeugte Sequenzen jedoch auch eine andere Umgruppierung von Domänen denkbar. Deshalb stellen auch all Jene α-Amylasen Ausführungsformen der vorliegende Erfindung dar, die an beliebiger Stelle eine Teilsequenz enthalten, die sich durch SEQ ID NO. 208 beschreiben läßt

In den Beispielen werden die α-Amylasen der Stämme *Streptomyces sp.* B327*, B40OB, und B327 eingehender untersucht Die der Consensus-Sequenz (SEQ ID NO. 263) entsprechenden Teilbereiche dieser Amylasen werden im Sequenzprotokoll unter SEQ ID NO. 2, 4 und 208 gezeigt. Das höchste Maß an Homologie, welches zu einer dieser Amylasen durch Datenbanksuche ermittelt werden konnte, weist die α-Amylase aus *Streptomyces sp.* B327B auf. Es liegt bei 94% Identität mit der α-Amylase aus *Streptomyces griseus* (Datenbankeintrag X57568).

Aus diesem Grund werden erfindungsgemäß alle amyloytischen Proteine beansprucht, deren Aminosäuresequenz einen Teil enthält, der mit SEQ ID NO. 208 zu 95% und zunehmend bevorzugt zu 96%, zu 96,5%, zu 97%, zu 97.5%, zu 98%, zu 98,5%, zu 99%, zu 99,5% und besonders bevorzugt zu 100% identisch ist.

Weitere Lösungen der erfindungsgemäßen Aufgabe sind amylolytische, durch Insertionsmutation erhältliche oder amylolytische chimäre Proteine, welche wenigstens in einem eine amylolytische Aktivität verleihenden Teil aus einem der oben beschriebenen Proteine bestehen

Erfindungsgemäß sollen die durch die Fusion erhaltenen Proteine eine im weitesten Sinne amylolytische oder die Hydrolyse von α-1,4-glycosidischen Bindungen unterstützende Funktion aufweisen, herbeiführen oder modifizieren. Diese kann von einem Molekültell ausgeübt oder modifiziert werden, das sich von einem erfindungsgemäßen Protein herleitet und innerhalb des oben beanspruchten Ähnlichkeitsbereichs für diesesn Malekülteil liegt.

Bei solchen Molekülteiten kann es sich um Teile handeln, die über ihre Consensus-Sequenzen oder Boxen als homolog zu den Enzymen aus anderen Organismen erkannt werden können. Solche Bereiche verleihen dem Enzym zumeist seine charakteristischen enzymatischen Funktionen. Sie können auch über verschiedene Domänen, also globuläre Bereiche des Proteinmoleküls verteilt liegen. Zum Gegenstand der Erfindung gehören daher auch solche chimären Proteine, die aufgrund ihrer Konstruktion über ihre gesamte Aminosäure- und oder Nukleotidsequenz hinweg eine gegebenenfalls geringere Identität aufweisen als für den erfindungsgemäßen Ähnlichkeitsbereich oben definiert, die ihm aber in mindestens einer der durch Fusion eingebrachten Bereiche zugerechnet werden können und In diesem Teil dieselben Funktionen wie in einer Amylase ausüben, die in den oben definierten Homologiebereich fällt

Dasselbe gilt aufgrund ihrer prinzipiellen Gleichartigkeit auch für durch insertionsmutation zu erhaltende Varianten der oben genannten amylolytischen Proteine. Der Sinn von Insertionsmutagenese besteht besonders darin, einzelne Eigenschaften erfindungsgemäßer Proteine mit denen anderer Proteine zu kombinieren. Es handelt sich dann um erfindungsgeinäße, durch insertionsmutation zu erhaltende Proteine oder chimäre Proteine, wenn die Ober ihre Homologie auf die oben genannten Sequenzen zurückzuführenden Bereiche entsprechende Homologiewerte aufweisen und die emattene Variante aufgrund dieser Bereiche eine im weitesten Sinne amytolytische Funktion besitzt.

So ist es beispielsweise in Anwendung der Lehre von WO 99/57250 möglich, solch ein Enzym zur Erhöhung der Wechselwirkung mit dem Substrat mit einer Cellulose-Bindungsdomäne zu koppeln. In analoger Weise können beispielsweise auch andere wasch- oder reinigungsaktive Enzyme mit einer erfindungsgemäßen Amylase fusioniert werden. Es ist dabei prinzipiell unerheblich, ob die Fusion N- oder C-terminal oder über Insertion erfolgt

Die erfindungsgemäßen Proteine können vor allem während der Lagerung durch Stabilisatoren beispielsweise vor Denaturierung, Zerfall oder Inaktivierung, etwa durch physikalische Einflüsse, Oxidation oder Proteolyse geschützt werden. Vielfach werden auch sich ergänzende oder gegenseitig steigemde Kombinationen von Stabilisatoren verwendet.

Eine Gruppe von Stabilisatoren sind reversible Proteaseinhibitoren, wie beispielsweise Benzamidin-Hydrochlorid und Leupeptin, Borax, Borsäuren, Boronsäuren, deren Salze oder Ester, Peptidaldehyde oder rein peptidische inhibitoren wie Ovomucoid öder spezifische Subtilisin-Inhibitoren. Weitere geläufige Enzymstabilisatoren sind Aminoalkohole wie Mono-, Di-, Triethanol- und -propanolamin, aliphatische Carbonsäuren bis zu C₁₂, Dicarbonsäuren, niedere aliphatische Alkohole, v.a. aber Polyole, wie beispielsweise Glycerin, Ethylenglykol, Propylengiykol oder Sorbit Ebenso werden Calciumsalze verwendet, wie beispielsweise Calciumacetat oder Calcium-Formiat, Magnesiumsalze, verschiedenste Polymere wie beispielsweise Lignin, Cellulose-Ether, Polyamide oder wässerlösliche Vinyl-Copolymere, um die Enzym-Präparation v.a. gegenüber physikalischen Einflüssen oder pH-Wert-Schwankungen zu stabilisieren. Reduktionsmittel und Antioxidantien, wie beispielsweise Natrium-Sulfit oder reduzierende Zucker, erhöhen die Stabilität der Proteine gegenüber oxidativem Zerfall.

Umgekehrt können erfindungsgemäße proteine dazu verwendet werden, um verwandte Amylasegene oder Amylasen zu gewinnen, zu identifizieren oder zu untersuchen. Dies ist beispielsweise über alle molekularbiologischen Methoden möglich, bei denen Antikörper gegen entsprechende Bereiche verwendet werden, etwa bei einem Screening einer Expressionsgenbank.

Die SEQ ID NO. 34 bis SEQ ID NO. 262 beschreiben die Aminosäuresequenzen, die, wie in Beispiel 1 dargestellt ist, von bestimmten PCR-Produkten abgeleitet sind. Sie sind dadurch erhalten worden, daß die isolierten Nukleinsäuren aus einer Sammlung von mehreren hundert Actinomycetales-isolaten als Matrizen in Polymerase-Kettenreaktionen mit den Primerpaaren GEX024 (SEQ ID NO. 9) / GEX026 (SEQ ID NO. 10) und GEX029 (SEQ ID NO. 11) / GEX031 (SEQ ID NO. 12) zur Reaktion gebracht worden sind. Die Positionen 8 bis 93 gemäß der Consensus-Sequenz von SEQ ID NO. 263 liegen innerhalb der Primer GEX024 und GEX026, so daß sie als besonderes charakteristisch für die gefundenen Sequenzen angesehen werden können.

Aus folgenden Gründen müssen deshalb auch die zugehörigen Nukleinsäuren als Lösungen der erfindungsgemäßen Aufgabe und damit als eigene Erfindungsgegenstände angesehen werden: Zum einen werden Proteine dann molekularbiologisch faßbar, wenn die zugehörigen Gene zur Verfügung stehen. Dies gilt für die Identifizierung und die Charakterisierung, über die Mutagenese, bis hin zu biotechnologischen Produktion. Es werden auch die aufgrund der zwischen den verschiedenen Organismen variierenden Codon-Usage abgeleiteten Nukleotidsequenzen, als auch Ribonukleinsäuren miteingeschlossen, denn auch von ihnen können funktionsfähige Enzyme abgeleitet werden. Entsprechende Nukleinsäuren können auch zur Gewinnung, Identifizierung oder Untersuchung von Amylasegenen verwendet werden. So ist es beispielsweise möglich, entsprechende Sonden zum Genbank-Screen zu entwerfen.

Zum anderen beruht die in den Beispielen detailliert ausgeführte Methode zum Auffinden der betreffenden α-Amylasen auf der PCR und damit auf den entsprechenden Nukleinsäuren.

Somit stellen alle Nukleinsäuren, die für Proteine codieren, die einen Teil aufweisen, der innerhalb des oben definierten Ähnlichkeitsbereichs liegen, Ausführungsfonnen dieses Erfindungsgegenstands dar.

Ausführungsformen dieses Erfindungsgegenstands sind Nukleinsäuren, die für amylolytische Proteine codieren, deren Aminosäuresequenz einen Teil enthält, der mit der in SEQ ID NO. 208 angegebenen Aminosäuresequenz zu 95% und zunehmend bevorzugt zu 96%, zu 96,5%, zu 97%, zu 97,5%, zu 98%, zu 98,5%, zu 99%, zu 99,5% und besonders bevorzugt zu 100% identisch ist

Denn die über diese Teilsequenzen erhaltenen vollständigen Proteine sind in Beispiel 6 auf ihre biochemischen Eigenschaften hin untersucht worden. Sie erscheinen damit als aussichtsreiche Kandidaten für den Einsatz in technischen Prozessen oder zur weiteren Optimierung in Hinblick auf technische Prozesse. Ähnliche Eigenschaften sind für die Proteine zu erwarten, die ausgehend von den Nukleinsäuren dieses Erfindungsgegenstands erhalten werden können.

Entsprechend bevorzugten Ausführungsformen dieses Erfindungsgegenstands stellen die Nukleinsäuren dar, die für eines der oben bezeichneten amylolytischen Proteine.

Einen eigenen Erfindungsgegenstand stellt es dar, wenn die mit der vorliegenden Anmeldung zur Verfügung gestellten Proteine oder Nukleinsäuren zur Auffindung weiterer α-Amylasen verwendet werden können.

Somit wird die Verwendung eines Proteins nach dem ersten Erfindungsgegenstand zur Identifizierung eines amylolytischen Proteins beansprucht. Auch Nukleinsäuren nach dem zweiten Erfindungsgegenstand können zur Identifizierung und/oder Gewinnung einer neuen Amylase verwendet werden,

Bevorzugte Ausführungsformen stellen derartige Verwendungen zur gezielten Fusion, unter Ausnutzung hochhornologer Bereiche der Aminosäuresequenzen, gemeinsamer Restriktionsschnittstellen der Nukleinsäuren oder über PCR-basierte Fusion dar.

Eine weitere Möglichkeit besteht In der Verwendung in einem statistischen Rekombinationsverfahren, insbesondere unter Ausnutzung hochhomologer Bereiche der Aminosäuresequenzen, gemeinsamer Restriktionsschnittstellen der Nukleinsäuren oder über PCR-basierte Fusion.

Die hochhomologen Bereiche der Aminosäuresequenzen, die zumeist konservierte, funktionstragende Bereiche darsteilen, können dabei als Anker für PCR-Primer, gegebenenfalls von degenerierten Primem dienen. Gemeinsame Restrihonsschnittstellen der Nukleinsäuren zweier verschiedener Gene ermöglichen ein unmittelbare Fusion auf der DNA-Ebene. Für die Kombination der verschiedenen Genbereich stehen alle im Stand der Technik etablierten Methoden sowie die erfindungsgemäße Methode mit ihren verschiedenen Ausführungsformen zur Verfügung.

Einem eigenen Erfindungsgegenstand werden Vektoren zugerechnet, die einen der Nukleinsäurebereiche des zweiten Erfindungsgegenstands enthält.

Solche Vektoren bilden die bevorzugten Ausgangspunkte für molekularbiologische und biochemische Untersuchungen des betreffenden Gens oder zugehörigen Proteins, für erfindungsgemäße Weiterentwicklungen und für die Amplifizierung und Produktion erfindungsgemäßer Proteine.

Geeignete Vektoren können sich von bakteriellen Plasmiden, von Viren oder von Bacteriophagen ableiten, aber auch Elemente verschiedenster Herkunft enthalten. Mit den weiteren jeweils vorhandenen genetischen Elementen vermögen Vektoren, sich in den betreffenden Wirtszellen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.

Bevorzugte Ausführungsformen dieses Erfindungsgegenstands sind Klonierungsvektoren.

Diese eignen sich neben der Lagerung, der biologischen Amplifizierung oder der Selektion des interessierenden Gens für die Charakterisierung des betreffenden Gens, etwa über das Erstellen einer Restriktionskarte oder die Sequenzierung. Klonierungsvektoren sind auch deshalb bevorzugte Ausführungsformen der vorliegenden Erfindung, weil sie eine transportierbare und lagerfähige Form der beanspruchten DNA darstellen. Sie sind auch bevorzugte Ausgangspunkte für molekularbiologische Techniken, die nicht an Zellen gebunden sind, wie beispielsweise die Polymerasekettenreaktion.

Bevorzugte Ausführungsformen dieses Erfindungsgegenstands sind Expressionsvektoren.

Sie sind aufgrund der entsprechenden genetischen Elemente in der Lage, in den für die Produktion von Proteinen optimierten Wirtsorganismen zu replizieren und dort das enthaltene Gen zur Expression zu bringen. Bevorzugte Ausführungsformen sind Expressionsvektoren, die selbst alle zur Expression notwendigen genetischen Elemente tragen. Promotoren regulieren die Transkription eines Gens. Beispiele hierfür sind natürliche, ursprünglich vor den betreffenden Genen lokalisierte Promotoren. Da diese für die meisten der erfindungsgemäßen Gene jedoch nicht als bekannt vorausgesetzt werden können, sind solche Ausführungsformen bevorzugt, bei denen nach gentechnischer Fusion bekannte, andere Promotoren zur Regulation des Transgens bereitgestellt werden. Dabei kann es sich um einen Promotor der Wirtszelle, um einen modifizierten oder auch um einen völlig anderen Promotor aus einem anderen Organismus handeln; Bevorzugte Ausführungsformen sind solche Expressionsvektoren, die über Änderungen der Kulturbedingungen oder Zugabe von bestimmten Verbindungen, wie beispielsweise die Zelldichte oder spezielle Faktoren, regulierbar sind.

Expressionsvektoren ermöglichen in Abhängigkeit von den jeweiligen genetischen Elementen eine heterologe oder eine homologe Proteinexpression. Ausführungsformen der vorliegenden Erfindung können auch zellfreie Expressionssysteme sein, bei denen die Proteinbiosynthese *in vitro* nachvollzogen wird. Derartige Expressionssysteme sind im Stand der Technik ebenfalls etabliert und beruhen auf den in Klonierungs- oder Expressionsvektoren bereitgestellten Genen.

Diesem Erfindungsgegenstand werden Wirtszellen zugerechnet, die eines der erfindungsgemäßen Proteine oder Derivate exprimieren oder zu dessen Expression angeregt werden können. Dies geschieht vorzugsweise unter Einsatz eines entsprechenden Expressionsvektors.

Die In-vivo-Synthese eines erfindungsgemäßen amylolytischen Enzyms durch lebende Zellen, erfordert den Transfer des zugehörigen Gens in eine Wirtszelle, deren sogenannte Transformation. Als Wirtszellen eignen sich prinzipiell alle Organismen außer Mensch, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor und dessen stabile Etablierung angeht. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden.

Bevorzugte Ausführungsformen stellen solche Wirtszellen dar, die aufgrund genetischer Regulationselemente, die beispielsweise auf dem Expressionsvektor zur Verfügung gestellt werden, aber auch von vornherein in diesen Zellen vorhanden sein können, in ihrer Proteinbildungsrate regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine sehr wirtschaftliche Produktion der interessierenden Proteine.

Eine Variante dieses Versuchsprinzips stellen Expressionssysteme dar, bei denen zusätzliche Gene, beispielsweise solche, die auf anderen Vektoren zur Verfügung gestellt werden, die Produktion erfindungsgemäßer Proteine beeinflussen. Hierbei kann es sich um modifizierende Genprodukte handeln oder um solche, die mit dem erfindungsgemäßen Protein gemeinsam aufgereinigt werden sollen, etwa um dessen amylolytische Funktion zu beeinflussen. Dabei kann es sich beispielsweise um andere Proteine oder Enzyme, um Inhibitoren oder um solche Elemente handeln, die die Wechselwirkung mit verschiedenen Substraten beinflussen.

In einer bevorzugten Ausführungsform ist die Wirtszelle dadurch gekennzeichnet, daß sie ein Bakterium ist, insbesondere eines, das das gebildete Protein oder Derivat ins umgebende Medium sekretiert.

Bevorzugte Wirtszellen sind prokaryontische oder bakterielle Zellen. Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung erfindungsgemäßer Proteine etabliert werden. Eine bevorzugte Ausführungsform stellen Wirtszellen dar, die dadurch gekennzeichnet sind, daß sie gram-positive Bakterien sind.

Grampositive Bakterien, wie beispielsweise Bacilli oder Actinomyceten oder andere Vertreter der Actinomycetales, besitzen keine äußere Membran, so daß sekretierte Proteine sogleich in das die Zellen umgebende Nährmedium abgegeben werden, aus welchem sich nach einer anderen bevorzugten Ausführungsform die exprimierten erfindungsgemäßen Proteine direkt aufreinigen lassen.

Eine bevorzugte Ausführungsform stellen Wirtszelle dar, die dadurch gekennzeichnet sind, daß sie der Gattung Bacillus, vorzugsweise der Species *Bacillus licheniformis, Bacillus amyloliquefaciens, Bacillus subtilis* oder *Bacillus alcalophilus* angehören.

Diese stellen etablierte gram-positive Expressionssysteme dar.

Eine bevorzugte Ausführungsform stellen Wirtszellen dar, die dadurch gekennzeichnet sind, daß sie gram-negative Bakterien sind.

Denn an gram-negativen Bakterien, wie beispielsweise *E*. *coli* oder *Klebsiella,* hat man in der biotechnologischen Produktion bislang die meisten Erfahrungen machen können. Bei ihnen werden zudem eine Vielzahl von Proteinen in den periplasmatischen Raum sekretiert, also in das Kompartiment zwischen den beiden die Zellen einschließenden Membranen. Dies kann für spezielle Anwendungen ausgenutzt werden. Demgegenüber kann aber auch die gezielte Freisetzung der von gram-negativen Bakterien produzierten Enzyme in den extrazellulären Raum bevorzugt sein. Ein Verfahren hierfür wird beispielsweise in der internationalen Patentanmeldung PCT/EP01/04227 mit dem Titel "Verfahren zur Herstellung rekombinanter Proteine durch gram-negative Bakterien" beschrieben.

Bevorzugt sind solche Wirtszellen, die dadurch gekennzeichnet sind, daß sie der Gattung Escherichia, bevorzugt der Spezies *Escherichia coli,* besonders bevorzugt einem der Stämme *E*. *coli* JM 109, *E. coli* DH 100B oder *E*. *coli* DH 12S angehören.

Hierbei handelt es sich um etablierte, allgemein zugängliche Labor- und Produktionsstämme. Der Stamm *E*. *coli* DH 12S wurde in Beispiel 6 erfolgreich für die heterologe Expression der erhaltenen Actinomycetales-Gene verwendet; es konnten dadurch Proteine mit nachweisbarer α-Amylase-Aktivität erhalten werden.

Eine weitere Ausführungsform stellen Wirtszellen dar, die dadurch gekennzeichnet sind, daß sie eukaryontische Zellen sind, insbesondere solche, die das gebildete Protein posttranslational modifizieren.

Beispiele dafür sind Pilze oder Hefen wie *Saccharomyces* oder *Kluyveromyces.* Dies kann beispielsweise dann besonders vorteilhaft sein, wenn die Proteine im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccaride.

Eine weitere Ausführungsform dieses Erfindungsgegenstands stellen Verfahren zur Herstellung eines erfindungsgemäßen Proteins dar. Dafür werden erfindungsgemäße Nukleinsäuren eingesetzt, optional unter Verwendung eines entsprechenden Vektors und/oder unter Verwendung einer entsprechenden Wirtszeille oder unter Verwendung einer Zelle, die dieses natürlicherweise bildet.

Alle bereits oben ausgeführten Elemente können zu Verfahren kombiniert werden, um erfindungsgemäße Proteine herzustellen. Es ist dabei für Jedes erfindungsgemäße Protein eine Vielzahl von Kombinadonsmöglichkeiten an Verfahrensschritten denkbar. Sie alle verwirklichen die der vorliegenden Erfindung zugrundeliegende Idee, nämlich Vertreter eines über die amylolytische Funktion und gleichzeitig die hohe Homologie zu den in den Sequenzprotokollen angegebenen Sequenzen definierten Proteintyps mithilfe der zugehörigen genetischen Information quantitativ herzustellen. Das optimale Verfahren muß für jeden konkreten Einzelfall experimentell ermittelt werden.

Prinzipiell wird dabei folgendermaßen vorgegangen: Erfindungsgemäße Nukleinsäuren werden in Form der DNA in einen geeigneten Expressionsvektor ligiert. Dieser wird in die Wirtszelie transformiert, beispielsweise in Zellen eines leicht zu kultivierenden Bakterienstammes, der die Proteine, deren Gene unter der Kontrolle entsprechender genetischer Elemente stehen, in das umgebende Nährmedium ausschleust oder Im Zellinneren akkumuliert; regulierende Elemente dafür können beispielsweise vom Expressionsvektor zur Verfügung gestellt werden. Aus dem umgebenden Medium, beziehungsweise unter Zellaufschluß aus den Wirtszellen selbst, kann das erfindungsgemäße Protein Ober mehrere Aufreinigungsschritte, wie beispielsweise Fällungen oder Chromatographien, aufgreinigt werden. Ein Fachmann ist in der Lage, ein System, welches im Labormaßstab experimentell optimiert worden ist, auf einen großtechnischen Produktionsmaßstab zu übertragen.

Die technischen Einsatzmöglichkeiten für erfindungsgemäße Amylasen stellen einen eigenen Efindungsgegenstand dar. Denn die zugrundeliegende Aufgabe hatte darin bestanden, geeignete Amylasen für diverse technische Prozesse zur Verfügung zu stellen. Die wichtigsten davon werden Im folgenden ausgeführt.

Zahlreiche In der Technik etablierte Anwendungsmogilchkeften für amylolytische Enzyme werden in Handbüchern, wie beispielsweise dem Buch industrial enyzmes and their applicationsa von H. Uhlig. Wliey-Verlag, New York, 1998, ausgeführt Folgende Zusammenstellung ist nicht als abschließende Aufzähtung zu verstehen, sondern stellt eine Auswahl der technischen Verwendungsmöglichkeiten dar. Sollte sich herausstellen, daß einzelne, innerhalb des Ähnlichkeitsbereichs liegende Proteine aufgrund ihrer enyzmatischen, das heißt amylolytischen Eigenschaften für zusätzliche hier nicht ausdrücklich beanspruchte Anwendungsmöglichkeiten geeignet sind, so werden diese hiermit in den Schutzbereich der vorliegenden Erfindung miteingeschlossen.

Eine Ausführungsform dieses Erfindungsgegenstands sind Wasch- und Reinigungsmittel, die dadurch gekennzeichnet sind, daß sie ein erfindungsgemäßes Protein enthalten.

Ein wichtiges Einsatzgebiet für amylolytische Enzyme ist das als aktive Komponenten in Wasch- oder Reinigungsmitteln zur Reinigung von Textilien oder von festen Oberflächen, wie beispielsweise Geschirr, Fußböden oder Arbeitsgeräten. In diesen Anwendungen dient die amylotytische Aktivität dazu, kohlenhydrathaltige Verunreinigungen und insbesondere solche auf Stärkebasis hydrolytisch aufzulösen oder vom Untergrund abzulösen. Dabei können sie allein, in geeigneten Medien oder auch in Wasch- oder Reinigungsmitteln zur Anwendung gebracht werden. Die hierfür zu wählenden Bedingungen, wie beispielsweise Temperatur, pH-Wert, lonenstätke, Redox-Verhältnisse oder mechanische Einflüsse sollten für das jeweilige Reinigungsproblem optimiert werden, also in Bezug auf die Anschmutzung und auf das Trägermaterial. So liegen übliche Temperaturen für Wasch- und Reinigungsmittel in Bereichen von 10°C bei manuellen Mitteln über 40°C und 60°C bis hin zu 95° bei maschinellen Mitteln oder bei technischen Anwendungen. Vorzugsweise werden auch die Inhaltsstoffe der betreffenden Mittel aufeinander abgestimmt. Da bei modernen Wasch- und Spülmaschinen die Temperatur meist stufenlos einstellbar ist, sind auch alle Zwischenstufen der Temperatur eingeschlossen. Die übrigen Bedigungen können über die übrigen Bestandteile der Mittel ebenfalls sehr variabel auf den jeweiligen Reinigungszweck ausgelegt werden.

Bevorzugte erfindungsgemäße Mittel zeichnen sich dadurch aus, daß unter irgendwelchen der auf diese Weise definierbaren Bedingungen die Wasch- oder Reinigungsleistung dieses Mittels durch Zugabe eines erfindungsgemäßen amylolytischen Enzyms gegenüber der Rezeptur ohne dieses Enzym verbessert wird. Insofern werden bevorzugt solche amylolydschen Proteine in erfindungsgemäße Mittel eingearbeitet, die die Wasch- und/oder Reinigungsleistung eines Wasch- oder Reinigungsmittels zu verbessern vermögen.

Weiterhin bevorzugte Mittel zeichnen sich dadurch aus, daß die amylolyüschen Enzyme und die übrigen Komponenten synergistisch die Beseitigung der Verunreinigungen bewirken. Das geschieht beispielsweise so, daß die Hydrolyseprodukte der amylolytischen Proteine durch andere Bestandteile der Mittel wie etwa Tenside solubibiert werden. Ein erfindungsgemäßes Protein kann sowohl in Mitteln für Großverbraucher oder technische Anwender als auch in Produkten für den Privatverbraucher Anwendung finden, wobei alle im Stand der Technik etablierten und/oder zweckmäßigen Darreichungsformen auch Ausführungsformen der vorliegenden Erfindung darstellen.

Die Amylasen werden In erfindungsgemäßen Mittel beispielsweise mit einzelnen oder mehreren der folgenden inhaltsstoffe kombiniert anionische, kationische und/oder nichtionische Tenside, Builder, Bleichmittel, Bleichaktivatoren, Bleichkatalysatoren, Enzyme wie beispielsweise Proteasen, andere Amylasen, Lipasen, Cellufasen, Hemicellulasen oder Oxidasen, Stabilisatoren, insbesondere Enzymstabilisatoren, Lösungsmittel, Verdicker, Abrasivstoffe, Farbstoffe. Duftstoffe, Vergrauungsinhibitoren, Farbtransferinhibitoren, Schauminhibitoren, Korrosionsinhibitoren, insbesondere Silberschutzmittel, optische Aufheller, antimikrobielle Wirkstoffe, UV-Schutzmittel und andere Komponenten, die aus dem Stand der Technik bekannt sind.

Bevorzugte Mittel sind dadurch gekennzeichnet, daß sie 0,000001 Gewichts-Prozent bis 5 Gew.-%, und zunehmend bevorzugt 0,00001 bis 4 Gew.-%, 0,0001 bis 3 Gew.-%, 0,001 bis 2 Gew.-% oder 0,01 bis 1 Gew.-% des amylolytischen Proteins enthalten.

Bevorzugte Mittel sind dadurch gekennzeichnet, daß sie aus mehr als einer Phase bestehen. Hierunter können feste Mittel sein, insbesondere solche, bei denen mindestens zwei verschiedene feste Komponenten, insbesondere Pulver, Granulate oder Extrudate, in insgesamt loser Form miteinander vermischt vorliegen oder mindestens zwei feste Phasen miteinander verbunden vorilegen, insbesondere nach einem gemeinsamen Kompaktierungsschritt. Zusätzlich kann wenigstens eine der Phasen ein Amylase-sensitives Material, insbesondere Stärke enthalten oder von diesem zumindest teilweise umgeben oder beschichtet sein.

Ebenfalls bevorzugte Mittel sind dadurch gekennzeichnet, daß sie insgesamt flüssig, gelförmig oder pastös sind. Vorzugsweise liegen darin das enthaltene Protein und/oder wenigstens eines der enthaltenen Enzyme und/oder wenigstens eine der sonstigen enthaltenen Komponenten einzeln oder zusammen mit anderen Bestandteilen verkapselt vor, bevorzugt in Mikrokapseln, besonders bevorzugt in solchen aus einem Amylasesensitiven Material.

In einer bevorzugten Ausführungsform wird die amylolytische Aktivität durch einen der sonstigen Bestandteile des Mittels modifiziert, insbesondere stabilisiert und/oder in ihrem Beitrag zur Wasch-, beziehungsweise Reinigungsleistung des Mittels gesteigert.

Diesen Ausführungen entsprechend stellen auch alle Wasch- oder Reinigungsverfahren, die in wenigstens einem Teilschritt auf einer erfindungsgemäßen α-Amylase beruhen oder vorzugsweise ein erfindungsgemäßes Mittel zum Einsatz kommt, Ausführungsformen der vorliegenden Erfindung dar.

Ebenso stellt jede Verwendung einer erfindungsgemäßen α-Amylase oder vorzugsweise eines erfindungsgemäßen Mittels zum Waschen oder Reinigen von Textilien oder harten Oberflächen eine Ausführungsform der vorliegenden Erfindung dar.

Eine weitere Ausführungsform stellt die Verwendung eines erfindungsgemäßen Proteins oder Derivats zur Behandlung von Rohmaterialien oder Zwischenprodukten in der Textilherstellung dar, insbesondere zum Entschlichten von Baumwolle.

Rohmaterialien und Zwischenprodukte der Textilherstellung, beispielsweise für solche auf Baumwollbasis, werden im Rahmen ihrer Herstellung und Weiterverarbeitung mit Stärke ausgerüstet, um eine bessere Verarbeitung zu ermöglichen. Dieses sowohl auf Garne, auf Zwischenprodukte, als auch auf Textilien angewendete Verfahren nennt man Schlichten (sizing). Zur Entfernung der Schlichte, also der stärkehaltigen Schutzschicht (Entschlichten, desizing) sind erfindungsgemäße amylolytische Proteine geeignet.

Eine weitere Ausführungsform stellen Verfahren zur Stärkeverflüssigung, Insbesondere zur Ethanoiproduktion dar, die dadurch gekennzeichnet sind, daß darin ein erfindungsgemäßes Protein eingesetzt wird

Zur Stärkeverflüssigung wird In Wasser oder Puffer gequollene Stärke mit amylolytischen Enzymen inkubiert, wodurch das Polysaccharid in kleinere Bestandteile, zutetzt überwiegend in Maltose gespalten wird. Erfindungsgemäße Enzyme werden bevorzugt für ein solches Verfahren oder einen Teilschritt davon eingesetzt, wenn sie sich aufgrund Ihrer biochemischen Eigenschaften gut in ein entsprechendes Produktionsverfahren einpassen lassen. Dies kann beispielsweise dann der Fall sein, wenn sie in einem Schritt zusätzlich zu anderen Enzymen eingebracht werden sollen, die die gleichen Reaktionsbedingungen benötigen. Besonders bevorzugt sind erfindungsgemäße amylolytische Proteine, wenn gerade die von ihnen selbst gebildeten Produkte im Zentrum des interesses stehen. Die Stärkeverflüssigung kann auch einen Schritt in einem mehrstufigen Verfahren zur Gewinnung von Ethanol oder davon abgeleiteten Folgeprodukten, beispielsweise Essigsäure darstellen.

Eine weitere Ausführungsform stellt die Verwendung eines erfindungsgemäßen Proteins zur Herstellung von linearen und/oder kurzkettigen Oligosacchariden dar.

Aufgrund ihrer enzymatischen Aktivität bilden erfindungsgemäße amylolytische Proteine aus stärkeähnlichen Polymeren nach kürzerer Inkubationszeit überwiegend höherrnolekulare Oligosacchacide, wie beispielsweise Maltohexaose, Maltoheptaose oder Maltooctaose. Nach längerer Inkubationszeit steigt unter den Reaktionsprodukten der Anteil niedrigerer Oligosaccharfde, wie beispielsweise Maltose oder Maltotriose an. Bei besonderem Interesse an bestimmten Reaktionsprodukten können entsprechende Varianten erfindungsgemäßer Proteine verwendet und/oder die Reaktionsbedingungen entsprechend gestaltet werden. Dies ist insbesondere dann attraktiv, wenn es nicht auf reine Verbindungen, sondern auf Gemische ähnlicher Verbindungen ankommt, wie beispielsweise bei der Bildung von Lösungen, Suspensionen oder Gelen mit lediglich bestimmten physikalischen Eigenschaften.

Eine weitere Ausführungsform stellt die Verwendung eines erfindungsgemäßen Proteins Hydrolyse von Cyclodextrinen dar.

Cyclodextrine sind α-1,4-glycosidisch verknüpfte, cyclische Oligosaccharide, von denen die aus 6, 7 oder 8 Glucose-Monomeren, die α-, β-, beziehungsweise γ-Cyclodextrine (oder Cyclohexa-, -hepta-, beziehungsweise -oda-Amylosen), wirtschaftlich die größte Bedeutung besitzen. Sie können mit hydrophoben Gastmolekülen, wie beispielsweise Duftstoffen, Geschmacksstoffen oder pharmazeutischen Wirkstoffen, Einschlußverbindungen bilden, aus weichen die Gastmoleküle bei Bedarf wieder freigesetzt werden können. Solche Einschlußverbindungen sind je nach Einsatzgebiet der inhaltsstoffe beispielsweise für die Herstellung von Nahrungsmitteln, die Pharmazie oder die Kosmetik, beispielsweise in entsprechenden Produkten für den Endverbraucher von Bedeutung. Die Freisetzung von inhaltsstoffen aus Cyclodextrinen stellt somit eine Verwendungsmöglichkeit für erfindungsgemäße Proteine dar.

Eine weitere Ausführungsform stellt die Verwendung eines erfindungsgemäßen Proteins zur Freisetzung von niedermoiekutaren Verbindungen aus Polysaccharidträgem oder Cyclodextrinen dar.

Aufgrund ihrer enzymatischen Aktivität können erfindungsgemäße amylblytische Proteine niedermolekulare Verbindungen auch aus anderen α-1,4-glycosidisch verknüpften Polysacchariden freisetzen. Dieses kann sich wie bei den Cyclodextrinen auf molekularer Ebene abspielen als auch an größeren Systemen, wie beispielsweise Inhaltsstoffen, die In Form von Mikrokapsein verkapselt sind. Beispielsweise Stärke ist ein im Stand der Technik etabliertes Material, um Verbindungen wie beispielsweise Enzyme, die in definierten Mengen in Reaktionsansätze eingebracht werden sollen, während der Lagerung einzukapsein. Der kontrollierte Freisetzungsprozeß aus derartigen Kapseln kann von erfindungsgemäßen amylolytischen Enzymen unterstützt werden.

Eine weitere Ausführungsform stellt die Verwendung eines erfindungsgemäßen Proteins zur Herstellung von Lebensmitteln und/oder Lebensmittelbestandteilen dar.

Ebenso stellt die Verwendung eines erfindungsgemäßen Proteins zur Herstellung von Tierfutter und/oder Tierfutterbestandteilen eine Ausführungsform dieses Erfindungsgegenstands dar.

Wo immer Stärke oder von Stärke abgeleitete Kohlenhydrate als Lebensmittel- oder Tiemahrungsbestandteil eine Rolle spielen, kann eine amylolytische Akdvität zur Herstellung dieser Artikel zum Einsatz kommen. Sie erhöht den Anteil von Mono-, oder Oligomeren gegenüber dem polymeren Zucker, was beispielsweise dem Geschmack, der Verdaubarkeit oder der Konsistenz des Lebensmittels zugute kommen kann. Dies kann zur Herstellung bestimmte Tierfutter erforderlich sein, beispielsweise aber auch bei der Herstellung von Fruchtsäften, Wein oder anderer Lebensmittel, wenn der Anteil polymerer Zucker verringert und der von süßen und/oder leichter löslichen Zuckern erhöht werden soll. Die weiter oben ausgeführte Verwendungsmöglichkeit zur Stärkeverflüssigung und/oder Ethanolproduktion kann als großtechntsche Variante dieses Prinzips angesehen werden.

Amylasen wirken darüberhinaus auch dem unter Altbacken-Werden bekannten Geschmacksverlust von Backwaren (Anti staling-Effekt) entgegen. Dafür werden sie geeigneterweise dem Teig vor dem Backen zugesetzt. Bevorzugte Ausführungsformen der vorliegenden Erfindung sind also solche, bei denen erfindungsgemäße Proteine zur Herstellung von Backwaren verwendet werden.

Eine weitere Ausführungsform stellt die Verwendung eines erfindungsgemäßen Proteins Auflösung stärkehattiger Klebeverbindungen dar.

Ebenso stellen temporäre Klebeverfahren, die dadurch gekennzeichnet sind, daß darin ein efindungsgemäßes Protein eingesetzt wird, eine Ausführungsform dieses Erfindungsgegenstands dar.

Neben anderen Naturstoffen wird auch Stärke bereits seit Jahrhunderten als Bindemittel in der Papierherstellung und dem Verkleben unterschiedlicher Papiere und Pappen verwendet. Dies betrifft beispeilsweise Graphiken und Bücher. Im Laufe langer Zeiträume können solche Papiere unter ungünstigen Einflüssen, wie beispielsweise Feuchtigkeit, Wellen aufwerfen oder brechen, was bis zu deren völliger Zerstörung führen kann. Bei der Restaurierung solcher Papiere und Pappen kann das Auflösen der Klebeschichten erforderlich sein und durch Verwendung eines efindungsgemäßen amylolytischen Proteins erheblich erleichtert werden.

Pflanzliche Polymere wie Stärke oder Cellulose und deren wasserlösliche Derivate werden unter anderem als Klebstoffe oder Kleister verwendet. Dafür müssen sie in Wasser zunächst quellen und nach Aufbringen auf dem Klebegut trocknen, wodurch dieses am Untergrund fixiert wird. Das erfindungsgemäße Enzym kann solch einer wäßrigen Suspension zugesetzt werden, um die Klebeeigenschaften des entstehenden Kleisters zu beeinflussen. Es kann aber auch stattdessen oder zusätzlich zu dieser Funktion dem Kleister zugesetzt werden, um nach dem Antrocknen für lange Zeit, beispielsweise einige Jahre, inaktiv auf dem Klebegut zu verharren. Gezieltes Ändern der Umgebungsbedingungen, beispielsweise durch Anfeuchten, kann dann dazu verwendet werden, um das Enzym zu einem späteren Zeitpunkt zu aktivieren und damit ein Auflösen des Kleisters herbeizuführen. Auf diese Weise ist das Klebegut leichter wieder vom Untergrund abzulösen. In diesem Verfahren fungiert das erfindungsgemäße Enzym aufgrund seiner amylolytischen Aktivität als scheidendes Agens in einem temporären Klebeverfahren oder als sogenannter "Schalter" zum Ablösen des Klebeguts.

### Beispiele

### Beispiel 1

### Konstruktion von Primer-Oligonukleotiden zur Amplifizierung von variablen Sequenzbereichen von α-Amylasen

Basierend auf Vergleichen bekannter Sequenzen von α-Amylasen (E.C. 3.2.1.1) grampositiver Eubakterien verschiedener Gattungen der Ordnung Actinomycetales (*Streptomyces, Thermomonospora* und anderer) aus GenBank (National Center for Biotechnology Information NCBI, National Institutes of Health, Bethesda, MD, USA; Stand November 1998) wurden potentiell konservierte Sequenzbereiche identifiziert, die möglichst variable Sequenzbereiche flankieren. Als konservierte Sequenzblöcke wurden die in Figur 1 hervorgehobenen Blöcke A bis E identlfiziert. Sie erstrecken sich über folgende Aminosäure-Positionen: A: 58-91, B: 94-141, C: 155-207, D: 295-345, E: 392-427. Anhand der Sequenzen dieser Bereiche wurden die in Tabelle 1 zusammengestellten Oligonukleotide als erfindungsgemäße Primer entworfen; ihre Sequenzen werden im Sequenzprotokoll unter den Nummern 9 bis 33 angegeben. Ihre Lage im Verhältnis zu den konservierten Sequenzblöcken A bis E innerhalb eines α-Amylasegens ist beispielhaft an der α-Amylase aus *Streptomyces griseus* (GenBank Accession-Number X57568) in Figur 1 dargestellt. Sie lassen sich in Orientierung zu diesem Gen in forward- und reverse-Primer unterscheiden.

**Tabelle 1: PCR-Primer zur Amplifizierung von Teilsequenzen von α-Amylasen von Vertretern der Ordnung Actinomycetales. Angegeben sind jeweils die in Figur 1 verwendeten Namen, die zugehörigen Nummern nach dem Sequenzprotokoll der vorliegenden Patentanmeldung und die Orientierung der Primer in Relation zu den betreffenden Amylasegenen.**

| **Name** | **SEQ ID NO.** | **Orientierung** |
|---|---|---|
| GEX 015 | 13 | forward |
| GEX 016 | 14 | forward |
| GEX 017 | 15 | forward |
| GEX 018 | 16 | forward |
| GEX 019 | 17 | reverse |
| GEX 020 | 18 | reverse |
| GEX 021 | 19 | reverse |
| GEX 022 | 20 | reverse |
| GEX 023 | 21 | forward |
| GEX 024 | 9 | forward |
| GEX 025 | 22 | reverse |
| GEX 026 | 10 | reverse |
| GEX 027 | 23 | forward |
| GEX 028 | 24 | forward |
| GEX 029 | 11 | forward |
| GEX 030 | 25 | reverse |
| GEX 031 | 12 | reverse |
| GEX 036 | 26 | forward |
| GEX 037 | 27 | forward |
| GEX 038 | 28 | reverse |
| GEX 039 | 29 | reverse |
| GEX 040 | 30 | forward |
| GEX 041 | 31 | forward |
| GEX 042 | 32 | reverse |
| GEX 043 | 33 | reverse |

Als Matrize dienten nach Standardmethoden erhaltene genomische DNA-Präparationen bekannter und unbekannter bakterieller isolate aus diversen Bodenproben, die jeweils in Reinkultur vorlagen. Die Primer wurden in Kombinationen von jeweils einem *forward-* mit einem *reverse*-Primer in jeweils einer PCR eingesetzt. Die PCR-Produkte wurden wie unten beschrieben über Agarose-Gelelektrophorese aufgetrennt und zum Screening nach vollständigen Genen verwendet.

Unter den PCR-Ansätzen befanden sich auch solche mit den Primerkombinationen GEX024 (forward) / GEX026 (reverse) und GEX029 (forward) / GEX031 (reverse). Diese sind aus den Sequenzbereichen C (GEX024 und GEX029) und D (GEX026 und GEX031), abgeleitet (Figur 1), weiche den Amylasedomänen β4 und β7 der (αß)₈-Barreistruktur entsprechen, wie sie im Aufsatz,Alpha-Amylase family: molecular biology and evolution° (Janecek, S. (1997), Prog. Biophys. Mol. Biol. 67 (1), S. 67-97) definiert sind. Die Nukleotidsequenzen für diese vier Primer sind im Sequenzprotokoll unter den Bezeichnungen SEQ ID NO. 9 bis SEQ ID NO. 12 angegeben.

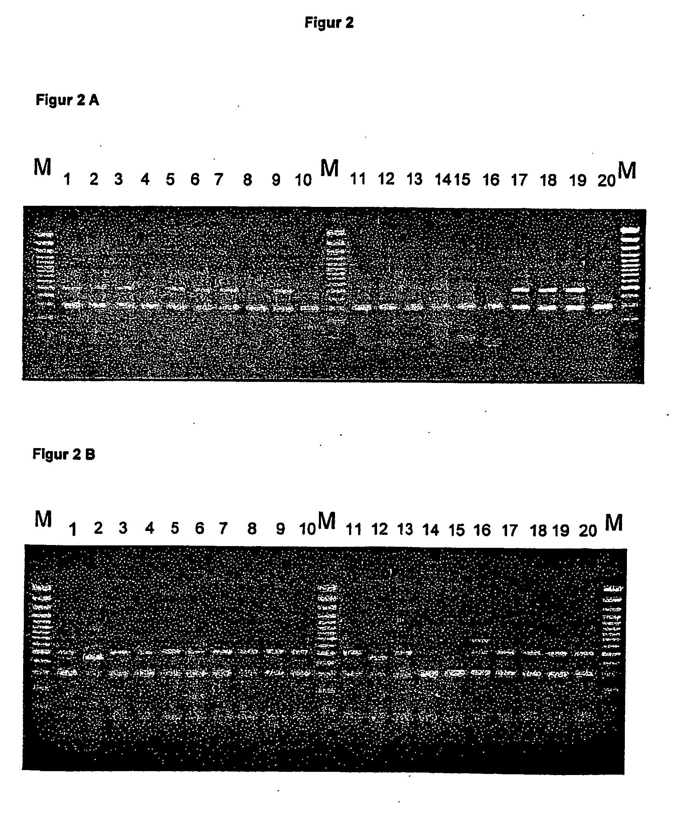

Diese vier Primer ergaben in den PCR-Ansätzen mit den isolierten Nukleinsäuren bakterieller isolate Fragmente reproduzierbarer Größe. Figur 2 zeigt eine beispielhafte Größenanalyse der erhaltenen Ampitfizierungsprodukte von 20 zufällig ausgewählten Präparationen von isolaten der Ordnung Actinomycetales; mit dem Primerpaar GEX024/GEX026 in Figur 2A und mit dem Primerpaar GEX029/GEX031 in Figur 2B. Die PCR-Bedingungen sind in Beispiel 2 angegeben; jeweils 1/10 des Reaktionsvolumens wurde nach der Reaktion in einem 2,5%igen Agarosegel aufgetrennt. Die Ansätze beruhen auf den in der folgenden Tabelle zusammengestellten Stämmen von *Streptomyces sp..*

**Tabelle 2: Stämme von Streptomyces sp., deren PCR-Produkte mit mit den Primerpaaren GEX024/GEX026 und GEX029/GEX031 In Figur 2 gezeigt sind.**

| **Bahnen** | ***Streptomyces sp. ...*** | **SEQ ID NO.** |
|---|---|---|
| 1 | B101A | 45 |
| 2 | B114C | 83 |
| 3 | B134 | 97 |
| 4 | B135A | 98 |
| 5 | B138A | 101 |
| 6 | B152A | 108 |
| 7 | B153(B) | 109 |
| 8 | B161A | 116 |
| 9 | B156B | 111 |
| 10 | B157C | 112 |
| 11 | B158A | 113 |
| 12 | B160B | 115 |
| 13 | B161A | 116 |
| 14 | B373 | 232 |
| 15 | B375 | 234 |
| 16 | B380 | 236 |
| 17 | B390 | 238 |
| 18 | B392A | 239 |
| 19 | B392A | 239 |
| 20 | B394 | 241 |

Mit beiden Primerpaaren wurden DNA-Fragmente entsprechend einer 300 bp-Bande amplifiziert. Daneben tauchte in einigen Ansätzen ein Fragment von ca. 500 bp auf. Beide Produkte wurden durch Sequenzierung (siehe Beispiel 2) als α-Amylase-Sequenzen bestimmt. Die anderen oben und im Sequenzprotokoll aufgeführten Primer zeigten eine geringere Selektivität.

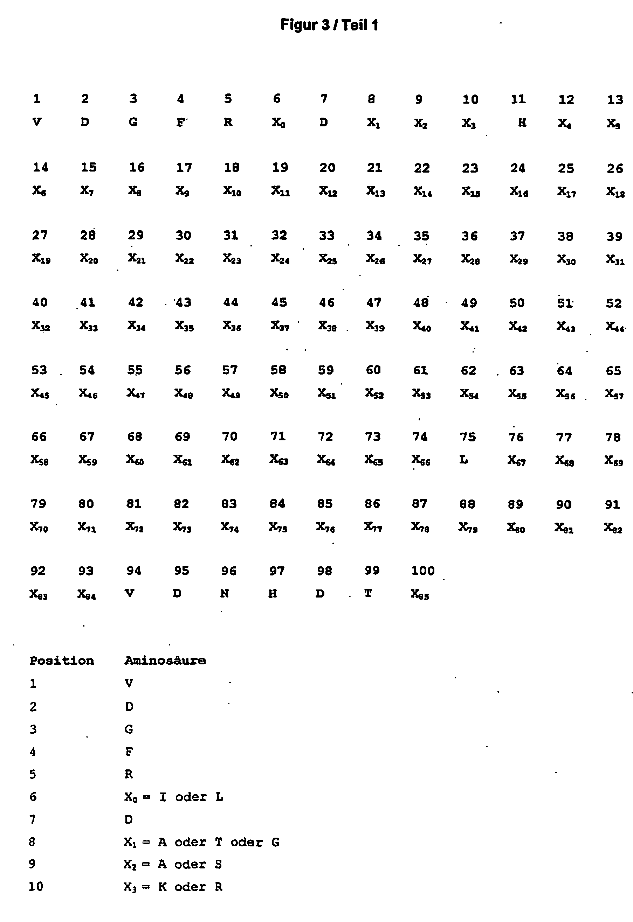

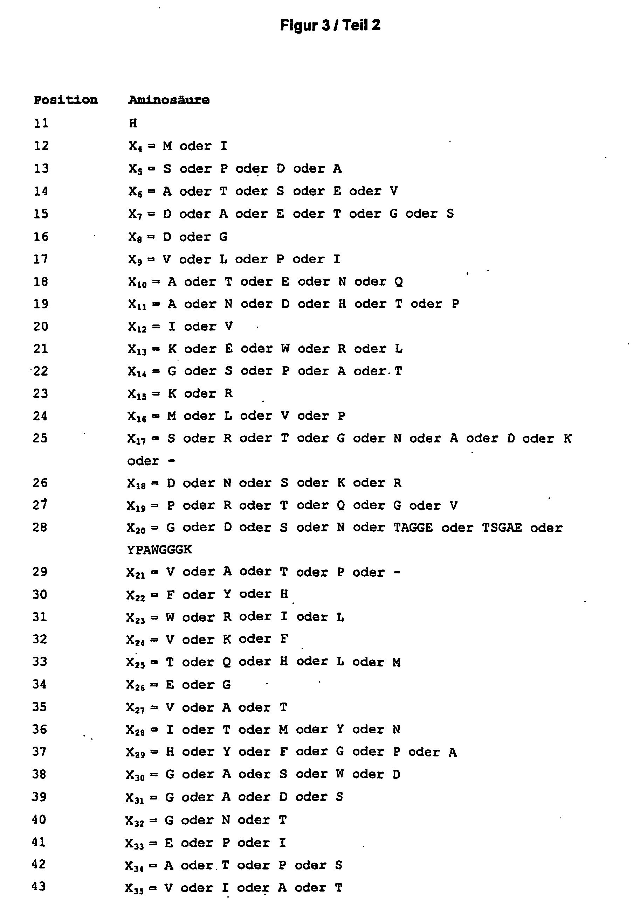

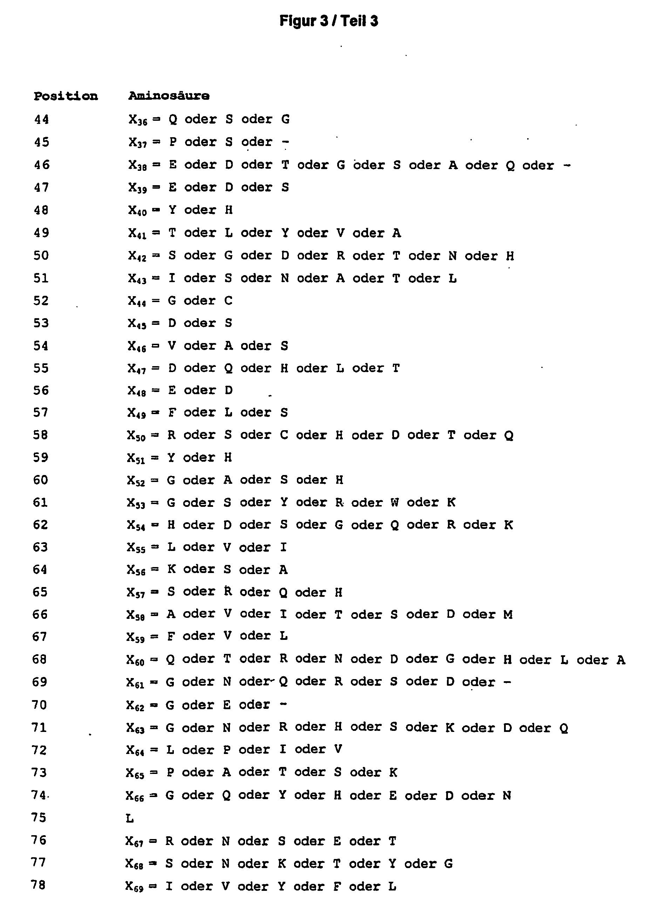

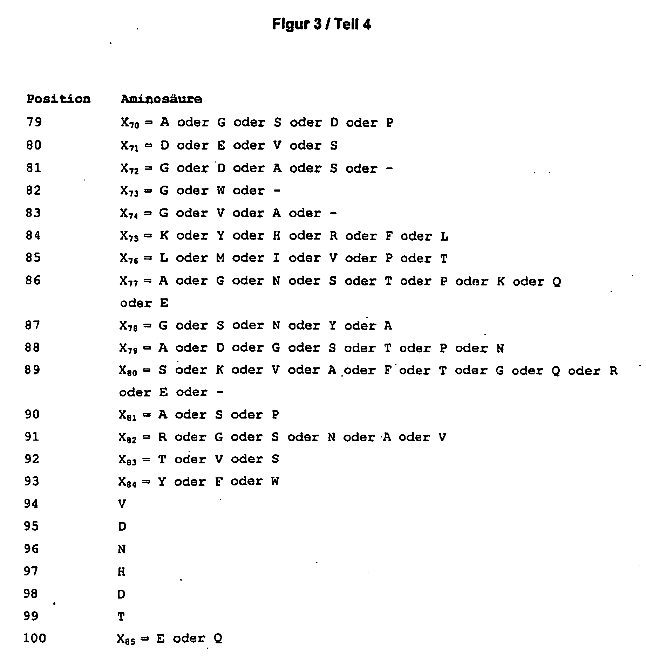

Alle im Sequenzprotokoll aufgeführten Stämme ergaben die gleichen Ergebnisse; insbesondere die Bande von 300 bp war für alle Stämme reproduzierbar. Alle Fragmente, insbesondere die 300 bp-Fragmente, die auf diese Weise von den getesteten Proben erhalten werden konnten, lassen sich homologisieren. Sie weisen insbesondere in den Primer-Bereichen große, wenn auch nicht notwendigerweise völlige Übereinstimmungen auf, die ausgereicht haben, um sie mit der erfindungsgemäßen Methode zu identifizieren. Sie definieren über ihre Sequenzvariationen zueinander, wie sie auch im Sequenzprotokoll unter SEQ ID NO. 263 und in Figur 3 angegeben sind, einen Sequenzvarianzraum innerhalb der Gruppe der amylolytischen Proteine.

### Beispiel 2

### Gewinnung der genomischen DNA aus Actinomyceten und PCR-basierte Amplifizierung und Klonierung von α-Amylase Gensequenzen

Die Anwendbarkeit der erfindungsgemäßen Methode wurde an einer Sammlung von mehreren hundert einzelnen Isolaten der Ordnung Actinomycetales erprobt, welche nach gängigen mikrobiologischen Methoden aus Bodenproben gewonnen worden waren. Es handelte sich jeweils um kultivierte Einzelstämme.

Die Isolierung erfolgte in diesem Beispiel nach Standardmethoden, ausgehend von Bodenproben unter Verwendung von GYM-Medium (4,0 g/l Glucose, 4,0 g/l Hefeextrakt; 10 g/l Malzextrakt, 2,0 g/l CaCO₃ 10 g/l Agar, pH 7,2) unter Zusatz von 50 µg/ml Nystatin zur Unterdrückung der Begleitflora.

### Anzucht der Stämme

Die Anzucht der Actinomyceten-Stämme erfolgte durch Inokulation von 5 ml GHPF-Medium (10 g/l Glucose; 5 g/l Pepton aus Casein; 5 g/l Fleischextrakt; 5 g/l Hefeextrakt; 0,74 g/l CaCl₂ x 2H₂O; pH 7,2) mit 50 µl Sporensuspension bei der jeweils Stammspezifischen Temperatur (28°C bis 50°C) und 200 rpm Schüttelgeschwindigkeit.

Nach ausreichender Mycelbildung wurden 200 ml YEME-Medium (3 g/l Hefeextrakt; 5 g/l Bacto-Trypton; 3 g/l Malzextrakt; 10 g/l D(+)Glucose; 340 g/l Saccharose; pH 7,2; nach Autoklavieren Zugabe von 5 mM MgCl₂ und Glycin, Stamm-spezifisch 0,5 - 20 g/l) mit der gesamten Vorkultur beimpft und bei gleicher Temperatur und bei 170 rpm 3 Tage lang in einem 1-Liter Schikanenkolben geschüttelt.

### DNA Isolierung

Für die DNA-Präparation wurden je 1 g in 20 % (v/v) Glycerin gewaschenes Mycel in 3 ml TE25S-Puffer (25 mM Tris, pH 8,0; 25 mM EDTA; 0,3 M Saccharose) unter Zugabe von 2 mg/ml Lysozym resuspendiert, 30 min bei 37°C invertiert und nach Zugabe von 4 ml 2x Kirby-Mix-Lösung (2 % SDS; 120 g/l Natrium-4-aminosalicylat; 100 mM Tris, pH 8,0; 6% Phenol, gesättigt mit 50 mM Tris/pH 8,0 und supplementiert mit 0,1 % Hydroxychinolin) weitere 10 min invertiert. Anschließend erfolgte eine zweimalige Extraktion der wäßrigen Phase mit nacheinander 8 und 3 ml Phenol/Chloroform/isoamylalkohol (25:24:1) über jeweils 10-minütiges invertieren und nachfolgendes Zentrifugieren bei 2500 x g. Die DNA wurde unter Zugabe von 1/10 Volumen 3 M Natriumacetat (ungepuffert) und 0,6 Volumen isopropanol bei leichtem invertieren gefällt, aufgespult und fünfmal mit 70 % Ethanol gereinigt. Das Pellet wurde in 3 ml HE-Puffer (10 mM HEPES pH 8,0 mit NaOH; 1 mM EDTA) aufgenommen, 1 h bei 50 - 55°C inkubiert und über Nacht bei 4°C gelöst.

Gegebenenfalls kann hieran noch ein Reinigungsschritt angeschlossen werden, um eventuelle, auf eine Amplifikationsreaktion inhibitorisch wirkende, die DNA begleitende Komponenten zu entfernen. Dies war bei einigen DNA-Präparationen erforderlich, bei denen zunächst kein PCR-Produkt (siehe unten) gebildet worden war. Hier mußte zunächst der Einfluß von inhibitorischen Komponenten in der DNA-Präparation durch Verdünnen (bis zu 1:100) oder durch Aufreinigung über eine QlAquick PCR-Purification Säule (Qiagen, Hilden) nach Herstellerangaben unterbunden werden.

Am Folgetag fand eine Aufarbeitung der DNA durch einstündige Inkubationen bei 37°C, nacheinander mit 40 µg/ml RNaseA (hitzeinaktiviert) und 0,5 mg/ml Proteinase K statt. Anschließend wurde die wäßrige Phase im gleichen Volumen Phenol/Chloroform/isoamylalkohol (25:24:1) extrahiert, 10 min invertiert, bei 2500 x g zentrifugiert und wie am Vortag gefällt, nach Aufspulen gewaschen und in 1 ml HE-Puffer gelöst.

### Amplifizierung der Amylasesequenzen durch die erfindungsgemäße PCR

Vor einer Charakterisierung der Amylase-Sequenzen erfolgte zunächst eine Amplifizierung durch die Polymerase Kettenreaktion (PCR). Diese wurde unter Verwendung von HotStar Taq-Polymerase (Firma Qiagen, Hilden) und der Amylasespezifischen Primer durchgeführt (Beispiel 1, Tabelle 3). Hierfür wurden jeweils 4 µl der nach obigem Verfahren isolierten genomischen DNA in Gegenwart von 200 µM dNTPs (jeweils dATP, dTTP, dCTP, dGTP), 20 pmol jeweils eines forward- und eines reverse-Primers, 2,5 U HotStar Taq-Polymerase (Firma Qiagen, Hilden) und 1 x PCR-Puffer in 50 µl Gesamtvolumen eingesetzt. Es wurden Fragmente der Größen 280 bis 500 bp in folgenden Zyklen amplifiziert: 1 Zyklus 15 min bei 95°C; 40 Zyklen mit je 30 s bei 95°C, 30 s bei 60°C und 30 s bei 72°C; 1 Zyklus 7 min bei 72°C.

Insbesondere das Primerpaar GEX024/GEX026 lieferte konstante Ergebnisse bei allen genomischen DNAs bezüglich eines 300 bp Produktes, das in der Sequenzierung Homologien zu Amylasen zeigte (siehe Beispiel 1). Bei einigen DNA-Präparationen kam es zunächst zu keiner Produktbildung. Hier mußte zunächst der Einfluß von inhibttorischen Komponenten in der DNA-Präparation durch Verdünnen (bis zu 1:100) oder durch Aufreinigung über eine QIAquick^{Ⓡ}-PCR-Purifcation Säule (Firma QIagen, Hilden) nach Herstelterangaben unterbunden werden.

Die mit der Primerkombination GEX02MGEX028 erhaltenen Banden (ca. 300 bp) wurden zur Sequenzierung mithilfe des QIAEX II^{Ⓡ}-kits der Firma Qiagen (Hilden) eluiert und mittels des TOPO TA^{®}-kits (Firma Invitrogen, Groningen. Niederlande) nach Herstellerangaben in den pCR2.1-TOPO^{Ⓡ}-Vektor kloniert. Mit den LIgationsprodukten wurde in *E.coli*-TOP^{®} 10F' (Firma Invitrogen, Groningen, Niederlande) transformiert. Nach Blau/weiß-Selektion rekombinanter Klone auf LB-Medium mit Ampicillin (100 µg/ml), IPTG (100 mM), X-Gal (40 mg/ml) wurde die Plasmid-DNA von weißen Klonen nach Minipräparation in einer Restriktionsanalyse mit *Eco*RI auf insert-DNA überprüft.

Die Sequenzanalyse von Plasmiden mit insert-DNA erfolgte routinemäßig auf einem ABI PRISM^{Ⓡ} 310 (Firma Perkin Elmer, Weiterstadt) nach Herstellerangaben mittels AmpliTaq-FS-Big-Dye^{Ⓡ} Terminator-Kif (Firma Perkin Eimer, Weiterstadt) unter Einsatz von 200-500 ng Plasmid-DNA, 10 pmol Primer (TopoF: 5'-GCTCGGATCCACTAGTAACG-3' und TopoR: 5'-CTCTAGATGCATGCTCGAG-3'), 4 µl Premix in einem Gesamtvolumen von 20 µl. Die erhaltenen DNA-Sequenzen wurden routinemäßig konzeptionell in eine Aminosäuresequenz umgeschrieben, über die BlastX, BlastN und BlastP Algorithmen mit den vorhandenen GenBank-Einträgen verglichen und auf diese Weise als Teilsequenzen von α-Amylasegenen identifiziert. Die gefundenen Teilsequenzen werden im Sequenzprotokoil unter den Nummern SEQ ID NO. 2, 4 und 34 bis 262 ausgeführt und In der Consensus-Sequenz der Figur 3, beziehungsweise der SEQ ID NO. 263 zusammengepaßt

### Beispiel 3

### Detektion von für α-Amylase codierenden, full-length Gensequenzen durch Aktivitäts-Screenlng von Expressionsgenbanken

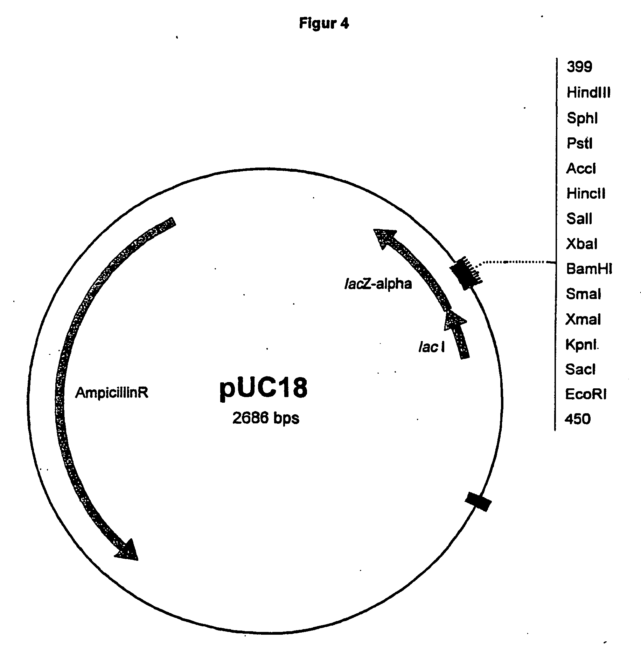

Zur Montierung und Isolierung vollständiger α-Amylasegene aus Actinomycetales wurde eine Expressionsklonierung im heterologen Wirtsorganismus *Escherlschla coli* und zwar dem Stamm DH 12S gewählt. Als Promotor wurde der mit IPTG (isopropylthlogalactosid) induzierbare β-Gatactosidase-Promotor des lac-Operons (*lac*-Promotor) eingesetzt Dieser wurde auf dem pUC18 Plasmidvektor (GenBank (National Center for Biotechnology Information NCBI, National Institutes of Health, Bethesda, MD, USA); Accession-Number L08752; Figur 4) bereitgestellt.

### Die Herstellung von Expressionsgenbanken für die jeweiligen Stämme

Gereinigte genomische DNA aus den jeweiligen Actinomycetales-Stämmen, die gemäß Beispiel 2 erhalten worden war, wurde partiell mit dem Restriktionsenzym *Aci* I gespalten, und der Größenbereich von 3 bis 5 kb in mit dem Restriktionsenzym *Acc* I linearisierte pUC18-Plasmidvektoren (Figur 4) ligiert. Bei einer mittleren Insertgröße von 4 kb und einer geschätzten Actinomyceten-Genomgröße von 8 Mb ergibt sich für eine gewünschte 5-fache Genomabdeckung in einer Genbank eine Zahl von 20 000 Primärklonen. Da die. Expression vom plasmideigenen *lac*-Promotor ausgeht, die Inserts aber jeweils in zwei Orientierungen integrieren können, erhöht sich diese Zahl auf mindestens 40 000 Klone. Dies war die für jede Genbank erreichte Mindestklonzahl.

Zur Ermittlung der optimalen Restriktionsparameter für den präparativen Partialverdau genomischer DNA aus Actinomyceten wurden zunächst Restriktionskinetiken unter Einsatz von 6 µg DNA und Restriktionsenzym (*Aci*I) in Konzentrationen von 0,1 bis 0,4 U pro µg DNA in 1 x-Puffer, gegebenenfalls laut Herstellerangaben mit Rinderserumalbumin (BSA) in einem Gesamtvolumen von 15 µl durchgeführt. Die Ansätze wurden dafür zunächst ohne Enzym auf 37°C temperiert. Dann wurde die jeweilige Reaktion durch Zugabe von Restriktionsendonuklease gestartet. In festgelegten Intervallen zwischen 0 und 10 min wurden jeweils 1,5 µl Reaktionsansatz zu 1 x-Stop-Puffer (6X: 10 mM Tris, pH 7,0; 20 % Glycerin; 0,1% SDS) auf Eis gegeben und auf einem 0,7 %-igen Agarosegel analysiert. Dadurch wurde individuell für jede DNA-Präparation die optimale Restriktionsdauer für einen partiellen Verdau ermittelt; er lag im Durchschnitt bei 4 bis 5 min.

Der präparative Partialverdau erfolgte wie oben beschrieben, jedoch im zehnfachen Ansatz unter Einsatz von 60 µg chromosomaler Actinomyceten-DNA und der zuvor optimierten Enzymmenge für die jeweils individuell bestimmte Dauer. Nach Abstoppen der Reaktion durch Zugabe von 1 x-Stop-Puffer wurde der Ansatz auf einem 0,7 %igen Agarosegel elektrophoretisch aufgetrennt, der Gelbereich mit DNA der Größe von 3-5 kb ausgeschnitten und dieser über zweistündige Elektroelution in Dialyseschläuchen bei 4°C isoliert. Die mit 1/10 Volumen von 3 M Natriumacetat und dem 2,5-fachen Volumen Ethanol gefällte und in einem kleineren Volumen aufgenommene DNA wurde zur weiteren Abtrennung von kleineren Fragmenten einer zweiten Gelelektrophorese mit anschließender Elektroelution und erneuter Aufkonzentrierung unterzogen.

Die Ligation mit dem Plasmidvektor pUC18 erfolgte über Nacht bei 16°C in einem Gesamtvolumen von 20 µl unter Einsatz von 150 ng mit *Acc* I linearisiertem, nach Herstellerangaben mit CIAP (Alkalische Phosphatase aus Kalbsthymus) dephosphoryliertem pUC18-Vektor und 450 ng partiell gespaltener genomischer DNA, sowie einer angemessenen Ligase-Menge (hier 400 NEB-Units) in 1 x-Ligase-Puffer.

Die Transformation kompetenter *E.coli* DH 12S Zellen (Firma Gibco Life Technologies, Karlsruhe) erfolgte über Elektro-Transformation. Hierfür wurden 1 µl Ligationsansatz und 30 µl Zellen gemischt, in einer Elektroporationsküvette 1 min lang auf Eis inkubiert und im Elektroporator (BTX^{®} ECM399, Firma Genetronics Inc., San Diego, CA, USA) nach Herstellerangaben behandelt. Nach sofortiger Überführung in 1 ml SOC-Medium (2% Bacto-Trypton; 0,5% Hefeextrakt; 10 mM NaCl; 2,5 mM KCI; pH 7,0, eingestellt mit NaOH; autoklaviert; supplementiert mit 10 mM MgSO₄ und MgCl₂ sowie 20 mM D(+)Glucose) folgte vor der Plattierung eine Erholungsphase von 1 h bei 37°C.

Zur Untersuchung der Qualität der Genbank wurde die Anzahl der insgesamt erzeugten Primärtransformanten und die Anzahl Insert-tragender Klone über Blau/Weiß-Selektion in einer Testplattierung bestimmt. Hierfür wurden je 1 und 10 µl des Ligationsansatzes auf LB-Medium mit Ampicillin, IPTG, X-Gal (wie oben beschrieben) ausplattiert und über Nacht bei 37°C inkubiert. Zur Bestätigung der tatsächlich klonierten Insertgrößen erfolgte die Isolation der Plasmide von mindestens 10 weißen Kolonien der Testplattierung und ein geeigneter Restriktionsverdau mit nachfolgender gelelektrophoretischer Größenanalyse.

### Aktivitäts-Test der Genbanken

Zunächst wurde die Fähigkeit der Genbanken zum Abbau von Stärke ermittelt. Hierfür wurde LB-Agar mit 1 % löslicher Stärke (Firma Merck, Darmstadt, Best.-Nr. #1252) verwendet, auf welcher Klone, die zum Abbau von Stärke befähigt sind, durch Lysehöfe zu identifizieren sind. Dafür war eine zwei- bis vierwöchige Lagerung der Platten bei 4°C bis zur Eintrübung notwendig wonach ein Abbau der Stärke als Aufklaren der Platten um die Kolonien herum sichtbar war. Ergänzend kam mit Cibacron Brilliant Red^{®} 3B-A (Firma Aldrich, Taufkirchen) markierte lösliche Stärke (ca. 0,6 % w/v) zum Einsatz (Blely, P., Mislovicova, D., Markovic, O., Kalac, V. (1988): "A new chromogenic substrate for assay and detection of alpha-amylase"; Anal. Biochem., Band 172 (1), Seiten 176-179), wobei Stärkeabbau an einer Entfärbung des rötlichen Agars im Bereich des positiven Klons erkennbar ist Vor Gebrauch wurden den Platten in beiden Fällen IPTG (100 mM) zur Induktion des Promotors und Ampicillin (100 µg/ml) zur Ausübung des Selekdonsdrucks auf die Transformanten zugesetzt.

Entsprechend des Titers der jeweils erzeugten Bank wurde ein definiertes Volumen des Transformationsansatzes mit ca. 10 000 Kolonien je Platte (14 cm Durchmesser) mittels Glaskugeln gleichmäßig ausplattiert (primäre Plattierung). Nach 16-stündiger Inkubation bei 37°C wurden die Platten weitere 24 - 48 h bei 28°C zur Amylase-Expression und Freisetzung inkubiert. Dabei war die Inkubation bei 28°C in der Regel für einen sichtbaren Abbau von Stärke unabdingbar. Hierbei mögen eine verbesserte Proteinfaltung und eine Permeabilisierung der äußeren Zellmembran eine Rolle spielen (Stathopoulos, C., Georgiou, G., Earhart, C. F. (1996): "Characterization of Escherichia coli expressing an Lpp'OmpA(46-159)-PhoA fusion protein localized in the outer membrane"; Appl. Microbiol. Biotechnol., Band 45 (1-2), Selten 112-119). Damit ging also eine Freisetzung der jeweils gebildeten α-Amylase einher, und eine zusätzlich durchzuführende Zell-Lyse zum Nachweis nicht exportierter cytoplasmatischer Amylasen war nicht nötig.

Nach einer Vereinzelung der Kolonien aus den Hofbereichen der primären Plattierung durch erneute (sekundäre) Plattierung auf den gleichen Stärkeplatten konnten Amylasebildende Einzelkolonien anhand erneuter Hofbildung selektiert werden. Für fotodokumentarische Zwecke hat sich nach Sicherung der Klone eine Färbung der Stärkeplatten mit 50% Lugal-Lösung (Firma Merck) zur eindeutigen Visualisierung von Stärke-freien Bereichen (Abbauhöfen) bewährt.

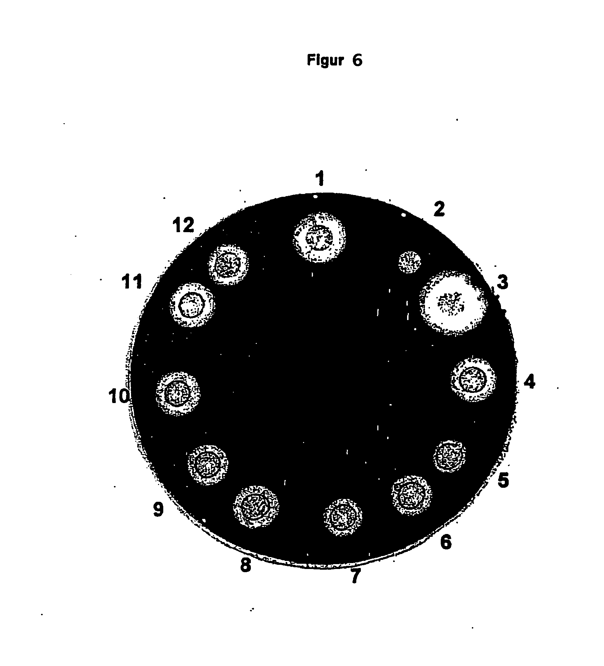

Figur 6 zeigt eine entsprechend gefärbte, stärkehaltige LB_{Amp}-Agarplatte, auf die jeweils Suspensionen von positiven Klonen der Sekundärplattierung aufgetropft worden sind.

### Isolierung einzeiner Klone

Die nach Minipräparation (Kit der Firma Qiagen, Hilden, Deutschland, verwendet nach Herstellerangaben) erhaltene Plasmid-DNA positiver Klone wurde in einer Restriktionsanalyse mit *Eco* R **I** oder *Sac **II** Hlnd* **III** auf die Insertgröße hin untersucht und anschließend sequenziert. Hierbei kamen zunächst die Insert-flankierenden M13-Primer (M13 forward: 5'-GTAAAACGACGGCCAG-3'; M13 reverse: 5'-CAGGAAACAGCTATGAC-3') zum Einsatz, um schließlich die Gesamtsequenz über sogenanntes Primer-walking zu erhalten, wie es aus dem Stand der Technik bekannt ist Das Prinzip dieses Vorgehens besteht darin, daß in einer sich wiederholenden Abfolge ausgehend von ermittelten Sequenzen neue Sequenzierprimer abgeleitet und für die Sequenzierung der sich anschließenden, noch unbekannten Bereiche verwendet werden; aus diesen lassen sich wiederum die nächsten Sequenzierprimer ableiten.

### Beispiel 4

### Heterologe Expression von α-Amylasen in prokaryontischen Expressionssystemen

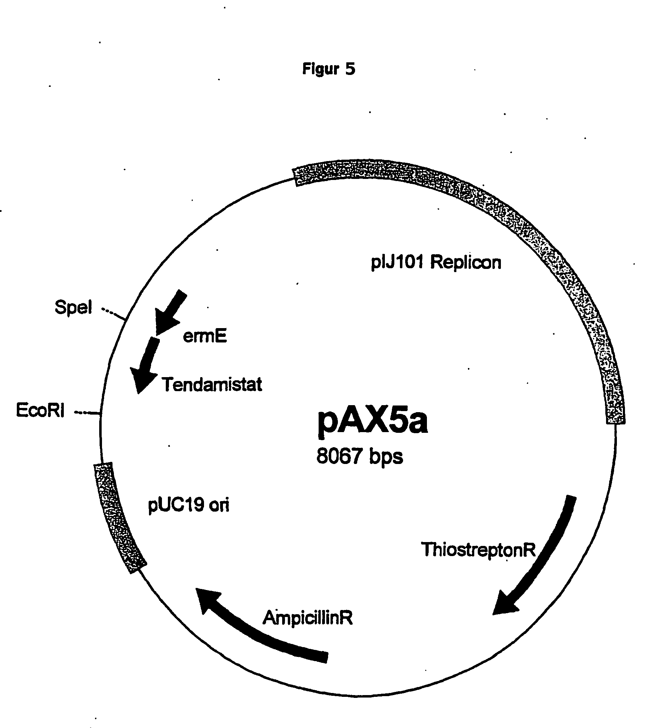

Zur Charakterisierung der biochemischen Eigenschaften der klonierten α-Amylasen sollten diese in größeren Mengen löslich produziert werden. Sie erfolgte für die vollständigen Amylasegene von *Streptomyces sp.327*. Streptomyces sp.* B400B und das Actinomycetales-Isolat *Streptomyces sp.* B327B. Als Expressionswirt wurde *Streptomyces lividans* TK24 eingesetzt; als Expressionsvektor diente pAX5a (Figur 5) Darin steht die Expression unter Kontrolle des konstitutiven ermE-Promotors.

Um die für die Klonierung notwendigen *Spe* I- und *Eco* RI-Schnittstellen einzufügen und um die selteneren und daher möglicherweise schlechter translatierten Startcodons der Amylasegene von B327* (GTG) und B400B (TTG) durch ATG zu ersetzen, erfolgte eine PCR mit entsprechend modifizierten Primern. So wurden beispielsweise zur Klonierung der vollständigen α-Amylase aus *Streptomyces sp.327** der forward-Primer: 5' aaaactagtA**AGGAG**AACCCCCACaTGATATCGAG 3' und der reverse-Primer: 5' gaattcgccctt**TCA**GCAGTTGGCCTTGCCCG 3' verwendet. Neue, nicht komplementäre Nukleotide sind hier in Kleinbuchstaben widergegeben. Im forward-Primer sind die Erkennungsstelle für das Restriktionsenzym *Spe* I und das Start-Codon unterstrichen; die Shine-Dalgarno-Sequenz ist darin fett hervorgehoben. Im reverse-Primer ist die Erkennungsstelle für das Restriktionsenzym *Eco* RI unterstrichen und das Stop-Codon (in umgekehrter Orientierung) fett hervorgehoben. Als Matrize dienten die aus den Genbanken isolierten genomischen Klone.

Nach einer Zwischenklonierung der mit *Pfx* Platinum Polymerase erzeugten PCR-Produkte in dem Vektor pCR Blunt II TOPO (Firma Invitrogen, Groningen, Niederlande) erfolgte die gerichtete Insertion in den Streptomyceten-Expressionsvektor pAX5a unter Verwendung der *Spe* I- und *Eco* RI-Restriktionsschnittstellen. Anschließend erfolgte die Protoplastentransformation von *S*. *lividans* TK 24 nach Kieser et al. (Kieser, T., Bibb, M.J., Buttner, M.J., Chater, K.F., Hopwood, D.A. (2000): "PEG-assisted transformation of Streptomyces protoplasts with plasmid DNA. Rapid small-scale procedure." in: "Practical Streptomyces Genetics", The John Innes Foundation (Herausgeber), Crows, Norwich, England) und eine Ausplattierung der Transformanten auf dem selektivem Regenerationsmedium R2YE. Dieses besteht aus: 103 g Sucrose, 0,25 g K₂SO₄, 0,12 g MgCl₂x6H₂O, 10 g Glucose, 100 mg Difco Casaminoacids (Firma Difco, Heidelberg), 10 ml 0,5%ige (w/v) KH₂PO₄-Lösung, 80 ml 3,68%ige (w/v) CaCl₂-Lösung, 1,5 ml L-Prolin, 100 ml TES-Puffer (pH 7,2; 2-{[Tris(hydroxymethyl)methyl)amino}-ethansulfonsäure), 0,2 ml Spurenelementelösung (40 mg/l ZnCl₂, 200 mg/l FeCl₃x 6 H₂O, 10 mg/l CuCl x 2 H₂O, 10 mg/l MnCl₂ x 2 H₂O, 10 mg/l Na₂B₄O₇x 10 H₂O, 10 mg/l (NH₄) 6 Mo₇O₂₄x 4 H₂O) und 5 ml 10%iges (w/v) Difco-Hefeextrakt (Firma Difco, Heidelberg), auf 1 l H₂O) mit 75 µg/ml Thiostrepton als Selektivantibiotikum.

Nach Umplattierung auf NBSA-Indikatorplatten (8 g/l Nutrient Broth Difco, 15 g/l Agar Serva, 1,5% (w/v) lösliche Stärke Merck #1252, pH 8,0; Hersteller: Firma Difco, Heidelberg; Firma Serva, Heidelberg; Firma Merck, Darmstadt) und Identifizierung Amylase-positiver Krone, welche anhand der entstehenden Stärkeabbauhöfe erkannt werden konnten, erfolgte eine Anzucht in Flüssigkultur zur Gewinnung enzymatisch aktiver Überstände. Hierzu wurde eine 5-ml-Vorkultur für 16 h und in Folge eine 50-ml-Hauptkultur in 300 ml Erlenmeyerkolben mit Schikanen unter Verwendung von GYM-Medium (4 g/l Glucose, 4 g/l Hefeextrakt, 10 g/l Malzextrakt, 2 g/l CaCO₃) mit 75 µg/ml Thiostrepton für 4 Tage kultiviert. Der Amylase-haltige Kulturüberstand wurde durch Abtrennung der Zellmasse durch Zentrifugation für 15 min bei 2500 g und anschließender Filtration (0,45 µm) gewonnen und direkt der Vermessung zugeführt oder nach Schockgefrieren in flüssigem Stickstoff im Gefrierschrank bei -20°C gelagert.

### Beispiel 5

### Charakterisierung der amylolytischen Aktivität von Vertretern der α-Amylasen

### Charakterisierung der enzymatischen Eigenschaften

Im Hinblick auf die vielfältigen Anwendungsgebiete von α-Amylasen erfolgt die Charakterisierung der enzymatischen Eigenschaften, welche die Grundlage zur anwendungsbezogenen Auswahl von Enzymen sein kann. Die Darstellung der Enzyme kann hierbei durch Klonierung und heterologe Expression der Gensequenzen entsprechend den Beispielen 3 und 4 und dem oben gezeigten Vorgehen erfolgen.

Die amylolytische Aktivität wurde mittels des Dinitrosalicylsäure-Tests (DNSS-Test) bestimmt. Dabei wird die Hydrolyse eines komplexen Stärke-Substrates anhand der Zunahme reduzierender Enden des Polysaccharides bestimmt, wobei die Absorption bei 540 nm an umgesetztem DNSS-Reagenz als Maß für die hydrolytische Aktivität dient

Für den Test wurden in einer 96-Well-Platte für PCR-Thermocycler (0,2 ml "thin-wall plate" #3416, MBP (Molecular BioProducts, San Diego, USA) in die Proben- und Blank-Kavitäten jeweils 25 µl Substratlösung (1% lösliche Stärke Merck p.a. in 180 mM Tris-Maleat-Puffer, pH 8,6, beziehungsweise in 180 mM des entsprechenden Test Puffers) vorgelegt und 5 min bei 50°C, beziehungsweise der jeweiligen Test-Temperatur in einem Thermocyder-Block vorinkubiert. Nach Zugabe von 20 µl Enzymlösung je Well erfolgte die Substratumsetzung für 15 min bei 50°C, beziehungsweise der jeweiligen Test Temperatur. Nach Zugabe von 65 µl DNSS-Reagenz (8,8 g Dinitrosalicylsäure, 250 g Kalium-Natrium-Tartrat, 6,3 g Natriumdisulflt, 334 ml 4,5% (w/v) NaOH, 915 ml H₂O) in die Proben- und Blank-Kavitäten sowie der Zugabe von 20 µl Enzymlösund in die Blank-Kavitäten wurde die Testplatte sofort in einen auf 100°C vorgewärmten Thermoblock transferiert und 20 min bei 100°C inkubiert. Die Messung der Proben erfolgte durch Überführen von je 60 µl des Testansatzes in 200 µl H₂O, welche in einer 96-Well-Meßplatte (PS-Microplate, 96 Well #655101, Greiner) vorgelegt worden waren, und anschließender Bestimmung der Absorption bei 540 nm mittels Mikrotiterplatten-Spektralphotometer (Spectramax 190, Firma Molecular Devices, Sunnivale, USA) mit H₂O als Referenz. Es wurden jeweils drei Messungen vorgenommen; die Auswertung erfolgte durch Subtraktion des Mittelwertes der 3 Blank-Kavitäten vom Mittelwert der 3 Proben- Kavitäten.

### Temperaturstabilität und Temperaturprofil

Zur Bestimmung der Temperaturstabilität erfolgte eine Vorinkubation der Lösungen rekombinant erhaltener α-Amylasen für 15 min bei verschiedenen Temperaturen und der anschließenden Bestimmung der Restaktivität gemäß der oben beschriebenen Vorgehensweise bei 50°C und 100 mM Tris-Maleat, pH 8,6. Für die α-Amylase aus *Streptomyces sp.* B327B ergab sich eine maximale Stabilität bei 45°C, doch war der Effekt erhöhter Temperaturen bis 61°C mit Restaktivitäten von mehr als 80% relativ moderat. Die weiteren Ergebnisse sind in Tabelle 3 zusammengestellt, wobei der optimale Wert von 45°C auf 100% gesetzt worden ist.

**Tabelle 3. Temperaturstabilität der α-Amylase aus Streptomyces sp. B327***

| **Temperatur der Vorinkubation [°C]** | **Restaktivität relativ zu der von 45°C [%]** |
|---|---|
| 45 | 100 |
| 50,4 | 94 |
| 55,8 | 86 |
| 61 | 84 |
| 64,7 | 46 |

Zur Bestimmung des Temperaturprofils erfolgte die Umsetzung gemäß der oben beschriebenen Vorgehensweise bei verschiedenen Temperaturen und 100 mM Trls-Maleat-Puffer, pH 8,6. Für die α-Amylase aus *Streptomyces sp.* B327* ergab sich eine maximale Aktivität bei 41,3°C. Höhere Temperaturen führten bei diesem Enzym zu deutlichen Aktivitätsverlusten (siehe Tabelle 4). Demgegenüber war die Aktivität der Amylase aus *Streptomyces sp.* B327B über den getesteten Temperaturbereich relativ konstant (siehe Tabelle 5).

**Tabelle 4: Temperaturprofll der α-Amylase aus Streptomyces sp. B32T***

| **Temperatur [°C]** | **Aktivität relativ zu der von 41,3°C [%]** |
|---|---|
| 40 | 87 |
| 41,3 | 100 |
| 50,7 | 38 |
| 56 | 38 |
| 59,8 | 35 |

**Tabelle 5. Temperaturprofil der α-Amylase aus Streptomyces sp. B327B.**

| **Temperatur [°C]** | **Aktivität relativ zu der von 41,3°C [%]** |
|---|---|
| 40 | 103 |
| 41,3 | 100 |
| 50,7 | 117 |
| 56 | 92 |
| 59,8 | 83 |

Man erkennt, daß die α-Amylase aus *Streptomyces sp.* B327B über einen breiteren Temperaturbereich und bis zu höheren Temperaturen stabiler ist als die aus *Streptomyces sp.* B327*. Die mit der vorliegenden Erfindung zur Verfügung gestellten amylolytischen Enzyme zeichnen sich hinsichtlich ihrer Temperatur-Sensitivität also über eine beachtliche Variabilität aus.

### Stabilität gegenüber pH-Wert-Schwankungen

Zur Bestimmung der Aktivität der α-Amylase-Amylasen unter verschiedenen pH-Wert-Bedingungen wurde die Stärke-Substratlösung Im pH-Bereich von 6,0 bis 8,6 mit dem entsprechend eingestellten 180 mM Tris-Maleat-Puffer angesetzt; für pH 8,6 und den darüber liegenden pH-Bereich wurde sie mit 180 mM Glycin-NaOH-Puffer angesetzt. Nach Verdünnung der Substratlösung ergaben sich unter Testbedingungen die gewünschten pH-Werte und Pufferkonzentrationen von 100 mM Tris-Maleat-, beziehungsweise 100 mM Glycin-NaOH-Puffer. Durch Messung bei pH 8,6 in beiden Puffersystemen konnten Einflüsse des Puffersystems ausgeglichen werden. Die Bestimmung der amylolytischen Aktivität unter den jeweiligen pH-Werten erfolgte wie oben angegeben bei 50°C. Die erhaltenen Ergebnisse sind In folgender Tabelle 9 zusammengestellt. Die Aktivitäten bei pH 6,5 der beiden α-Amylasen aus *Streptomyces sp.* B327* und B327 wurden jeweils auf 100% gesetzt; die jeweils nachfolgenden Werte sind auf diese bezogen.

**Tabelle 6. Amylolytische Aktivität der α-Amylasen aus Streptomyces sp. B327* und dem Actinomycetales-Isolat Streptomyces sp. B327B bei alkalischen pH-Werten.**

| **pH-Wert der Vorinkubation** | **Relative Aktivität der α-Amylase aus *St. sp.* B327* [%]** | **Relative Aktivität der α-Amylase aus *St*. *sp*. B327B [%]** |
|---|---|---|
| 6,5 | 100 | 100 |
| 8,6 | 38 | 129 |
| 10 | 21 | 84 |
| 12 | 13 | 43 |

Gegenüber der α-Amylase aus *Streptomyces sp.* B327* ist die aus *Streptomyces sp.* B327 in einem stärker alkalischen Medium stabiler und weist auch bis zu sehr hohen pH-Werten eine höhere Stabilität, beziehungsweise höhere amylolytische Aktivität auf.

### Stabilität gegenüber Tensiden

Zur Bestimmung der Stabilität gegenüber Tensiden wurden zwei Aktivitäts-Meßreihen vorgenommen. In der ersten wurde die α-Amylase wie oben beschrieben mit einer Stärke-Substratlösung mit 0,018 % (w/v) Natriumdocecylsulfat (SDS) vorinkubiert, so daß sich nach Verdünnung eine Konzentration von 0,01% SDS im Testansatz ergab. Die Bestimmung der Aktivität erfolgte wiederum bei 50°C und 100 mM Tris-Maleat-Puffer, pH 8,6. Setzt man diese Werte jeweils auf 100%, so weisen beide untersuchtenα-Amylasen in Abwesenheit von SDS geringere Aktivtäten auf: die der α-Amylase aus *Streptomyces* sp. B327* verfügt ohne SDS über eine Aktivität von 94,2% und die *ausStreptomyces sp.* B327B über eine von 89%. Dies zeigt, daß beide stichprobenartig ausgewählten Vertreter der neuen Gruppe von α-Amylasen eine ausreichende Stabilität in Anwesenheit einer für viele Anwendungen typischen Tensidkonzentration besitzen.

Zur Charakterisierung der enzymatischen Aktivität in Anwesenheit von potentiell stabilisierenden zweiwertigen Ionen, beziehungsweise in Anwesenheit von Komplexbildnern wurden stichprobenartig ausgewählte Enzyme mit 3,6 mM CaCl₂-haltiger Stärke-Substratlösung (1% Stärke) inkubiert oder mit einer 1,8 mM EDTA enthaltenden Stärke-Substratlösung, so daß sich im wie oben beschrieben durchgeführten Test Endkonzentrationen von 2 mM Ca²⁺, beziehungsweise 1 mM EDTA ergaben. Die Bestimmung der Restaktivität erfolgte wiederum bei 50°C und 100 mM Tris-Maleat-Puffer, pH 8,6.

Setzt man die Aktivitäten beider Enzyme bei 2 mM CaCl₂ jeweils auf 100%, so sank die Aktivität der α-Amylase aus *Streptomyces sp.* B327* in Anwesenheit von 1 mM EDTA auf 91%. Die der α-Amylase aus *Streptomyces sp.* B327B sinkt in Anwesenheit von 1 mM EDTA auf 57%. Dies zeigt, daß beide Enzyme in Gegenwart von Tensiden und von Komplexbildnern bemerkenswert hohe Restaktivitäten aufweisen. Allerdings reagieren beide Enzyme deutlich unterschiedlich und divers auf diese Einflüsse, wie die verschiedene Aktivität in Anwesenheit von Komplexbildnern zeigt.

Die Ergebnisse dieses Beispieles zeigen zum einen, daß die gefundenen erfindungsgemäßen Enzyme nicht allein aufgrund ihrer DNA- und Protein-Sequenzen als α-Amylasen anzusehen sind, sondern tatsächlich auch über eine Stärke-spaltende Aktivität verfügen. Die Abweichungen beider untersuchter Enzyme hinsichtlich ihrer Anforderungen an die Reaktionsbedingungen können als Beleg dafür angesehen werden, daß die vorliegende Erfindung über die grundlegende Übereinstimmung hinaus ein breites Spektrum an amylolytischen Enzymen mit individuellen Unterschieden zur Verfügung stellt.

### Beschreibung der Figuren

### Figur 1: Position der potentiell konservierten Aminosäuresequenzblöcke ("Sequenzanker" A-E) in α-Amylasen (Glykosylhydrolasen Familie 13, E.C. 3.2.1.1), abgeleitet aus α-Amylase-Sequenzen von Actinomycetales, und die relative Lage der erfindungswesentlichen in Tabelle 3 zusammengestellten und im Sequenzprotokoll angegebenen PCR-Primer (Beispiel 1).

Beispielhaft dargestellt sind die 567 Aminosäuren, beziehungsweise 1701 bp umfassenden Aminosäure-, beziehungsweise DNA-Sequenzen der α-Amylase aus *Streptomyces griseus* (Genbank (National Center for Biotechnology Information NCBI,

National Institutes of Health, Bethesda, MD, USA), Accession-Number X57568) mit den konservierten Domänen A bis E, die sich über folgende Aminosäure-Positionen erstrecken: A: 58-91, B: 94-141, C: 155-207, D: 295-345, E: 392-427.

### Figur 2: Größenanalyse der Reaktionsprodukte der erfindungsgemäßen PCR der Primerkombinationen GEX024 / GEX026 (Figur 2 A) und GEX29 / GEX031 (Figur 2 B) an Streptomyceten-DNA (Beispiel 1).

Als Matrize dienten hier Präparationen genomischer DNA von folgenden 20 zufällig ausgewählten *Streptomyces sp*.-Stämmen:
1: B101A, 2: B114C, 3: B134, 4: B135A, 5: B138A, 6: B152A, 7: B153B, 8: B161A, 9: B156B1 , 10: B157C, 11: B158A, 12: B160B, 13: B161A, 14: B373, 15 : B375, 16: B380, 17: B390, 18: B392A, 19: B392A, 20: B394.

Die Reaktionsprodukte (1/10 des Reaktionsvolumens) wurden jeweils in einem 2,5%igen Agarosegel aufgetrennt; beide teilweise vorhandenen Produkte von ca. 300 bp und ca. 500 bp Größe wurden über Sequenzierung als Amylase-Sequenzen identifiziert.
Marker (M): 100 bp DNA Leiter (in bp von oben nach unten: 3000, 2000, 1500, 1200, 1031, 900, 800, 700, 600, 500, 400, 300, 200, 100).

### Figur 3: Consensus-Sequenz von α-Amylasen aus 231 Stämmen der Gattung Streptomyces (SEQ ID NO. 2, 4 und 34 bis 262) mit entsprechenden Varianten für jede Position (Beispiel 3).

Der Consensus-Sequenz liegt ein Alignment mit dem Programm Clustal X^{®}, Version 1.64b zugrunde (Standardeinstellungen; beschrieben in: Thompson, J.D., Higgins, D.G. und Gibson, T.J. (1994), "CLUSTAL W: improving the sensitivity of progressive multiple sequence alignment through sequence weighting, position specific gap penalties and weight matrix choice", Nucleic Acids Res., Band 22, Seiten 4673-4680). Die Aminosäurepositionen 1-7 und 94-100 sind durch die PCR-Primersequenzen (GEX024/GEX026 und GEX029/031) abhängig von der Selektivität der durchgeführten PCR weitgehend vorgegeben.

Es handelt sich um eine alternative Darstellung der in SEQ ID NO. 263 angegebenen Consensus-Sequenz

### Figur 4: Schematische Darstellung des für die Expressions-Genbanken verwendeten Plasmidvektors pUC18 (GenBank (National Center for Biotechnology Information NCBI, National Institutes of Health, Bethesda, MD, USA), Accession-Number L08752; Beispiel 4).

Der Vektor wurde jeweils mit dem Restriktionsenzym *Acc* I linearisiert, um *Aci* I-fragmentierte, genomische Streptomyceten-DNA aufzunehmen.

ORI: Replikationsursprung; *lacI*: Gen für den *lac*-Repressor; *lacZ*-alpha: Gen für das α-Peptid der β-Galactosidase; AmpicillinR: Das Ampicillin-Resistenzgen der β-Lactamase.

### Figur 5. Der für die expression von Amylasen in Streptomyces lividans TK 24 verwendete Plasmid-Vektor pAX5a (Beispiel 4).

Darin bedeuten:
pUC19 ori: Replikationsursprung in E. coli; AmpicillinR, Ampicillinresistenzgen (beta-Lactamase); ThiostreptonR, Thiostreptonresistenzgen; plJ101, Replicon Replikationsursprung in Streptomyces; *erm*E, *erm*E-up-Promotor von *S*. *erythrea* (beschrieben in: Faß S.H., Engels, J.W. 1996, "influence of Specific Signal Peptide Mutations on the Expression and Secretion of the alpha-Amylase Inhibitor Tendamistat in Streptomyces lividans", J. Biol. Chem., Band 271 (Nummer 25), Seiten 15244-15252). Unter Ersatz der Tendamistatsequenz können zu klonierende Gene über *Spe*l- (5'-Ende) und EcoRI- (3'-Ende) Erkennungsstellen in pAX5a inseriert und darin, ausgehend vom konstitutiven *erm*E-Promotor exprimlert werden.

### Figur 6: Nachweis der amylolytischen Aktivität der erfindungsgemäßen α-Amylasen aus Streptomyces sp. B 327* und Streptomyces sp. B400B nach deren heterologer Expression in Escherichia coli.

Nach heterologer Expression der entsprechenden Gensequenzen in ***Escherichia coli*** (Beispiel 3), dem Aufbringen der transformierten Wirtszellen auf LB_{Amp}-Agarplatten mit 1% löslicher Stärke und anschließender Färbung der Platten mittels Lugolscher Lösung sind an den Abbauhöfen die Kolonien zu erkennen, deren Zellen die eingeschleusten Gensequenzen exprimieren.

Für diese Abbildung wurden Proben von aus den Transformationsansätzen durch Vereinzelung von abgeleiteten Klonen aufgetragen:
1. Positiv-Kontrolle: Expressionsstamm mit der α-Amylase aus *Streptomyces griseus* in pUC18);
2. Negativ-Kontrolle (pUC18 ohne Insert-DNA);
3. Probe eines Amylase-positiven Klons, der genomische DNA aus *Streptomyces sp.* B4008 enthält;
4. und 5. Proben zweier aus derselben Transformation erhaltener genomischer Klone aus *Streptomyces sp.* B327*;
6. bis 8. Proben dreier aus derselben Transformation erhaltener genomischer Klone aus *Streptomyces sp.* B327;
9. bis 12. Proben von vier aus derselben Transformation erhaltenen genomischen Klonen aus *Streptomyces griseus.*

## Patentansprüche

1. Amylolytisches Protein, dessen Aminosäuresequenz einen Teil enthält, der mit der in SEQ ID NO. 208 angegebenen Aminosäuresequenz zu 95%, bevorzugt zu 97,5%, besonders bevorzugt zu 100% identisch ist.

2. Amylolytisches, durch Insertionsmutation erhältliches oder amylolytisches chimäres Protein, welches wenigstens in einem eine amylolytische Aktivität verleihenden Teil aus einem Protein nach Anspruch 1 besteht.

3. Amylolytisches Protein gemäß einem der Ansprüche 1 oder 2, **dadurch gekennzeichnet, daß** es aus einem natürlichen Organismus, insbesondere aus einem Mikroorganismus erhältlich ist.

4. Amylolytisches Protein gemäß Anspruch 3, **dadurch gekennzeichnet, daß** es sich bei dem Mikroorganismus um ein gram-positives Bakterium handelt.

5. Amylolytisches Protein gemäß Anspruch 4, **dadurch gekennzeichnet, daß** es sich bei dem gram-positiven Bakterium um eines der Ordnung Actinomycetales handelt.

6. Amylolytisches Protein gemäß Anspruch 5, **dadurch gekennzeichnet, daß** es sich um eine Streptomyces-Spezies handelt.

7. Nukleinsäure, die für ein amylolytisches Protein codiert, dessen Aminosäuresequenz einen Teil enthält, der mit der in SEQ ID NO. 208 angegebenen Aminosäuresequenzen zu 95%, bevorzugt zu 97,5%, besonders bevorzugt zu 100% identisch ist.

8. Für eines der in Anspruch 2 bezeichneten amylolytischen Proteine codierende Nukleinsäure.

9. Verwendung eines Proteins nach einem der Ansprüche 1 bis 6 zur Identifizierung eines amylolytischen Proteins.

10. Verwendung einer Nukleinsäure nach einem der Ansprüche 7 oder 8 zur Identifizierung und/oder Gewinnung einer neuen Amylase.

11. Vektor, der einen in den Ansprüchen 7 oder 8 bezeichneten Nukleinsäurebereich enthält.

12. Klonierungsvektor gemäß Anspruch 11.

13. Expressionsvektor gemäß Anspruch 11.

14. Wirtszelle, die eines der in den Ansprüchen 1 bis 6 bezeichneten Proteine exprimiert oder zu dessen Expression angeregt werden kann, unter Einsatz eines Expressionsvektors gemäß Anspruch 13.

15. Wirtszelle gemäß Anspruch 14, **dadurch gekennzeichnet, daß** sie ein Bakterium ist, insbesondere eines, das das gebildete Protein ins umgebende Medium sekretiert.

16. Wirtstelle gemäß Anspruch 15, **dadurch gekennzeichnet, daß** sie ein gram-positives Bakterium ist.

17. Wirtszelle gemäß Anspruch 16, **dadurch gekennzeichnet, daß** sie der Gattung Bacillus, vorzugsweise der Species *Bacillus licheniformis, Bacillus amyloliquefaciens, Bacillus subtilis* oder *Bacillus alcalophilus* angehört.

18. Wirtszelle gemäß Anspruch 15, **dadurch gekennzeichnet, daß** sie ein gramnegatives Bakterium ist.

19. Wirtszelle gemäß Anspruch 18, **dadurch gekennzeichnet, daß** sie der Gattung Escherichia, bevorzugt der Spezies *Escherichia coli,* besonders bevorzugt einem der Stämme *E*. *coli* JM 109, *E*. *coli* DH 100B oder *E*. *coli* DH 12S angehört.

20. Wirtszelle gemäß Anspruch 14, **dadurch gekennzeichnet, daß** sie eine eukaryontische Zelle ist, insbesondere eine, die das gebildete Protein posttranslational modifiziert.

21. Verfahren zur Herstellung eines Proteins gemäß einem der Ansprüche 1 bis 6 unter Verwendung einer Nukleinsäure gemäß einem der Ansprüche 7 oder 8 und/oder unter Verwendung eines Vektors gemäß einem der Ansprüche 11 bis 13 und/oder unter Verwendung einer Wirtszelle gemäß einem der Ansprüche 14 bis 20 oder unter Verwendung einer Zelle, die dieses natürlicherweise bildet.

22. Wasch- oder Reinigungsmittel, **dadurch gekennzeichnet, daß** es ein Protein gemäß einem der Ansprüche 1 bis 6 enthält.

23. Verwendung eines Proteins gemäß einem der Ansprüche 1 bis 6 zur Behandlung von Rohmaterialien oder Zwischenprodukten in der Textilherstellung, insbesondere zum Entschlichten von Baumwolle.

24. Verfahren zur Stärkeverflüssigung, insbesondere zur Ethanolproduktion, **dadurch gekennzeichnet, daß** darin ein Protein gemäß einem der Ansprüche 1 bis 6 eingesetzt wird.

25. Verwendung eines Proteins gemäß einem der Ansprüche 1 bis 6 zur Herstellung von linearen und/oder kurzkettigen Oligosacchariden.

26. Verwendung eines Proteins gemäß einem der Ansprüche 1 bis 6 zur Hydrolyse von Cyclodextrinen.

27. Verwendung eines Proteins gemäß einem der Ansprüche 1 bis 6 zur Freisetzung von niedermolekularen Verbindungen aus Polysaccharidträgern oder Cyclodextrinen.

28. Verwendung eines Proteins gemäß einem der Ansprüche 1 bis 6 zur Herstellung von Lebensmitteln und/oder Lebensmittelbestandteilen.

29. Verwendung eines Proteins gemäß einem der Ansprüche 1 bis 6 zur Herstellung von Tierfutter und/oder Tierfutterbestandteilen.

30. Verwendung eines Proteins gemäß einem der Ansprüche 1 bis 6 zur Auflösung stärkehaltiger Klebeverbindungen.

31. Temporäres Klebeverfahren, **dadurch gekennzeichnet, daß** darin ein Protein gemäß einem der Ansprüche 1 bis 6 eingesetzt wird.

## Claims

1. An amylolytic protein, the amino acid sequence of which contains a portion which is 95%, preferably 97.5%, particularly preferably 100% identical to the amino acid sequence stated in SEQ ID NO. 208.

2. An amylolytic protein obtainable by insertion mutation or an amylolytic chimeric protein, which, at least in a portion which imparts amylolytic activity, consists of a protein according to claim 1.

3. An amylolytic protein according to either one of claim 1 or claim 2, **characterised in that** it is obtainable from a natural organism, in particular from a microorganism.

4. An amylolytic protein according to claim 3, **characterised in that** the microorganism is a Gram-positive bacterium.

5. An amylolytic protein according to claim 4, **characterised in that** the Gram-positive bacterium is a bacterium from the order Actinomycetales.

6. An amylolytic protein according to claim 5, **characterised in that** it is a Streptomyces species.

7. A nucleic acid which encodes an amylolytic protein, the amino acid sequence of which contains a portion which is 95%, preferably 97.5%, particularly preferably 100% identical to the amino acid sequence stated in SEQ ID NO. 208.

8. A nucleic acid which encodes one of the amylolytic proteins denoted in claim 2.

9. Use of a protein according to any one of claims 1 to 6 for identifying an amylolytic protein.

10. Use of a nucleic acid according to either one of claim 7 or claim 8 for identifying and/or isolating a novel amylase.

11. A vector which contains a nucleic acid domain denoted in claim 7 or claim 8.

12. A cloning vector according to claim 11.

13. An expression vector according to claim 11.

14. A host cell which expresses one of the proteins denoted in claims 1 to 6 or may be stimulated to express them, using an expression vector according to claim 13.

15. A host cell according to claim 14, **characterised in that** it is a bacterium, in particular a bacterium which secretes the protein formed into the surrounding medium.

16. A host cell according to claim 15, **characterised in that** it is a Gram-positive bacterium.

17. A host cell according to claim 16, **characterised in that** it belongs to the genus Bacillus, preferably to the species *Bacillus licheniformis, Bacillus amyloliquefaciens, Bacillus subtilis* or *Bacillus alcalophilus.*

18. A host cell according to claim 15, **characterised in that** it is a Gram-negative bacterium.

19. A host cell according to claim 18, **characterised in that** it belongs to the genus Escherichia, preferably to the species *Escherichia coli,* particularly preferably to one of the strains *E*. *coli* JM 109, *E. coli* DH 1008 or *E*. *coli* DH 12S.

20. A host cell according to claim 14, **characterised in that** it is a eukaryotic cell, in particular a eukaryotic cell which posttranslationally modifies the protein formed.

21. A method for producing a protein according to any one of claims 1 to 6 using a nucleic acid according to either one of claim 7 or claim 8 and/or using a vector according to any one of claims 11 to 13 and/or using a host cell according to any one of claims 14 to 20 or using a cell which naturally forms said protein.

22. A washing or cleaning agent, **characterised in that** it contains a protein according to any one of claims 1 to 6.

23. Use of a protein according to any one of claims 1 to 6 for treating raw materials or intermediates in textiles manufacture, in particular for scouring cotton.

24. A method for starch liquefaction, in particular for ethanol production, **characterised in that** a protein according to any one of claims 1 to 6 is used therein.

25. Use of a protein according to any one of claims 1 to 6 for producing linear and/or short-chain oligosaccharides.

26. Use of a protein according to any one of claims 1 to 6 for hydrolysing cyclodextrins.

27. Use of a protein according to any one of claims 1 to 6 for releasing low molecular weight compounds from polysaccharide substrates or cyclodextrins.

28. Use of a protein according to any one of claims 1 to 6 for producing foodstuffs and/or foodstuff ingredients.

29. Use of a protein according to any one of claims 1 to 6 for producing animal feed and/or animal feed ingredients.

30. Use of a protein according to any one of claims 1 to 6 for dissolving starch-containing adhesive bonds.

31. A temporary adhesive bonding method, **characterised in that** a protein according to any one of claims 1 to 6 is used therein.

## Revendications

1. Protéine amylolytique dont la séquence d'acides aminés contient une partie qui est identique à la séquence d'acides aminés indiquée dans la SEQ ID NO. 208, à concurrence de 95 %, de préférence à concurrence de 97,5 %, de manière particulièrement préférée, à concurrence de 100 %.

2. Protéine amylolytique que l'on obtient par mutation d'insertion ou protéine amylolytique chimère qui est constituée, au moins dans une partie conférant une activité amylolytique, d'une protéine selon la revendication 1.

3. Protéine amylolytique selon l'une quelconque des revendications 1 ou 2, **caractérisée en ce qu'**elle peut être obtenue à partir d'un organisme naturel, en particulier à partir d'un micro-organisme.

4. Protéine amylolytique selon la revendication 3, **caractérisée en ce qu'**il s'agit, en ce qui concerne le micro-organisme, d'une bactérie Gram-positive.

5. Protéine amylolytique selon la revendication 4, **caractérisée en ce qu'**il s'agit, en ce qui concerne la bactérie Gram-positive, d'une bactérie de l'ordre des actinomycètes.

6. Protéine amylolytique selon la revendication 5, **caractérisée en ce qu'**il s'agit d'une espèce streptomyces.

7. Acide nucléique qui code pour une protéine amylolytique, dont la séquence d'acides aminés contient une partie qui est identique à la séquence d'acides aminés indiquée dans la SEQ ID NO. 208, à concurrence de 95 %, de préférence à concurrence de 97,5 %, de manière particulièrement préférée, à concurrence de 100 %.

8. Acide nucléique codant pour une des protéines amylolytiques décrites à la revendication 2.

9. Utilisation d'une protéine selon l'une quelconque des revendications 1 à 6 pour l'identification d'une protéine amylolytique.

10. Utilisation d'un acide nucléique selon l'une quelconque des revendications 7 ou 8 pour l'identification et/ou pour l'obtention d'une nouvelle amylase.

11. Vecteur qui contient un domaine d'acide nucléique représenté dans les revendications 7 ou 8.

12. Vecteur de clonage selon la revendication 11.

13. Vecteur d'expression selon la revendication 11.

14. Cellule hôte qui exprime une des protéines décrites dans les revendications 1 à 6 ou qui peut être excitée pour l'expression d'une de ces dernières, à l'intervention d'un vecteur d'expression selon la revendication 13.

15. Cellule hôte selon la revendication 14, **caractérisée en ce qu'**il s'agit d'une bactérie, en particulier une bactérie qui sécrète dans le milieu environnant la protéine obtenue.

16. Cellule hôte selon la revendication 15, **caractérisée en ce qu'**il s'agit d'une bactérie Gram-positive.

17. Cellule hôte selon la revendication 16, **caractérisée en ce qu'**elle fait partie du genre Bacillus, de préférence de l'espèce *Bacillus licheniformis, Bacillus amyloliquefaciens, Bacillus subtilis* ou *Bacillus alcalophilus.*

18. Cellule hôte selon la revendication 15, **caractérisée en ce qu'**il s'agit d'une bactérie Gram-négative.

19. Cellule hôte selon la revendication 18, **caractérisée en ce qu'**elle fait partie du genre Escherichia, de préférence de l'espèce *Escherichia coli,* de manière particulièrement préférée d'une des souches *E*. *coli* JM 109, *E. coli* DH 100B ou *E*. *coli* DH 12S.

20. Cellule hôte selon la revendication 14, **caractérisée en ce qu'**il s'agit d'une cellule eucaryote, en particulier d'une cellule eucaryote qui soumet la protéine obtenue à une modification posttraductionnelle.

21. Procédé pour la préparation d'une protéine selon l'une quelconque des revendications 1 à 6 via l'utilisation d'un acide nucléique selon l'une quelconque des revendications 7 ou 8 et/ou via l'utilisation d'un vecteur selon l'une quelconque des revendications 11 à 13 et/ou via l'utilisation d'une cellule hôte selon l'une quelconque des revendications 14 à 20 ou bien via l'utilisation d'une cellule qui fournit ledit acide nucléique de manière naturelle.

22. Agent de lavage ou de nettoyage **caractérisé en ce qu'**il contient une protéine selon l'une quelconque des revendications 1 à 6.

23. Utilisation d'une protéine selon l'une quelconque des revendications 1 à 6 pour le traitement de matières premières ou de produits intermédiaires dans la fabrication des textiles, en particulier pour le désencollage du coton.

24. Procédé pour la liquéfaction de l'amidon, en particulier pour la production d'éthanol, **caractérisé en ce que** l'on y met en oeuvre une protéine selon l'une quelconque des revendications 1 à 6.

25. Utilisation d'une protéine selon l'une quelconque des revendications 1 à 6, pour l'obtention d'oligosaccharides linéaires et/ou à courtes chaînes.

26. Utilisation d'une protéine selon l'une quelconque des revendications 1 à 6, pour l'hydrolyse de cyclodextrines.

27. Utilisation d'une protéine selon l'une quelconque des revendications 1 à 6, pour la libération de composés à bas poids moléculaire à partir de porteurs de polysaccharides ou de cyclodextrines.

28. Utilisation d'une protéine selon l'une quelconque des revendications 1 à 6, pour la fabrication de denrées alimentaires et/ou de constituants de denrées alimentaires.

29. Utilisation d'une protéine selon l'une quelconque des revendications 1 à 6, pour la fabrication d'aliments pour animaux et/ou de constituants d'aliments pour animaux.

30. Utilisation d'une protéine selon l'une quelconque des revendications 1 à 6, pour la dissolution de composés adhésifs contenant de l'amidon.

31. Procédé de collage temporaire, **caractérisé en ce qu'**on y met en oeuvre une protéine selon l'une quelconque des revendications 1 à 6.
